# EUROPEAN PATENT SPECIFICATION

(11) **EP 2 519 642 B1**
(45) Date of publication and mention of the grant of the patent: **25.10.2017**
(21) Application number: 10844275.7
(22) Date of filing: 28.12.2010
(51) Int. Cl.: C12P 7/58, C12N 15/74

(54) **RECOMBINANT THRAUSTOCHYTRIDS THAT GROW ON XYLOSE, AND COMPOSITIONS, METHODS OF MAKING, AND USES THEREOF**
AUF XYLOSE WACHSENDE REKOMBINANTE THRAUSTOCHYTRIDE SOWIE ZUSAMMENSETZUNGEN, HERSTELLUNGSVERFAHREN UND VERWENDUNGEN DAVON
THRAUSTOCHYTRIDES RECOMBINANTS QUI CROISSENT SUR XYLOSE, ET COMPOSITIONS, PROCÉDÉS DE PRÉPARATION, ET UTILISATIONS ASSOCIÉES

(30) Priority: 28.12.2009 US 290471 P
(43) Date of publication of application: 07.11.2012
(73) Proprietor: DSM IP Assets B.V., 6411 TE Heerlen (NL)
(72) Inventor: LIPPMEIER, James Casey, Columbia, Maryland 21045 (US); APT, Kirk E., Ellicott City, Maryland 21042 (US)
(74) Representative: D Young & Co LLP
(86) International application number: PCT/US2010/062275
(87) International publication number: WO 2011/090730

(56) References cited:
- WO-A1-2007/080440
- WO-A1-2007/091117
- WO-A2-02/083869
- WO-A2-2008/129358
- WO-A2-2009/120731
- US-A1- 2009 053 784
- US-A1- 2009 064 567
- LEANDRO MARIA JOSE ET AL: "Two glucose/xylose transporter genes from the yeast Candida intermedia: first molecular characterization of a yeast xylose-H+ symporter", BIOCHEMICAL JOURNAL, THE BIOCHEMICAL SOCIETY, LONDON, GB, vol. 395, no. Part 3, 1 May 2006 (2006-05-01), pages 543-549, XP002431284, ISSN: 0264-6021, DOI: 10.1042/BJ20051465 & DATABASE EMBL [Online] 17 January 2006 (2006-01-17), "Candida intermedia mRNA for glucose/xylose symporter 1 (GXS1 gene)", retrieved from EBI accession no. EMBL:AJ875406 Database accession no. AJ875406
- JIN Y S ET AL: "Molecular Cloning of XYL3 (D-Xylulokinase) from Pichia stipitis and Characterizationof Its Physiological Function", APPLIED AND ENVIRONMENTAL MICROBIOLOGY, AMERICAN SOCIETY FOR MICROBIOLOGY, US, vol. 68, no. 3, 1 March 2002 (2002-03-01), pages 1232-1239, XP002904289, ISSN: 0099-2240, DOI: 10.1128/AEM.68.3.1232-1239.2002 & DATABASE EMBL [Online] 30 May 2000 (2000-05-30), "Pichia stipitis D-xylulokinase gene, complete cds.", retrieved from EBI accession no. EMBL:AF127802 Database accession no. AF127802
- HARHANGI HARRY R ET AL: "Xylose metabolism in the anaerobic fungus Piromyces sp. strain E2 follows the bacterial pathway", ARCHIVES OF MICROBIOLOGY, SPRINGER, DE, vol. 180, no. 2, 1 August 2003 (2003-08-01), pages 134-141, XP002530809, ISSN: 0302-8933, DOI: 10.1007/S00203-003-0565-0 [retrieved on 2003-06-13] & DATABASE EMBL [Online] 3 March 2000 (2000-03-03), "Piromyces sp. E2 mRNA for D-xylulokinase (xylB gene)", retrieved from EBI accession no. EMBL:AJ249910 Database accession no. AJ249910
- AMORE R ET AL: "Cloning and expression in Saccharomyces cerevisiae of the NAD(P)H-dependent xylose reductase-encoding gene (XYL1) from the xylose-assimilating yeast Pichia stipitis", GENE, ELSEVIER, AMSTERDAM, NL, vol. 109, no. 1, 20 December 1991 (1991-12-20), pages 89-97, XP023542201, ISSN: 0378-1119, DOI: 10.1016/0378-1119(91)90592-Y [retrieved on 1991-12-20] & DATABASE EMBL [Online] 19 October 1992 (1992-10-19), "P.stipitis XYL1-gene for NAD(P)H-dependent Xylose reductase", retrieved from EBI accession no. EMBL:X59465 Database accession no. X59465
- KOETTER P ET AL: "ISOLATION AND CHARACTERIZATION OF THE PICHIA STIPITIS XYLITOL DEHYDROGENASE GENE, XYL2, AND CONSTRUCTION OF A XYLOSE-UTILIZING SACCHAROMYCES CEREVISIAE TRANSFORMANT", CURRENT GENETICS, NEW YORK, NY, US, vol. 18, no. 6, 1 January 1990 (1990-01-01), pages 493-500, XP000909634, ISSN: 0172-8083, DOI: 10.1007/BF00327019 & DATABASE EMBL [Online] 21 February 1991 (1991-02-21), "Pichia stipitis XYL2 gene for xylitol dehydrogenase.", retrieved from EBI accession no. EMBL:X55392 Database accession no. X55392
- DATABASE EMBL [Online] 4 August 2004 (2004-08-04), "Arabidopsis thaliana At5g17010 gene, complete cds.", XP002697559, retrieved from EBI accession no. EMBL:BT015128 Database accession no. BT015128
- LEWIS T E ET AL: "The biotechnological potential of thraustochytrids", MARINE BIOTECHNOLOGY, SPRINGER VERLAG, NEW YORK, NY, US, vol. 1, 1 January 1999 (1999-01-01), pages 580-587, XP002436177, ISSN: 1436-2228, DOI: 10.1007/PL00011813
- DATABASE GENBANK [Online] 24 October 2006 'Candida intermedia mRNA for glucose/xylosé symporter 1 (GXS1 gene)' Database accession no. AJ875406.1.

## Description

### CROSS-REFERENCE TO RELATED APPLICATIONS

This application claims the benefit of the filing date of U.S. Appl. No. 61/290,471, filed December 28, 2009.

### BACKGROUND OF THE INVENTION

### Field of the Invention

The present invention is directed to a method of producing a thraustochytrid cell culture, comprising: a. transforming a thraustochytrid cell with nucleic acid molecules comprising a polynucleotide sequence encoding a heterologous xylose transporter, a heterologous xylose isomerase, and a heterologous xylulose kinase; or with nucleic acid molecules comprising a polynucleotide sequence encoding a heterologous xylose transporter, a heterologous xylulose kinase, a heterologous xylose reductase, and a heterologous xylitol dehydrogenase; wherein the heterologous genes are codon-optimised for expression in the thraustochytrid cell, and the heterologous genes are under the control of any promoter sequence, any terminator sequence and/or any other regulatory sequence that is functional in a thraustochytrid cell; and b. growing the transformed thraustochytrid cell in a culture medium comprising xylose as a carbon source, and cell cultures comprising the recombinant thraustochytrids, as well as methods of producing cell cultures, biomass, microbial oils, compositions, and biofuels using the recombinant thraustochytrids.

### Background

Fatty acids are classified based on the length and saturation characteristics of the carbon chain. Fatty acids are termed short chain, medium chain, or long chain fatty acids based on the number of carbons present in the chain, are termed saturated fatty acids when no double bonds are present between the carbon atoms, and are termed unsaturated fatty acids when double bonds are present. Unsaturated long chain fatty acids are monounsaturated when only one double bond is present and are polyunsaturated when more than one double bond is present.

Polyunsaturated fatty acids (PUFAs) are classified based on the position of the first double bond from the methyl end of the fatty acid: omega-3 (n-3) fatty acids contain a first double bond at the third carbon, while omega-6 (n-6) fatty acids contain a first double bond at the sixth carbon. For example, docosahexaenoic acid ("DHA") is an omega-3 long chain polyunsaturated fatty acid (LC-PUFA) with a chain length of 22 carbons and 6 double bonds, often designated as "22:6 n-3." Other omega-3 LC-PUFAs include eicosapentaenoic acid ("EPA"), designated as "20:5 n-3," and omega-3 docosapentaenoic acid ("DPA n-3"), designated as "22:5 n-3." DHA and EPA have been termed "essential" fatty acids. Omega-6 LC-PUFAs include arachidonic acid ("ARA"), designated as "20:4 n-6," and omega-6 docosapentaenoic acid ("DPA n-6"), designated as "22:5 n-6."

Omega-3 fatty acids are biologically important molecules that affect cellular physiology due to their presence in cell membranes, regulate production and gene expression of biologically active compounds, and serve as biosynthetic substrates. Roche, H. M., Proc. Nutr. Soc. 58: 397-401 (1999). DHA, for example, accounts for approximately 15%-20% of lipids in the human cerebral cortex and 30%-60% of lipids in the retina, is concentrated in the testes and sperm, and is an important component of breast milk. Jean-Pascal Bergé & Gilles Barnathan, Fatty Acids from Lipids of Marine Organisms: Molecular Biodiversity, Roles as Biomarkers, Biologically Active Compounds, and Economical Aspects, in Marine Biotechnology I 49 (T. Scheper, ed., 2005). DHA accounts for up to 97% of the omega-3 fatty acids in the brain and up to 93% of the omega-3 fatty acids in the retina. Moreover, DHA is essential for both fetal and infant development as well as maintenance of cognitive functions in adults. *Id.* Omega-3 fatty acids, including DHA and EPA, also possess anti-inflammatory properties. *See,* e.g., *id.* and Simopoulos, A.P., J. Am. Coll. Nutr. 21: 495-595 (2002). In particular, EPA competes with arachidonic acid for synthesis of eicosanoids such as prostaglandins and leukotrienes through cyclooxygenases and lipoxygenases. *Id.* Because omega-3 fatty acids are not synthesized de novo in the human body, these fatty acids must be derived from nutritional sources.

Thraustochytrids, which are microalgal organisms of the order Thraustochytriales, are recognized as alternative sources for the production of lipids. For example, strains of thraustrochytrid species have been reported to produce omega-3 fatty acids as a high percentage of the total fatty acids produced by the organisms. *See,* e.g., U.S. Patent No. 5,130,242; Huang, J. et al., J. Am. Oil. Chem. Soc. 78: 605-610 (2001); Huang, J. et al., Mar. Biotechnol. 5: 450-457 2003). Thraustochytrids have also been recognized as sources of lipids for the production of biofuels. *See,* e.g., U.S. Publ. No. 2009/0064567 and WO 008/067605.

Plant biomass is composed of three major fractions; cellulose, hemicelluloses, and lignin. Cellulose is composed primarily of glucose polymers, hemicellulase which is enriched with xylose polymers, and lignin which is composed primarily of complex polyphenolic compounds. The carbohydrates derived from these biomass fractions have been referred to collectively as lignocellulosic sugars and have been investigated as a source of low cost renewable feedstocks for fermentation of ethanol.

Plant hemicellulose from sources such as sugar cane bagasse, corn stover, switch grass, wheat straw, hard and soft wood, and the like can be a source of xylose enriched sugars. The sugars can be released from hemicellulose through acid hydrolysis and/or enzymatic digestion.

While thraustochytrids metabolize glucose and/or fructose, they do not appear to naturally metabolize xylose. See, e.g., Honda et al., Mycol. Res. 4:439-448 (1998); Goldstein, American J. of Botany 50:271-279 (1963); Damare, Indian Journal of Marine Sciences 35:326-340 (2006). As such, thraustochytrid cultures, including commercial and industrial cultures, currently require glucose syrups as carbon and energy sources.

A continuing need exists for producing thraustochytrids with the ability to grow on alternative carbon sources such as xylose for use in commercial and industrial applications.

### BRIEF SUMMARY OF THE INVENTION

The present invention is directed to a method of producing a thraustochytrid cell culture, comprising: a. transforming a thraustochytrid cell with nucleic acid molecules comprising a polynucleotide sequence encoding a heterologous xylose transporter, a heterologous xylose isomerase, and a heterologous xylulose kinase; or with nucleic acid molecules comprising a polynucleotide sequence encoding a heterologous xylose transporter, a heterologous xylulose kinase, a heterologous xylose reductase, and a heterologous xylitol dehydrogenase; wherein the heterologous genes are codon-optimised for expression in the thraustochytrid cell, and the heterologous genes are under the control of any promoter sequence, any terminator sequence and/or any other regulatory sequence that is functional in a thraustochytrid cell ; and b. growing the transformed thraustochytrid cell in a culture medium comprising xylose as a carbon source. The present invention is defined by the claims. Subject-matter outside the scope of the claims is provided for information only. In some embodiments, the thraustochytrid cell expresses a heterologous xylose transporter, a heterologous xylose isomerase, and a heterologous xylulose kinase. In some embodiments, the thraustochytrid cell expresses a heterologous xylose transporter, a heterologous xylose reductase, a heterologous xylitol dehydrogenase, and a heterologous xylulose kinase. In some embodiments, the polynucleotide sequence encoding the polypeptide associated with xylose import is at least 90% identical to a sequence selected from the group consisting of: the polynucleotide sequence of Accession No. AJ875406, BT015128, AF127802, AJ249910, X59465, or X55392; a polynucleotide sequence encoding the amino acid sequence of Accession No. CAB76571; the polynucleotide sequence of SEQ ID NO:2, SEQ ID NO:3, SEQ ID NO:6, SEQ ID NO:7, SEQ ID NO:21, SEQ ID NO:22, or SEQ ID NO:23; and combinations thereof. In some embodiments, the thraustochytrid is a *Schizochytrium* or a *Thraustochytrium.* In some embodiments, the invention is directed to a thraustochytrid cell culture produced by the method.

In some embodiments, the thraustochytrid cell expresses a heterologous xylose transporter, a heterologous xylulose kinase, a heterologous xylose reductase, and a heterologous xylitol dehydrogenase. In some embodiments, the polynucleotide sequence encoding the polypeptide associated with xylose import is at least 90% identical to a sequence selected from the group consisting of: the polynucleotide sequence of Accession No. AJ875406, BT015128, AF127802, AJ249910, X59465, or X55392; a polynucleotide sequence encoding the amino acid sequence of Accession No. CAB76571; the polynucleotide sequence of SEQ ID NO:2, SEQ ID NO:3, SEQ ID NO:6, SEQ ID NO:7, SEQ ID NO:21, SEQ ID NO:22, or SEQ ID NO:23; and combinations thereof. In some embodiments, the thraustochytrid is a *Schizochytrium* or a *Thraustochytrium.*

The present invention is also directed to a thraustochytrid culture comprising: (a) any of the thraustochytrid cells described herein, and (b) a cell culture medium comprising xylose as a carbon source.

The present invention is also directed to a method of producing a thraustochytrid biomass, comprising: (a) growing any of the thraustochytrid cells described herein in a culture medium comprising xylose as a carbon source, and (b) harvesting the biomass from the culture medium.

The present invention is also directed to a method of producing a microbial oil, comprising: (a) growing any of the thraustochytrid cells described herein in a culture medium comprising xylose as a carbon source to produce a biomass, and (b) extracting an oil from the biomass.

The present invention is also directed to a method of producing a food product, cosmetic, industrial composition, or pharmaceutical composition for an animal or human, comprising: (a) growing any of the thraustochytrid cells described herein in a culture medium comprising xylose as a carbon source, (b) harvesting a biomass from the culture medium, and (c) preparing the food product, cosmetic, industrial composition, or pharmaceutical composition from the biomass. In some embodiments, the method further comprises extracting an oil from the biomass and preparing the food product, cosmetic, industrial composition, or pharmaceutical composition from the oil. In some embodiments, the food product is an infant formula. In some embodiments, the infant formula is suitable for premature infants. In some embodiments, the food product is milk, a beverage, a therapeutic drink, a nutritional drink, or a combination thereof. In some embodiments, the food product is an additive for animal or human food. In some embodiments, the food product is a nutritional supplement. In some embodiments, the food product is an animal feed. In some embodiments, the animal feed is an aquaculture feed. In some embodiments, the animal feed is a domestic animal feed, a zoological animal feed, a work animal feed, a livestock feed, or a combination thereof.

The present invention is also directed to a method for producing a biofuel, comprising: (a) growing any of the thraustochytrid cells described herein in a culture medium comprising xylose as a carbon source to produce a biomass, (b) extracting an oil from the biomass, and (c) producing a biofuel by transesterifying the oil, cracking the oil, processing the oil by thermal depolymerization, adding the oil to a petroleum refining process, or a combination thereof. In some embodiments, the biofuel is produced by transesterifying the oil. In some embodiments, the biofuel is a biodiesel. In some embodiments, the biofuel is produced by cracking the oil. In some embodiments, the biofuel is a jet biofuel. In some embodiments, the biofuel is produced by processing the oil by thermal depolymerization. In some embodiments, the biofuel is a renewable diesel. In some embodiments, the biofuel is produced by adding the oil to a petroleum refining process. In some embodiments, the biofuel is a co-processed renewable diesel.

### BRIEF DESCRIPTION OF THE DRAWINGS/FIGURES

**FIG. 1** shows a plasmid map of pCL0120 (SEQ ID NO: 12).
**FIG. 2** shows a codon usage table for *Schizochytrium.*
**FIG. 3** shows the codon-optimized polynucleotide sequence (SEQ ID NO:2) encoding the *Candida intermedia* xylose transporter protein GXS1, corresponding to GenBank Accession No. AJ875406.
**FIG. 4** shows the codon-optimized polynucleotide sequence (SEQ ID NO:3) encoding the *Arabidopsis thaliana* xylose transporter protein At5g17010, corresponding to GenBank Accession No. BT015128.
**FIG. 5** shows a plasmid map of pCL0130 (SEQ ID NO:14).
**FIG. 6** shows a plasmid map of pCL0131 (SEQ ID NO:15).
**FIGS. 7A** and **7B** shows the polynucleotide sequence of pCL0121 (SEQ ID NO:4). The vector confers resistance to the antibiotic ZEOCIN™ and also harbors a gene encoding a secreted form of eGFP.
**FIGs. 8A** and **8B** shows the polynucleotide sequence of pCL0122 (SEQ ID NO:5). The vector confers resistance to the antibiotic paromomycin and also harbors a gene encoding a secreted form of eGFP.
**FIG. 9** shows a plasmid map of pCL0121.
**FIG. 10** shows a plasmid map of pCL0122.
**FIG. 11** shows the codon-optimized polynucleotide sequence (SEQ ID NO:6) encoding the *Piromyces* sp. E2 xylose isomerase protein "XylA", corresponding to GenBank Accession No. CAB76571.
**FIG. 12** shows the codon-optimized polynucleotide sequence (SEQ ID NO:7) encoding the *Piromyces* sp. E2 xylulose kinase protein "XylB", corresponding to GenBank Accession No. AJ249910.
**FIG. 13** shows a plasmid map of pCL0132 (SEQ ID NO:16).
**FIG. 14** shows a plasmid map of pCL0136 (SEQ ID NO:20).
**FIG. 15** shows the codon-optimized polynucleotide sequence (SEQ ID NO:21) encoding the *Pichia stipitis* xylose reductase protein "Xyll" corresponding to GenBank Accession No. X59465.
**FIG. 16** shows the codon-optimized polynucleotide sequence (SEQ ID NO:22) encoding the *Pichia stipitis* xylulose kinase protein "Xuk3" corresponding to GenBank Accession No. AF127802.
**FIG. 17** shows a plasmid map of pCL0133 (SEQ ID NO:17).
**FIG. 18** shows a plasmid map of pCL0135 (SEQ ID NO:19).
**FIG. 19** shows a plasmid map of pCL0123 (SEQ ID NO:13).
**FIG. 20** shows the codon-optimized polynucleotide sequence (SEQ ID NO:23) encoding the *Pichia stipitis* xylitol dehydrogenase protein "Xyl2" corresponding to GenBank Accession No. X55392.
**FIG. 21** shows a plasmid map of pCL0134 (SEQ ID NO:18).
**FIG. 22** shows Western blots of culture pellet lysates from cultures of SMM-resistant *Schizochytrium* clones transformed with either pCL0130 (**FIG**. **22A**) or pCL0131 (**FIG. 22B**) and *Schizochytrium* wild type strain ATCC 20888 (WT), probed with antibodies that recognize either the *Candida intermedia* xylose transporter protein GXS1 (pCL0130 transformant) or the *Arabidopsis thaliana* xylose transporter protein At5g17010 (pCL0131 transformant).

### DETAILED DESCRIPTION OF THE INVENTION

The present invention is directed to a method of producing a thraustochytrid cell culture, comprising: a. transforming a thraustochytrid cell with nucleic acid molecules comprising a polynucleotide sequence encoding a heterologous xylose transporter, a heterologous xylose isomerase, and a heterologous xylulose kinase; or with nucleic acid molecules comprising a polynucleotide sequence encoding a heterologous xylose transporter, a heterologous xylulose kinase, a heterologous xylose reductase, and a heterologous xylitol dehydrogenase; wherein the heterologous genes are codon-optimised for expression in the thraustochytrid cell, and the heterologous genes are under the control of any promoter sequence, any terminator sequence and/or any other regulatory sequence that is functional in a thraustochytrid cell ; and b. growing the transformed thraustochytrid cell in a culture medium comprising xylose as a carbon source and cell cultures comprising the recombinant thraustochytrids, as well as methods of producing cell cultures, biomasses, microbial oils, compositions, and biofuels using the recombinant thraustochytrids.

### Thraustochytrids

According to the present invention, the term "thraustochytrid" refers to any member of the order Thraustochytriales, which includes the family Thraustochytriaceae. The current taxonomic placement of the thraustochytrids can be summarized as follows:
Realm: Stramenopila (Chromista)
   Phylum: Labyrinthulomycota (Heterokonta)
      Class: Labyrinthulomycetes (Labyrinthulae)
         Order: Labyrinthulales
            Family: Labyrinthulaceae
         Order: Thraustochytriales
            Family: Thraustochytriaceae
For purposes of the present invention, strains described as thraustochytrids include the following organisms: Order: Thraustochytriales; Family: Thraustochytriaceae; Genera: *Thraustochytrium* (Species: sp., *arudimentale, aureum, benthicola, globosum, kinnei, motivum, multirudimentale, pachydermum, proliferum, roseum, striatum*), *Ulkenia* (Species: sp., *amoeboidea, kerguelensis, minuta, profunda, radiata, sailens, sarkariana, schizochytrops, visurgensis, yorkensis*), *Schizochytrium* (Species: sp., *aggregatum, limnaceum, mangrovei, minutum, octosporum*), *Japonochytrium* (Species: sp., *marinum), Aplanochytrium* (Species: sp., *haliotidis, kerguelensis, profunda, stocchinoi), Althornia* (Species: sp., *crouchii),* or *Elina* (Species: sp., *marisalba, sinorifca*). For the purposes of this invention, species described within *Ulkenia* will be considered to be members of the genus *Thraustochytrium. Aurantiochytrium, Oblongichytrium, Botryochytrium, Parietichytrium,* and *Sicyoidochytrium* are additional genuses encompassed by the present invention.

Strains of *Thraustochytriales* include, but are not limited to, deposited strains PTA-10212, PTA-10213, PTA-10214, PTA-10215, PTA-9695, PTA-9696, PTA-9697, PTA-9698, PTA-10208, PTA-10209, PTA-10210, PTA-10211, *Thraustochytrium* sp. (23B) (ATCC 20891); *Thraustochytrium striatum* (Schneider)(ATCC 24473); *Thraustochytrium aureum* (Goldstein) (ATCC 34304); *Thraustochytrium roseum* (Goldstein) (ATCC 28210); and *Japonochytrium* sp. (L1) (ATCC 28207). *Schizochytrium* include, but are not limited to *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209 and deposited *Schizochytrium limacinum* strain IFO 32693.

In some embodiments, the thraustochytrid cell of the invention is a *Schizochytrium* or a *Thraustochytrium* cell. In some embodiments, the thraustochytrid is from a species selected from *Schizochytrium* sp., *Schizochytrium aggregatum, Schizochytrium limacinum, Schizochytrium minutum, Thraustochytrium* sp., *Thraustochytrium striatum, Thraustochytrium aureum, Thraustochytrium roseum, Japonochytrium* sp., and strains derived therefrom.

According to the present invention, the term "transformation" is used to refer to any method by which a nucleic acid molecule (i.e., a recombinant nucleic acid molecule) can be inserted into a thraustochytrid cell of the invention. In microbial systems, the term "transformation" is used to describe an inherited change due to the acquisition of exogenous nucleic acids by the microorganism and is essentially synonymous with the term "transfection." Suitable transformation techniques for introducing nucleic acid molecules into thraustochytrid cells include, but are not limited to, particle bombardment, electroporation, microinjection, lipofection, adsorption, infection, and protoplast fusion.

Although the phrase "nucleic acid molecule" primarily refers to the physical nucleic acid molecule and the phrases "nucleic acid sequence" or "polynucleotide sequence" primarily refers to the sequence of nucleotides in the nucleic acid molecule, the phrases are used interchangeably, especially with respect to a nucleic acid molecule, polynucleotide sequence, or a nucleic acid sequence that is capable of encoding a protein. In some embodiments, isolated nucleic acid molecules as described herein are produced using recombinant DNA technology (e.g., polymerase chain reaction (PCR) amplification or cloning) or chemical synthesis. In accordance with the present invention, an "isolated" nucleic acid molecule is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include DNA, RNA (e.g., mRNA), or derivatives of either DNA or RNA (e.g., cDNA). Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on sequence, function, and/or the biological activity of the encoded peptide or protein.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10, or 20 amino acids. In some embodiments, a polypeptide as described herein is a heterologous polypeptide. The term "heterologous" as used herein refers to a sequence, for example, that is not naturally found in the thraustochytrid cell of the invention.

The expressed polypeptide is a heterologous polypeptide. Described is a thraustochytrid cell comprising a polynucleotide sequence encoding a heterologous xylose transporter (e.g., accession nos. AJ875406 and/or BT015128), heterologous xylose isomerase (e.g., accession no. CAB76571), heterologous xylulose kinase (e.g., accession no. AF127802 and/or AJ249910), heterologous xylose reductase (e.g., accession no. X59465), heterologous xylitol dehydrogenase (e.g., accession no. X55392), or any combination thereof. In some examples, the thraustochytrid cell comprises a polynucleotide sequence encoding a xylose transporter (e.g., accession nos. AJ875406 and/or BT015128), a xylose isomerase (e.g., accession no. CAB76571), and a xylulose kinase (e.g., accession nos. AF127802 and/or AJ249910). In some examples, the xylose isomerase is as described in WO 03/062430 A1. The polynucleotide sequence is codon-optimized to maximize translation efficiency in the thraustochytrid cell. In some examples, a polynucleotide sequence encoding a xylose transporter, a xylose isomerase, a xylulose kinase, a xylose reductase, a xylitol dehydrogenase, or any combination thereof is integrated into the genome of the thraustochytrid cell. In some examples, a polynucleotide sequence encoding a xylose transporter, a xylose isomerase, a xylulose kinase, a xylose reductase, a xylitol dehydrogenase, or any combination thereof is stably integrated into the genome of the thraustochytrid cell.

In some examples, the expression system used for the production of a xylose transporter, a xylose isomerase, a xylulose kinase, a xylose reductase, a xylitol dehydrogenase, or any combination thereof in a thraustochytrid cell comprises regulatory elements that are derived from thraustochytrid sequences. In some examples, the expression system used for the production of a xylose transporter, a xylose isomerase, a xylulose kinase, a xylose reductase, a xylitol dehydrogenase, or any combination thereof in a thraustochytrid cell comprises regulatory elements that are derived from non-thraustochytrid sequences, including sequences derived from non-thraustochytrid algal sequences. In some examples, the expression system comprises a polynucleotide sequence encoding a xylose transporter, a xylose isomerase, a xylulose kinase, a xylose reductase, a xylitol dehydrogenase, or any combination thereof wherein the polynucleotide sequence is operably linked to any promoter sequence, any terminator sequence, and/or any other regulatory sequence that is functional in a thraustochytrid cell. Inducible or constitutively active regulatory sequences can be used. Regulatory sequences include but are not limited to the *Schizochytrium* regulatory sequences described in U.S. Publ. No. 2010/0233760, U.S. Patent No. 7,001,772, U.S. Publ. No. 2006/0275904, and U.S. Publ. No. 2006/0286650, such as: an OrfC (also termed Pfa3) promoter, an OrfC terminator, an EF1 short promoter, an EF1 long promoter, a Secl promoter, a 60S short promoter, a 60S long promoter, an acetolactate synthase promoter, an acetolactate synthase terminator, an α-tubulin promoter, a promoter from a polyketide synthase (PKS) system, a fatty acid desaturase promoter, an actin promoter, an actin terminator, an elongation factor 1 alpha (eflα) promoter, an efla terminator, a glyceraldehyde 3-phosphate dehydrogenase (gapdh) promoter, a gapdh terminator, and combinations thereof.

In some examples, the xylose transporter, xylose isomerase, xylulose kinase, xylose reductase, xylitol dehydrogenase, or any combination thereof is expressed by a recombinant thraustochytrid transformed with a xylose transporter gene, a xylose isomerase gene, a xylulose kinase gene, a xylose reductase gene, a xylitol dehydrogenase gene, or any combination thereof. In some examples, any of one or more of the xylose transporters is a plasma-membrane bound protein. In some examples, any of one or more of the xylose isomerase, xylulose kinase, xylose reductase, or xylitol dehydrogenase is an intracellular protein.

In some examples, the xylose transporter, xylose isomerase, xylulose kinase, a xylose reductase, xylitol dehydrogenase, or any combination thereof is expressed intracellularly. In some examples, the xylose transporters are membrane bound. In certain examples, the xylose transporters are localized to the exterior of the plasma membrane.

In some examples, an expression cassette is used for the expression of a xylose transporter, a xylose isomerase, a xylulose kinase, a xylose reductase, a xylitol dehydrogenase, or any combination thereof in a thraustochytrid cell. The design and construction of expression cassettes use standard molecular biology techniques known to persons skilled in the art. *See,* e.g., Sambrook et al., 2001, Molecular Cloning: A Laboratory Manual, 3rd edition. In some examples, the thraustochytrid cell comprises an expression cassette containing genetic elements, such as at least the following, operably linked in such a way that they are functional in the thraustochytrid cell: a promoter; a coding sequence comprising a xylose transporter, a xylose isomerase, a xylulose kinase, a xylose reductase, a xylitol dehydrogenase, or any combination thereof; and a terminator region. In some examples, the expression cassette further comprises a polynucleotide sequence encoding a signal peptide or a membrane domain operably linked to the xylose transporter, xylose isomerase, xylulose kinase, xylose reductase, or xylitol dehydrogenase. The term "membrane domain" as used herein refers to any domain within a polypeptide that targets the polypeptide to a membrane and/or allows the polypeptide to maintain association with a membrane and includes, but is not limited to, a transmembrane domain (e.g., a single or multiple membrane spanning region), an integral monotopic domain, a signal anchor sequence, an ER signal sequence, an N-terminal or internal or C-terminal stop transfer signal, a glycosylphosophatidylinositol anchor, and combinations thereof. A membrane domain can be located at any position in the polypeptide, including the N-terminal, C-terminal, or middle of the polypeptide. A membrane domain can be associated with permanent or temporary attachment of a polypeptide to a membrane. In some embodiments, a recombinant vector comprises the expression cassette. Recombinant vectors include, but are not limited to, plasmids, phages, and viruses. In some embodiments, the recombinant vector is a linearized vector. In some embodiments, the recombinant vector is an expression vector. As used herein, the phrase "expression vector" refers to a vector that is suitable for production of an encoded product (e.g., a xylose transporter, a xylose isomerase, a xylulose kinase, a xylose reductase, a xylitol dehydrogenase, or any combination thereof). In some examples, a polynucleotide sequence encoding the xylose transporter, xylose isomerase, xylulose kinase, xylose reductase, xylitol dehydrogenase, or any combination thereof is inserted into the recombinant vector to produce a recombinant nucleic acid molecule. In some examples, the polynucleotide sequence encoding the xylose transporter, xylose isomerase, xylulose kinase, xylose reductase, xylitol dehydrogenase, or any combination thereof is inserted into the vector in a manner that operatively links the nucleic acid sequence to regulatory sequences in the vector, which enables the transcription and translation of the nucleic acid sequence within the recombinant microorganism. In some embodiments, a selectable marker enables the selection of a recombinant microorganism into which a recombinant nucleic acid molecule of the present invention has successfully been introduced. Selectable markers include but are not limited to the selectable markers described in U.S. Publ. No. 2010/0233760 and U.S. Patent No. 7,001 772, such as *Schizochytrium* acetolactate synthase or bacterial ble. In some embodiments, the selection marker is an auxotrophic marker, a dominant selection marker (such as, for example, an enzyme that detoxifies an antibiotic), or another protein involved in transformation selection.

In some embodiments, a polynucleotide sequence of any of the methods of the invention encodes a heterologous polypeptide comprising an amino acid sequence that is at least 90%, at least 91%, at least 92%, at least 93%, at least 94%, at least 95%, at least 96%, at least 97%, at least 98%, at least 99%, or that is identical to the amino acid sequence of a xylose transporter, xylose isomerase, xylose reductase, xylitol dehydrogenase, or xylulose kinase. In some embodiments, a polynucleotide sequence of any of the methods of the invention encodes a polypeptide comprising an amino acid sequence that is at least 90%, at least 91%, at least 92%, at least 93%, at least 94%, at least 95%, at least 96%, at least 97%, at least 98%, at least 99%, or that is identical to: the amino acid sequence encoded by Accession No. AJ875406, BT015128, AF127802, AJ249910, X59465, or X55392; or the amino acid sequence of Accession No. CAB76571. In some embodiments, a polynucleotide sequence of any of the methods of the invention encodes a polypeptide comprising an amino acid sequence that is at least 90%, at least 91%, at least 92%, at least 93%, at least 94%, at least 95%, at least 96%, at least 97%, at least 98%, at least 99%, or that is identical to the amino acid sequence encoded by SEQ ID NO:2, SEQ ID NO:3, SEQ ID NO:6, SEQ ID NO:7, SEQ ID NO:21, SEQ ID NO:22, or SEQ ID NO:23. In some embodiments, a polynucleotide sequence of any of the methods of the invention is at least 90%, at least 91%, at least 92%, at least 93%, at least 94%, at least 95%, at least 96%, at least 97%, at least 98%, at least 99%, or that is identical to: the polynucleotide sequence of Accession No. AJ875406, BT015128, AF127802, AJ249910, X59465, or X55392; a polynucleotide sequence of Accession No. AJ875406, BT015128, AF127802, AJ249910, X59465, or X55392 that is codon-optimized for expression in *Schizochytrium*; a polynucleotide sequence encoding the amino acid sequence of Accession No. CAB76571; a polynucleotide sequence encoding the amino acid sequence of accession no. CAB76571, wherein the polynucleotide sequence is codon-optimized for expression in *Schizochytrium;* or the polynucleotide sequence of SEQ ID NO:2, SEQ ID NO:3, SEQ ID NO:6, SEQ ID NO:7, SEQ ID NO:21, SEQ ID NO:22, or SEQ ID NO:23.

### Thraustochytrid Cultures, Biomasses, and Microbial Oils

The present invention is also directed to a thraustochytrid cell culture comprising any of the thraustochytrid cells of the invention as described herein and a cell culture medium comprising xylose as a carbon source.

The present invention is also directed to a method of producing a thraustochytrid cell culture, comprising transforming a thraustochytrid cell with a nucleic acid molecule comprising a polynucleotide sequence encoding a polypeptide associated with xylose metabolism as described herein, selecting the transformed thraustochytrid cell, and growing the transformed thraustochytrid cell in a culture medium comprising xylose as a carbon source, wherein a thraustochytrid cell culture is produced. The present invention is also directed to a thraustochytrid cell culture produced by the method. Sources of xylose include but are not limited to sugar cane bagasse, corn stover, switch grass, wheat straw, hard and softwood, or other plant material.

In some embodiments, xylose is the primary carbon source in the cell culture medium. In some embodiments, xylose is the only carbon source in the cell culture medium. In some embodiments, the culture medium comprises molasses, a syrup, hydrolysate, extract, or juice from any xylose-producing plant, or combinations thereof. In some embodiments, the culture medium comprises a hemicellulose-containing feedstock, such as sugar cane bagasse, corn stover, switch grass, wheat straw, hard and soft wood or other plant material.

In some embodiments, the cell culture medium comprises a xylose transporter, a xylose isomerase, a xylulose kinase, a xylose reductase, a xylitol dehydrogenase, or any combination thereof. In some embodiments, the thraustochytrid cell exports a heterologous xylose transporter, a heterologous xylose isomerase, a heterologous xylulose kinase, a heterologous xylose reductase, a heterologous xylitol dehydrogenase, or any combination thereof into the culture medium.

Culture conditions for thraustochytrid cells include, but are not limited to, effective media, bioreactor, temperature, pH, and oxygen conditions that permit protein production and/or recombination. An effective medium refers to any medium in which a thraustochytrid cell is typically cultured. Such medium typically comprises an aqueous medium having assimilable carbon, nitrogen, and phosphate sources, as well as appropriate salts, minerals, metals, and other nutrients, such as vitamins. Nitrogen sources include, but are not limited to, peptone, yeast extract, polypeptone, malt extract, meat extract, casamino acid, corn steep liquor, organic nitrogen sources, sodium glutamate, urea, inorganic nitrogen sources, ammonium acetate, ammonium sulfate, ammonium chloride, ammonium nitrate, and sodium sulfate. Non-limiting culture conditions suitable for thraustochytrids are described, for example, in U.S. Patent No. 5,340,742. Various fermentation parameters for inoculating, growing, and recovering microflora are also described, for example, in U.S. Patent No. 5,130,242. Liquid or solid media can contain natural or artificial sea water. Thraustochytrid cells of the present invention can be cultured in fermentation bioreactors, shake flasks, test tubes, microtiter dishes, and petri plates.

The fermentation volume can be any volume used for the growth of thraustochytrids, including commercial and industrial volumes. In some embodiments, the fermentation volume (volume of culture) is at least 2 L, at least 10 L, at least 50 L, at least 100 L, at least 200 L, at least 500 L, at least 1000 L, at least 10,000 L, at least 20,000 L, at least 50,000 L, at least 100,000 L, at least 150,000 L, at least 200,000 L, or at least 250,000 L, at least 300,000 L, at least 350,000 L, at least 400,000 L, or at least 500,000 L. In some embodiments, the fermentation volume is 2 L to 2,000,000 L, 2 L to 1,000,000 L, 2 L to 500,000 L, 2 L to 200,000 L, 2 L to 100,000 L, 2 L to 50,000 L, 2 L to 25,000 L, 2 L to 20,000 L, 2 L to 15,000 L, 2 L to 10,000 L, 2 L to 5,000 L, 2 L to 1,000 L, 2 L to 500 L, or 2 L to 100 L.

The present invention is also directed to a method of producing a thraustochytrid biomass comprising growing a thraustochytrid cell of the invention in a culture medium comprising xylose as a carbon source, and harvesting a biomass from the culture medium. A thraustochytrid biomass is a harvested cellular biomass obtained by any conventional method for the isolation of a thraustochytrid biomass, such as described in U.S. Patent No. 5,130,242 and U.S. Publ. No. 2002/0001833. A harvested biomass can contain thraustochytrid cells as well as thraustochytrid cellular fragments.

The present invention is also directed to a method of producing a microbial oil comprising growing a thraustochytrid cell of the invention in a culture medium comprising xylose as a carbon source to produce a biomass, and extracting an oil from the biomass. The oil can be extracted from a freshly harvested biomass or can be extracted from a previously harvested biomass that has been stored under conditions that prevent spoilage. Known methods can be used to culture a thraustochytrid of the invention, to isolate a biomass from the culture, to extract microbial oil from the biomass, and to analyze the fatty acid profile of oils extracted from the biomass. *See,* e.g., U.S. Patent No. 5,130,242.

A microbial oil can be any oil derived from any thraustochytrid, including, for example: a crude oil extracted from the biomass of a thraustochytrid without further processing; a refined oil that is obtained by treating a crude oil with further processing such as refining, bleaching, and/or deodorizing; a diluted oil obtained by diluting a crude or refined oil; or an enriched oil that is obtained, for example, by treating a crude or refined oil with further methods of purification to increase the concentration of a fatty acid in the oil.

In some embodiments, the crude oil can be isolated from a thraustochytrid using standard techniques, without being subjected to further refinement or purification. For example, the crude oil can be isolated using solvent extraction, such as, but not limited to, hexane extraction or isopropanol extraction. In some embodiments, the crude oil can be isolated using physical and/or mechanical extraction methods such as, but not limited to, extraction through the use of a homogenizer or by pressing.

In some embodiments, the crude oil can be subjected to further processing, such as refining, desolventization, deodorization, winterization, chill filtration, and/or bleaching. Such "processed" oils include microbial oils that have been subjected to solvent extraction and one or more further processing. In some embodiments, oils are minimally processed. "Minimally processed" oils include microbial oils that have been subjected to solvent extraction and filtration. In certain embodiments, minimally processed oils are further subjected to winterization.

In some embodiments, a method similar to the FRIOLEX® process (Westfalia Separator Industry GmbH, Germany) is used to extract the microbial oils produced by the thraustochytrids. The FRIOLEX® process is a water-based physical oil extraction process, whereby raw material containing oil can be used directly for extracting oil without using any conventional solvent extraction methods. In this process, a water-soluble organic solvent can be used as a process aid and the oil is separated from the raw material broth by density separation using gravity or centrifugal forces. WO 96/05278 discloses such extraction.

After the oil has been extracted, the oil can be recovered or separated from non-lipid components by any suitable means known in the art. In some embodiments, low-cost physical and/or mechanical techniques are used to separate the lipid-containing compositions from non-lipid compositions. For example, if multiple phases or fractions are created by the extraction method used to extract the oil, where one or more phases or fractions contain lipids, a method for recovering the lipid-containing phases or fractions can involve physically removing the lipid-containing phases or fractions from the non-lipid phases or fractions, or vice versa. In some embodiments, a FRIOLEX® type method is used to extract the lipids produced by the microorganisms, and the lipid-rich light phase is then physically separated from the protein-rich heavy phase (such as by skimming off the lipid-rich phase that is on top of the protein-rich heavy phase after density separation).

The microbial oils produced by thraustochytrids of the present invention can be recovered from autolysis or induced lysis by exposing thraustochytrid cells to a condition including, but not limited to, a certain pH, a certain temperature, the presence of an enzyme, the presence of a detergent, physical disruptions, or combinations thereof. In some embodiments, lysis or autolysis of the thraustochytrid cells is performed by the use of mechanical forces. In further embodiments of the present invention, the lysis or autolysis of thraustochytrid cells is followed by mechanical separation of the lipids from the non-lipid compositions.

Suitable enzymes that can be used to induce lysis of the thraustochytrid cells include, but are not limited to, commercially available enzymes or enzyme mixtures such as Proteinase K or ALCALASE®. In some embodiments, the oil-producing thraustochytrids undergo induced lysis in the presence of a detergent such as ionic (cationic or anionic) detergents, nonionic detergents, zwitterionic detergents, or combinations thereof. In further embodiments of the present invention, physical disruption methods such as mechanical grinding, liquid homogenization, use of high frequency sound waves in sonication, freeze/thaw cycles methods, pressing, extruding, or milling can be used to induce lysis of the oil-producing thraustochytrids. The extraction of the oils can take place in the fermentors at the end of the fermentation by in-tank lysis of the thraustochytrid cells.

In some embodiments, the cell cultures, biomasses, and microbial oils produced from the thraustochytrid cells of the invention grown on xylose have the same or substantially similar characteristics (e.g., cell densities, dry cell weights, fatty acid productivities, and fatty acid profiles) associated with cultures, biomasses, and microbial oils produced from the corresponding untransformed thraustochytrid cells grown on glucose and/or fructose. In some embodiments, the dry cell weight of a biomass produced from a thraustochytrid culture of the invention is higher after growth on xylose than the dry cell weight obtained under identical conditions from growth of a culture of the corresponding untransformed thraustochytrid cells on glucose and/or fructose. In some embodiments, the total amount of fatty acids as a percentage of the dry cell weight of the biomass is higher after growth of a thraustochytrid of the invention on xylose than the amount obtained under identical conditions from growth of a corresponding untransformed thraustochytrid cell on glucose and/or fructose.

### Methods of Producing Compositions

The present invention is also directed to a method of preparing a food product, cosmetic, industrial composition, or pharmaceutical composition for animals or humans, comprising growing a thraustochytrid cell of the invention in a culture medium comprising xylose as a carbon source to produce a biomass, harvesting the biomass, and preparing the food product, cosmetic, industrial composition, or pharmaceutical composition for animals or humans from the biomass.

Thraustochytrid biomasses can be dried prior to use in a composition by methods including, but not limited to, freeze drying, air drying, spray drying, tunnel drying, vacuum drying (lyophilization), or a similar process. Alternatively, a harvested and washed biomass can be used directly in a composition without drying. *See,* e.g., U.S. Patent Nos. 5,130,242 and 6,812,009.

In some embodiments, the method of preparing a food product, cosmetic, industrial composition, or pharmaceutical composition for animals or humans, further comprises extracting oil from the biomass. Microbial oils can be used as starting material to more efficiently produce a product enriched in a fatty acid such as DHA or EPA. For example, the microbial oils can be subjected to various purification techniques known in the art, such as distillation or urea adduction, to produce a higher potency product with higher concentrations of DHA, EPA, or another fatty acid. The microbial oils can also be used in chemical reactions to produce compounds derived from fatty acids in the oils, such as esters and salts of DHA, EPA, or another fatty acid.

Any thraustochytrid that has been transformed as described herein to grow on xylose can be selected for preparing any of the described compositions based on a desirable attribute of the thraustochytrid, such as but not limited to the cell density of a cell culture of the thraustochytrid, the percentages and types of fatty acids associated with the dry cell of a biomass of the thraustochytrid, the fatty acid profile associated with the biomass and/or the microbial oil of the thraustochytrid, or a combination thereof. In some embodiments, the thraustochytrid biomass or microbial oil comprises a greater percentage of polyunsaturated fatty acids than monounsaturated fatty acids. In some embodiments, the thraustochytrid biomass or microbial oil comprises a greater percentage of omega-3 fatty acids than omega-6 fatty acids. In some embodiments, the thraustochytrid biomass or microbial oil comprises a greater percentage of DHA than other omega-3 fatty acids. In some embodiments, the thraustochytrid biomass comprises a greater percentage of DHA than other polyunsaturated fatty acids. In some embodiments, the thraustochytrid biomass or microbial oil comprises a greater percentage of EPA than other omega-3 fatty acids. In some embodiments, the thraustochytrid biomass or microbial oil comprises a greater percentage of EPA than other polyunsaturated fatty acids.

A composition can include one or more excipients. As used herein, "excipient" refers to a component, or mixture of components, that is used in a composition to give desirable characteristics to the composition, including foods as well as pharmaceutical, cosmetic, and industrial compositions. An excipient can be described as a "pharmaceutically acceptable" excipient when added to a pharmaceutical composition, meaning that the excipient is a compound, material, composition, salt, and/or dosage form which is, within the scope of sound medical judgment, suitable for contact with the tissues of human beings and animals without excessive toxicity, irritation, allergic response, or other problematic complications over the desired duration of contact commensurate with a reasonable benefit/risk ratio. In some embodiments, the term "pharmaceutically acceptable" means approved by a regulatory agency of the Federal or a state government or listed in the U.S. Pharmacopeia or other generally recognized international pharmacopeia for use in animals, and more particularly in humans. Various excipients can be used. In some embodiments, the excipient can be, but is not limited to, an alkaline agent, a stabilizer, an antioxidant, an adhesion agent, a separating agent, a coating agent, an exterior phase component, a controlled-release component, a solvent, a surfactant, a humectant, a buffering agent, a filler, an emollient, or combinations thereof. Excipients in addition to those discussed herein can include excipients listed in, though not limited to, Remington: The Science and Practice of Pharmacy, 21st ed. (2005).

In some embodiments, the composition is a food product. A food product is any food for animal or human consumption, and includes both solid and liquid compositions. A food product can be an additive to animal or human foods. Foods include, but are not limited to, common foods; liquid products, including milks, beverages, therapeutic drinks, and nutritional drinks; functional foods; supplements; nutraceuticals; infant formulas, including formulas for pre-mature infants; foods for pregnant or nursing women; foods for adults; geriatric foods; and animal foods.

In some embodiments, a thraustochytrid biomass or microbial oil can be used directly as or included as an additive within one or more of: an oil, shortening, spread, other fatty ingredient, beverage, sauce, dairy-based or soy-based food (such as milk, yogurt, cheese and ice-cream), a baked good, a nutritional product, e.g., as a nutritional supplement (in capsule or tablet form), a vitamin supplement, a diet supplement, a powdered drink, a finished or semi-finished powdered food product, and combinations thereof.

A partial list of food compositions that can include a microbial oil includes, but is not limited to, soya based products (milks, ice creams, yogurts, drinks, creams, spreads, whiteners); soups and soup mixes; doughs, batters, and baked food items including, for example, fine bakery wares, breakfast cereals, cakes, cheesecakes, pies, cupcakes, cookies, bars, breads, rolls, biscuits, muffins, pastries, scones, croutons, crackers, sweet goods, snack cakes, pies, granola/snack bars, and toaster pastries; candy; hard confectionery; chocolate and other confectionery; chewing gum; liquid food products, for example milks, energy drinks, infant formula, carbonated drinks, teas, liquid meals, fruit juices, fruit-based drinks, vegetable-based drinks; multivitamin syrups, meal replacers, medicinal foods, and syrups; powdered beverage mixes; pasta; processed fish products; processed meat products; processed poultry products; gravies and sauces; condiments (ketchup, mayonnaise, etc.); vegetable oil-based spreads; dairy products; yogurt; butters; frozen dairy products; ice creams; frozen desserts; frozen yogurts; semi-solid food products such as baby food; puddings and gelatin desserts; processed and unprocessed cheese; pancake mixes; food bars including energy bars; waffle mixes; salad dressings; replacement egg mixes; nut and nut-based spreads; salted snacks such as potato chips and other chips or crisps, corn chips, tortilla chips, extruded snacks, popcorn, pretzels, potato crisps, and nuts; specialty snacks such as dips, dried fruit snacks, meat snacks, pork rinds, health food bars, and rice/corn cakes.

In some embodiments, a microbial oil can be used to supplement infant formula. Infant formula can be supplemented with a microbial oil alone or in combination with a physically refined oil derived from an arachidonic acid (ARA)-producing microorganism. An ARA-producing microorganism, for example, is *Mortierella alpina* or *Mortierella* sect. *schmuckeri.* Alternatively, infant formulas can be supplemented with a microbial oil in combination with an oil rich in ARA, including ARASCO® (Martek Biosciences, Columbia, MD).

In some embodiments, the composition is an animal feed. An "animal" means any non-human organism belonging to the kingdom Animalia, and includes, without limitation, aquatic animals and terrestrial animals. The term "animal feed" or "animal food" refers to any food intended for non-human animals, whether for fish; commercial fish; ornamental fish; fish larvae; bivalves; mollusks; crustaceans; shellfish; shrimp; larval shrimp; artemia; rotifers; brine shrimp; filter feeders; amphibians; reptiles; mammals; domestic animals; farm animals; zoo animals; sport animals; breeding stock; racing animals; show animals; heirloom animals; rare or endangered animals; companion animals; pet animals such as dogs, cats, guinea pigs, rabbits, rats, mice, or horses; primates such as monkeys (e.g., cebus, rhesus, African green, patas, cynomolgus, and cercopithecus), apes, orangutans, baboons, gibbons, and chimpanzees; canids such as dogs and wolves; felids such as cats, lions, and tigers; equids such as horses, donkeys, and zebras; food animals such as cows, cattle, pigs, and sheep; ungulates such as deer and giraffes; rodents such as mice, rats, hamsters and guinea pigs; and so on. An animal feed includes, but is not limited to, an aquaculture feed, a domestic animal feed including pet feed, a zoological animal feed, a work animal feed, a livestock feed, or a combination thereof.

In some embodiments, the composition is a feed or feed supplement for any animal whose meat or products are consumed by humans, such as any animal from which meat, eggs, or milk is derived for human consumption. When fed to such animals, nutrients such as LC-PUFAs can be incorporated into the flesh, milk, eggs or other products of such animals to increase their content of these nutrients.

In some embodiments, the composition is a spray-dried material that can be crumbled to form particles of an appropriate size for consumption by zooplankton, artemia, rotifers, and filter feeders. In some embodiments, the zooplankton, artemia, or rotifers fed by the composition are in turn fed to fish larvae, fish, shellfish, bivalves, or crustaceans.

In some embodiments, the composition is a pharmaceutical composition. Suitable pharmaceutical compositions include, but are not limited to, an anti-inflammatory composition, a drug for treatment of coronary heart disease, a drug for treatment of arteriosclerosis, a chemotherapeutic agent, an active excipient, an osteoporosis drug, an anti-depressant, an anticonvulsant, an anti-Helicobacter pylori drug, a drug for treatment of neurodegenerative disease, a drug for treatment of degenerative liver disease, an antibiotic, a cholesterol lowering composition, and a triglyceride lowering composition. In some embodiments, the composition is a medical food. A medical food includes a food that is in a composition to be consumed or administered externally under the supervision of a physician and that is intended for the specific dietary management of a condition, for which distinctive nutritional requirements, based on recognized scientific principles, are established by medical evaluation.

In some embodiments, the microbial oil can be formulated in a dosage form. Dosage forms can include, but are not limited to, tablets, capsules, cachets, pellets, pills, powders and granules, and parenteral dosage forms, which include, but are not limited to, solutions, suspensions, emulsions, and dry powders comprising an effective amount of the microbial oil. It is also known in the art that such formulations can also contain pharmaceutically acceptable diluents, fillers, disintegrants, binders, lubricants, surfactants, hydrophobic vehicles, water soluble vehicles, emulsifiers, buffers, humectants, moisturizers, solubilizers, preservatives, and the like. Administration forms can include, but are not limited to, tablets, dragees, capsules, caplets, and pills, which contain the microbial oil and one or more suitable pharmaceutically acceptable carriers.

For oral administration, the microbial oil can be combined with pharmaceutically acceptable carriers well known in the art. Such carriers enable the microbial oils to be formulated as tablets, pills, dragees, capsules, liquids, gels, syrups, slurries, suspensions, and the like, for oral ingestion by a subject to be treated. In some embodiments, the dosage form is a tablet, pill, or caplet. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl cellulose, sodium carboxymethyl cellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate. Pharmaceutical preparations that can be used orally include, but are not limited to, push-fit capsules made of gelatin, as well as soft, sealed capsules made of gelatin and a plasticizer, such as glycerol or sorbitol.

In some embodiments, the composition is a cosmetic. Cosmetics include, but are not limited to, emulsions, creams, lotions, masks, soaps, shampoos, washes, facial creams, conditioners, make-ups, bath agents, and dispersion liquids. Cosmetic agents can be medicinal or non-medicinal.

In some embodiments, the composition is an industrial composition. In some embodiments, the composition is a starting material for one or more manufactures. A manufacture includes, but is not limited to, a polymer; a photographic photosensitive material; a detergent; an industrial oil; or an industrial detergent. For example, U.S. Patent No. 7,259,006 describes use of DHA-containing fat and oil for production of behenic acid and production of photographic sensitive materials using behenic acid.

### Methods of Producing Biofuels

The present invention is also directed to a method for producing a biofuel from a thraustochytrid of the invention.

As used herein, "biofuel" refers to any fuel that is produced from or using a biomass or a microbial oil of a thraustochytrid of the invention, including, but not limited to, a biodiesel, a renewable diesel, a co-processed renewable diesel, a jet biofuel, a heating oil, and a fuel additive. Any method known in the art can be used to generate a biofuel from the biomasses and microbial oils of the thraustochytrids of the present invention, including but not limited to any methods described in WO 2005/035693, U.S. Publ. No. 2005/0112735, WO 2007/027633, WO 2006/127512, U.S. Publ. No. 2007/0099278, U.S. Publ. No. 2007/0089356, WO 2008/067605, U.S. Publ. No. 2009/0035842, and U.S. Publ. No. 2009/0064567. Any of the biofuels described herein can be blended with a fossil fuel. For example, a biodiesel can be blended with a fossil diesel at ratios of 1%-99%. In some embodiments, a microbial oil of a thraustochytrid of the invention is used as a starting point for production of a biofuel even though it has not been subjected to conventional processing. Examples of conventional processing that can be avoided include refining (e.g., physical refining, silica refining or caustic refining), desolventization, deodorization, winterization, chill filtration, and/or bleaching. Thus, in certain embodiments, the oils have not been subjected to refining, desolventization, deodorization, winterization, chill filtration, bleaching, or a combination thereof.

In some embodiments, the method of producing a biofuel comprises growing a thraustochytrid of the invention in a culture medium comprising xylose as a carbon source to produce a biomass, extracting an oil from the biomass, and producing a biofuel by transesterifying the oil, cracking the oil, processing the oil by thermal depolymerization, adding the oil to a traditional petroleum refining process, or a combination thereof.

In some embodiments, the biofuel is produced by transesterifying the oil. In some embodiments, the biofuel is a biodiesel. Various forms of biodiesel are known and include, but are not limited to, biodiesels described in WO 07/061903, U.S. Pat. No. 7,172,635, EP Pat. No. 1 227 143, WO 02/38709, WO 02/38707, and U.S. Publ. No. 2007/0113467. Transesterification of triglycerides in the oil produces long chain fatty acid esters, i.e., alkyl esters, and can be performed by any method known in the art for the production of biofuels. In some embodiments, the alkyl ether is a methyl ester or an ethyl ester. In some embodiments, the extracting the oil from the thraustochytrid biomass and transesterifying the oil can be performed simultaneously. In some embodiments, the extracting the oil from the thraustochytrid biomass and transesterifying the oil are performed separately. *See,* e.g., U.S. Publ. No. 2009/0064567.

In some embodiments, transesterification is performed using a lower alkyl alcohol and an acid or base catalyst. Alcohols suitable for use in the present invention include any lower alkyl alcohol containing from 1 to 6 carbon atoms (i.e., a C₁₋₆ alkyl alcohol, such as methyl, ethyl, isopropyl, butyl, pentyl, hexyl alcohols and isomers thereof). In some embodiments, the alcohol comprises from 5 wt. % to 70 wt. %, 5 wt. % to 60 wt. %, 5 wt. % to 50 wt. %, 7 wt. % to 40 wt. %, 9 wt. % to 30 wt. %, or 10 wt. % to 25 wt. % of the mixture of the oil composition, the alcohol, and a catalytic base. In some embodiments, the oil composition and the base can be added to either pure ethanol or pure methanol. The amount of alcohol used can vary with the solubility of the oil in the alcohol. Any base known in the art to be suitable for use as a reactant can be used, including bases of the formula RO-M, wherein M is a monovalent cation and RO is an alkoxide of a C₁₋₆ alkyl alcohol. Examples of suitable bases include, but are not limited to, elemental sodium, sodium methoxide, sodium ethoxide, potassium methoxide, and potassium ethoxide. In some embodiments, the base is sodium ethoxide. In some embodiments, the base is added to the reaction with the oil composition and the alcohol in an amount of from 0.05 to 2.0 molar equivalents of triglycerides in the oil, 0.05 to 1.5 molar equivalents, 0.1 to 1.4 molar equivalents, 0.2 to 1.3 molar equivalents, or 0.25 to 1.2 molar equivalents. The oil comprising triglycerides, the alcohol, and the base are reacted together at a temperature and for an amount of time that allows the production of an ester from the fatty acid residues and the alcohol. In some embodiments, the reacting of the composition in the presence of an alcohol and a base is performed at a temperature from 20 °C to 140 °C, from 20 °C to 120 °C, from 20 °C to 110 °C, from 20 °C to 100 °C, or from 20 °C to 90 °C. In some embodiments, the reacting of the composition in the presence of an alcohol and a base is performed at a temperature of at least 20 °C, at least 75 °C, at least 80 °C, at least 85 °C, at least 90 °C, at least 95 °C, at least 105 °C, or at least 120 °C. In some embodiments, the reacting of the composition in the presence of an alcohol and a base is performed at a temperature of 20 °C, 75 °C, 80 °C, 85 °C, 90 °C, 95 °C, 105 °C, or 120 °C. In some embodiments, the reacting of the composition in the presence of an alcohol and a base is performed for a time from 2 hours to 36 hours, from 3 hours to 36 hours, from 4 hours to 36 hours, from 5 hours to 36 hours, or from 6 hours to 36 hours. In some embodiments, the reacting of the composition in the presence of an alcohol and a base is performed for 0.25, 0.5, 1, 2, 4, 5, 5.5, 6, 6.5, 7, 7.5, 8, 8.5, 10, 12, 16, 20, 24, 28, 32, or 36 hours. In some embodiments, the reacting of the oil composition, alcohol, and base can be conducted by refluxing the components to produce the fatty acid esters, such as PUFA esters. In some embodiments, the reacting of the oil composition can be carried out at a temperature that does not result in the refluxing of the reaction components. For example, carrying out the reacting of the oil composition under pressures greater than atmospheric pressure can increase the boiling point of the solvents present in the reaction mixture. Under such conditions, the reaction can occur at a temperature at which the solvents would boil at atmospheric pressure, but would not result in the refluxing of the reaction components. In some embodiments, the reaction is conducted at a pressure from 5 pounds per square inch (psi) to 20 psi; from 7 psi to 15 psi; or from 9 psi to 12 psi. In some embodiments, the reaction is conducted at a pressure of 7 psi, 8 psi, 9 psi, 10 psi, 11 psi, or 12 psi. Reactions conducted under pressure can be carried out at the reaction temperatures listed above. In some embodiments, reactions conducted under pressure can be carried out at least from 20 °C to 140 °C, from 20 °C to 120 °C, from 20 °C to 110 °C, from 20 °C to 100 °C, or from 20 °C to 90 °C. In some embodiments, reactions conducted under pressure can be carried out at least 70 °C, at least 75 °C, at least 80 °C, at least 85 °C, or at least 90 °C. In some embodiments, reactions conducted under pressure can be carried out at 70 °C, 75 °C, 80 °C, 85 °C, or 90 °C.

Reduced amounts of PUFA in oil used for producing a biofuel, such as a biodiesel, can increase the energy density of the biofuel and can reduce the number of sites for potential oxidation or sulfation. In some embodiments, the thraustochytrid biomass or microbial oil, or triglyceride fraction thereof, comprises a greater percentage of monounsaturated fatty acids than polyunsaturated fatty acids. In some embodiments, the microbial oil is fractionated to remove polyunsaturated fatty acids. In some embodiments, the oil is hydrogenated in order to convert polyunsaturated fatty acids to monounsaturated fatty acids. To ensure that greater percentages of microalgal oil-derived biodiesel can be burned, any method of partial or total oil hydrogenation, as is routine in the manufacture of margarines, can be used. In some embodiments, thraustochytrids that produce low levels of PUFAs are used to produce the microbial oils. In some embodiments, thraustochytrids of the invention are selected that produce greater amounts of monounsaturated fatty acids than polyunsaturated fatty acids. In some embodiments, less than 50% of unsaturated fatty acids in the biological oil are PUFAs. In some embodiments, the unsaturated fatty acids in the biological oil contain less than 40%, less than 30%, less than 20%, less than 10%, or less than 5% PUFAs. In some embodiments, the microbial oil comprises less than 50%, less than 40%, less than 30%, less than 20%, less than 10%, or less than 5% by weight of PUFAs.

In addition to the transesterification methods described above, other techniques of reducing the viscosity of the microbial oils can also be used to produce biofuels. These techniques include, but are not limited to, the use of lipases, supercritical methanol catalysis, and the use of whole-cell systems involving cytoplasmic overexpression of lipases in a host cell followed by permeabilization of the host to allow catalysis of transesterification of triglycerides within the cytoplasm. In some embodiments, a thraustochytrid cell of the invention also comprises a polynucleotide sequence encoding a lipase for catalysis of transesterification of triglycerides within the cytoplasm. *See,* for example, U.S. Pat. No. 7,226,771, U.S. Publ. No. 2004/0005604, WO 03/089620, WO 05/086900, U.S. Publ. No. 2005/0108789, WO 05/032496, WO 05/108533, U.S. Pat. No. 6,982,155, WO 06/009676, WO 06/133698, WO 06/037334, WO 07/076163, WO 07/056786, and WO 06/124818.

In some embodiments, the biofuel is produced by cracking the oil. In some embodiments, the biofuel is a jet biofuel. "Cracking" is understood in the art to describe the reduction of the chain length of fatty acids in an oil by methods such as those used in the oil industry. In some embodiments, the presence of a significant amount of polyunsaturated fatty acid in the oil will provide greater flexibility and variety for the production of hydrocarbons, since the multiple sites of unsaturation in a polyunsaturated fatty acid provide multiple sites for cleavage to make hydrocarbons. For example, certain jet fuels require hydrocarbons with two to eight carbons. Polyunsaturated fatty acids can be cleaved through known processes in the art, such as cracking, to produce shorter hydrocarbons of various chain lengths.

In some embodiments, the biofuel is produced by thermal depolymerization. In some embodiments, the biofuel is a renewable diesel. As used herein, thermal depolymerization includes any process for the production of renewable diesel using superheated water.

In some embodiments, the biofuel is produced by adding the oil to a petroleum refining process during the production of diesel fuel. In some embodiments, the biofuel is a co-processed renewable diesel.

Having generally described this invention, a further understanding can be obtained by reference to the examples provided herein. These examples are for purposes of illustration only and are not intended to be limiting.

### EXAMPLE 1

### Construction of Xylose Transporter, Xylose Reductase, Xylulose Kinase, Xylitol Dehydrogenase, and Xylose Isomerase Expression Vectors

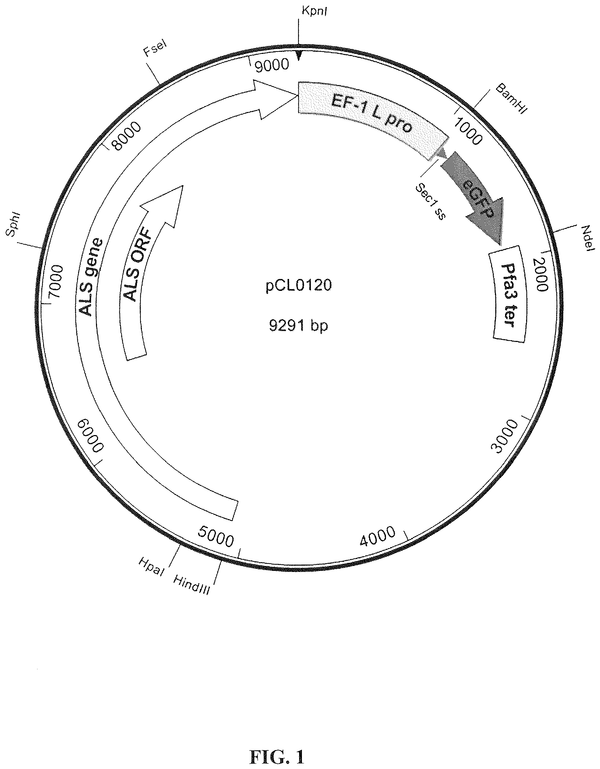

The vector pAB0018 (ATCC Accession No. PTA-9616) was digested with HindIII, treated with mung bean nuclease, purified, and then further digested with KpnI generating four fragments of various sizes. A fragment of 2552 bp was isolated by standard electrophoretic techniques in an agar gel and purified using commercial DNA purification kits. A second digest of pAB0018 with Pmel and KpnI was then performed. A fragment of 6732 bp was isolated and purified from this digest and ligated to the 2552 bp fragment. The ligation product was then used to transform commercially supplied strains of competent DH5-α *E. coli* cells (Invitrogen) using the manufacturer's protocol. Plasmids from ampicillin-resistant clones were propagated, purified, and then screened by restriction digests or PCR to confirm that the ligation generated the expected plasmid structures. One verified plasmid was designated pCL0120. *See* **FIG. 1** and SEQ ID NO:12.

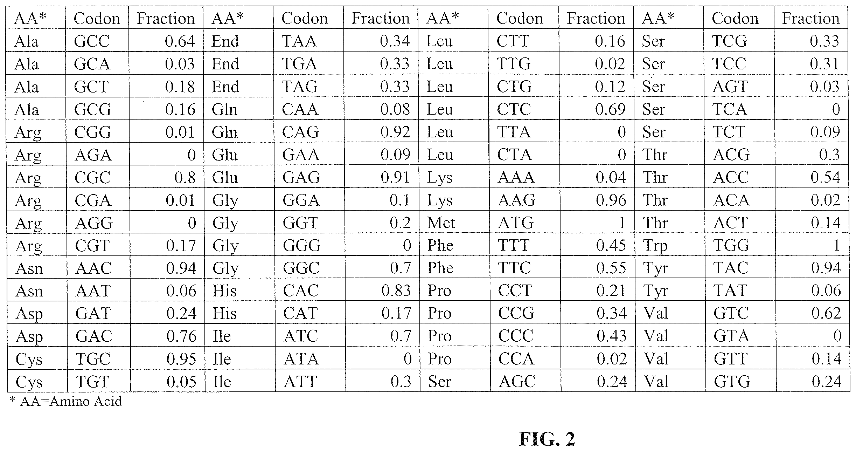

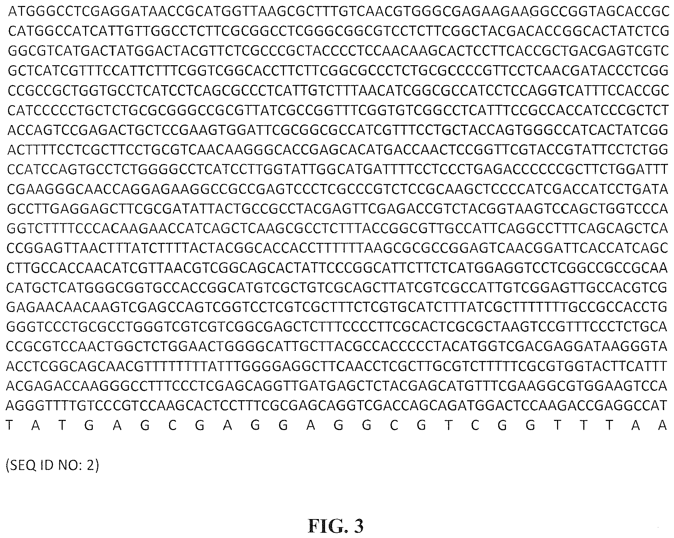

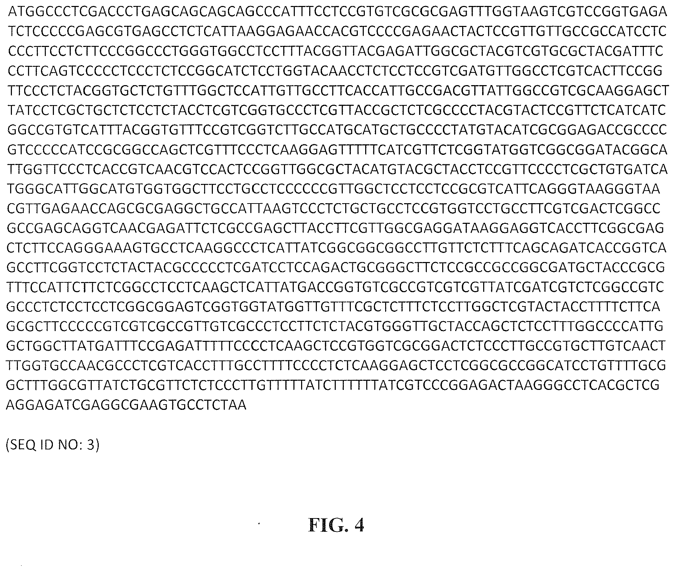

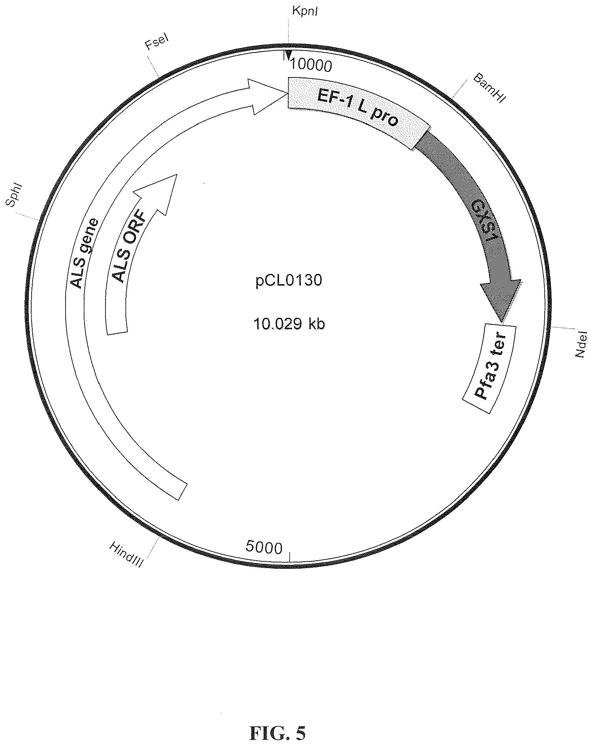

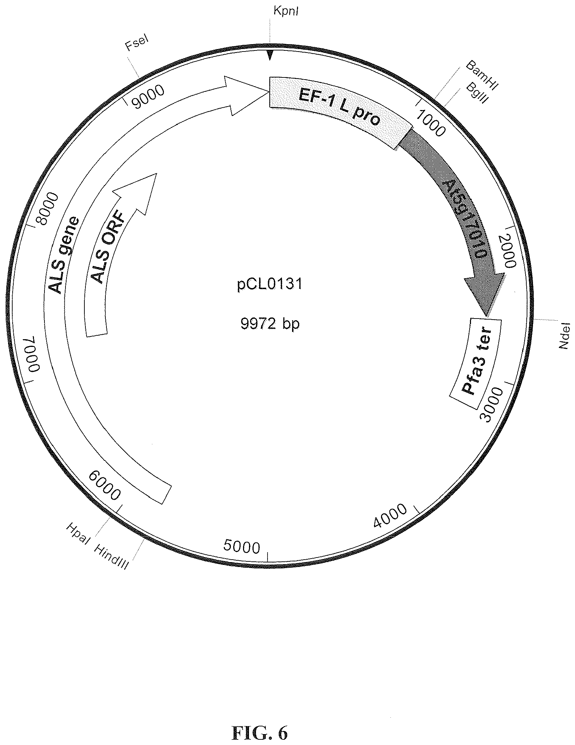

Sequences encoding the *Candida intermedia* xylose transporter protein GXS1 (GenBank Accession No. AJ875406) and the *Arabidopsis thaliana* xylose transporter protein At5g17010 (GenBank Accession No. BT015128) were sent to Blue Heron Biotechnology (Bothell,WA) for codon optimization and synthesis as guided by the *Schizochytrium* codon usage table shown in **FIG. 2****.** SEQ ID NO:2 is the codon-optimized nucleic acid sequence of GSX1 (**FIG. 3**), while SEQ ID NO:3 is the codon-optimized nucleic acid sequence of At5g17010 (**FIG. 4**). SEQ ID NO:2 and SEQ ID NO:3 were respectively cloned into pCL0120 using the 5' and 3' restriction sites BamHI and NdeI for insertion and ligation according to standard techniques. Maps of the resulting vectors, pCL0130 and pCL0131 are shown in **FIG. 5** and **FIG. 6**, respectively. Sequences for the vectors pCL0130 and pCL0131 are provided as SEQ ID NOs: 14 and 15, respectively.

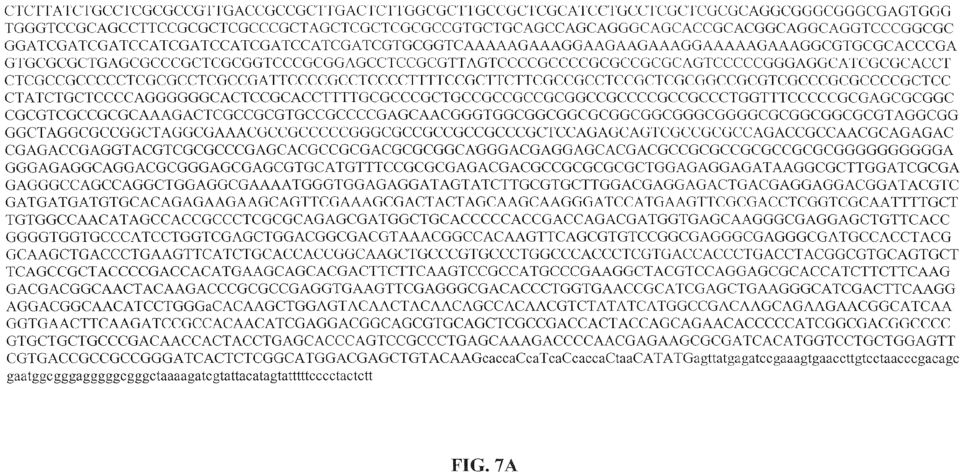

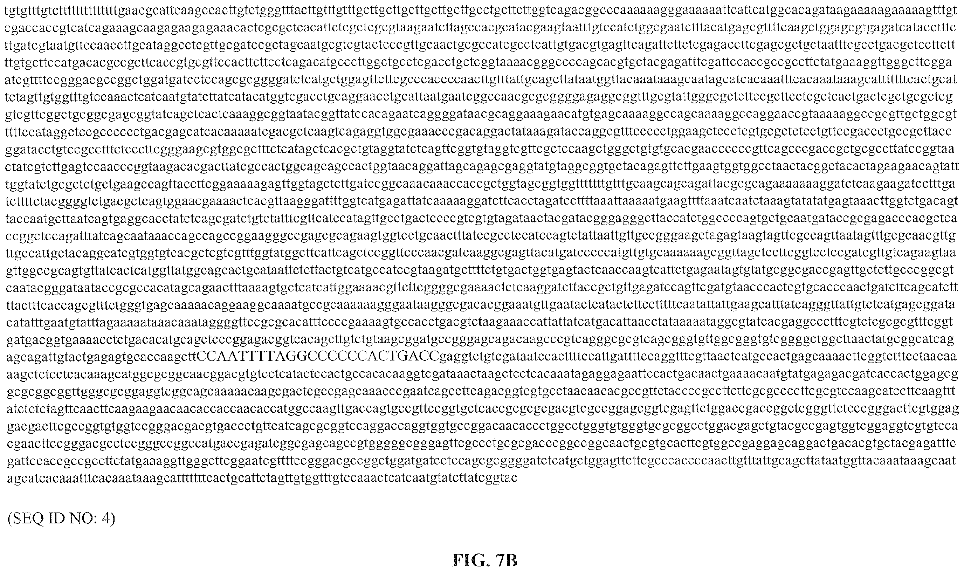

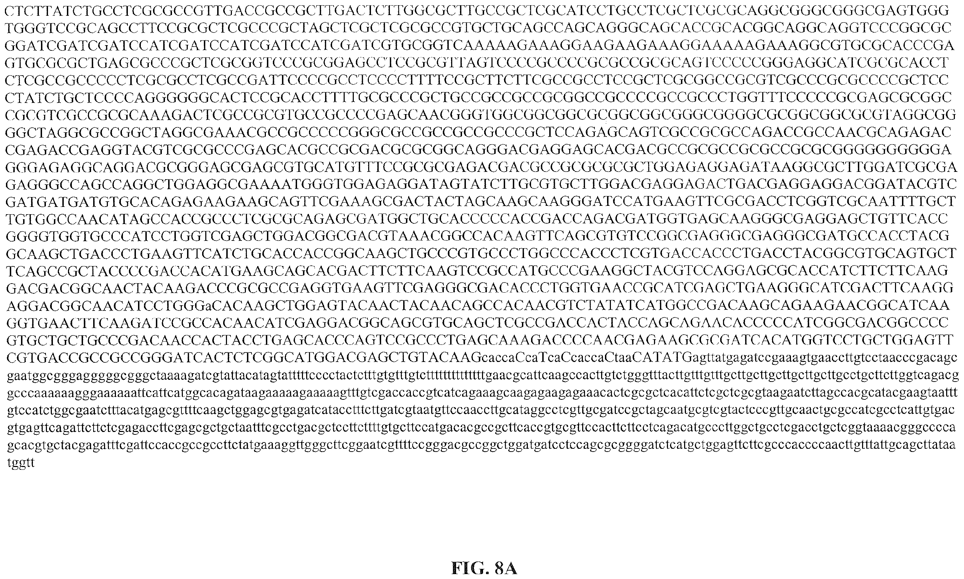

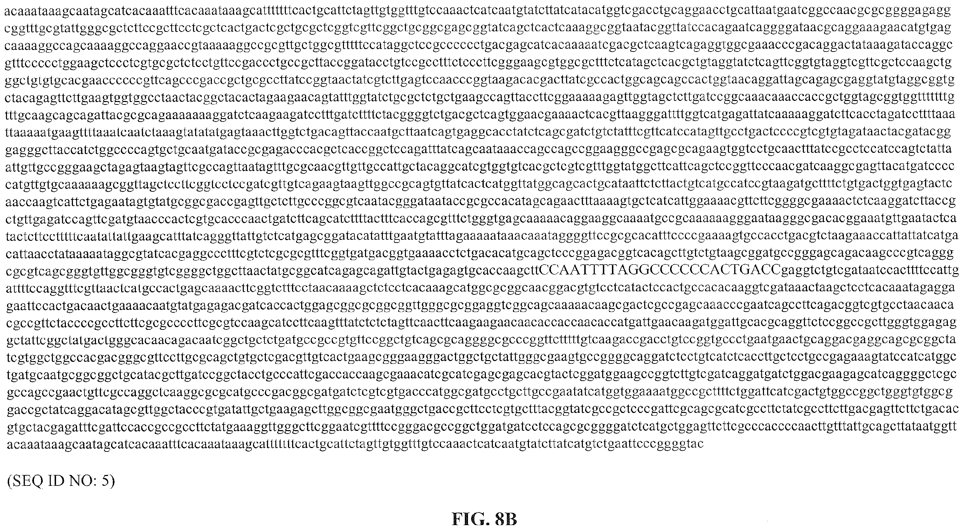

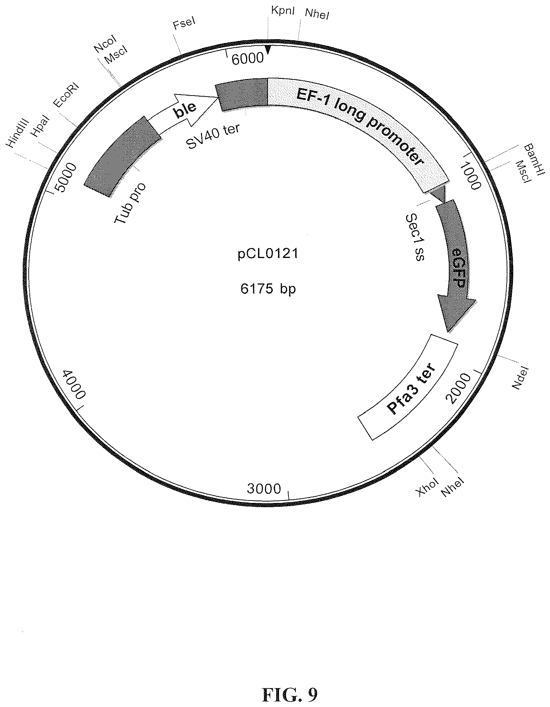

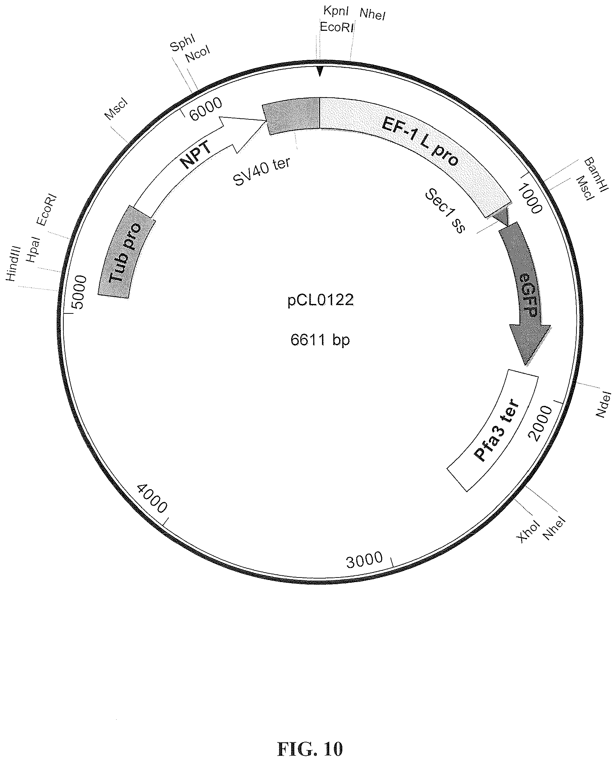

Vectors pCL0121 and pCL0122 were created by ligating a 5095 bp fragment which had been liberated from pCL0120 by digestion with HindIII and KpnI to synthetic selectable marker cassettes designed to confer resistance to either ZEOCIN™ or paromomycin. These cassettes were comprised of an alpha tubulin promoter to drive expression of either the sh ble gene (for ZEOCIN™) or the npt gene (for paromomycin). The transcripts of both selectable marker genes were terminated by an SV40 terminator. The full sequence of vectors pCL0121 (SEQ ID NO:4) and pCL0122 (SEQ ID NO:5) are shown in **FIG. 7** and **8**, respectively. Maps of vectors pCL0121 and pCL0122 are shown in **FIG. 9** and **10**, respectively.

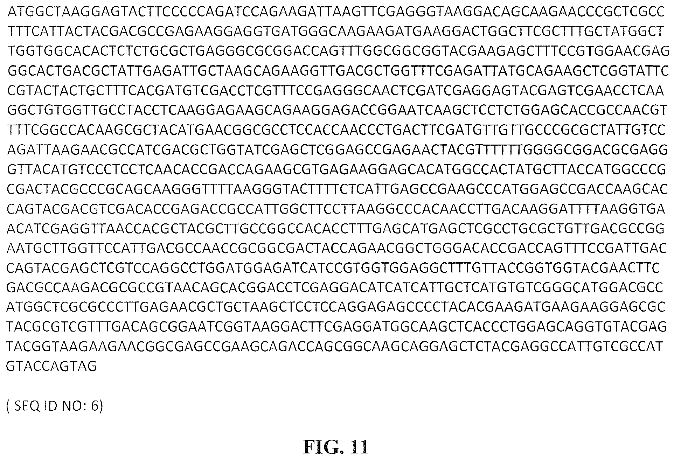

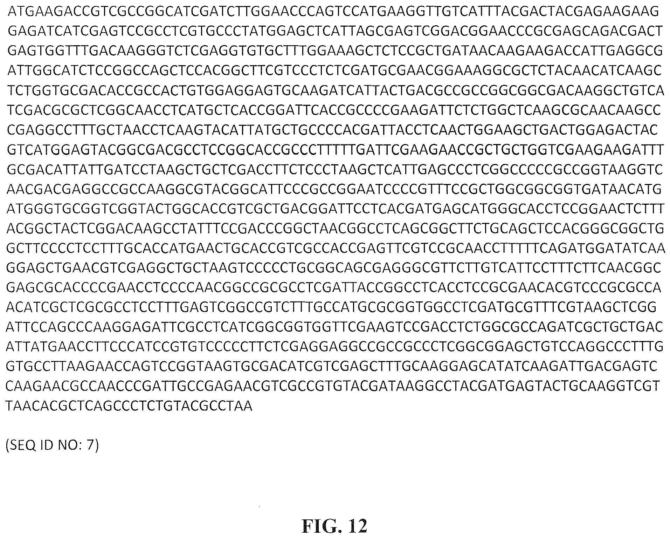

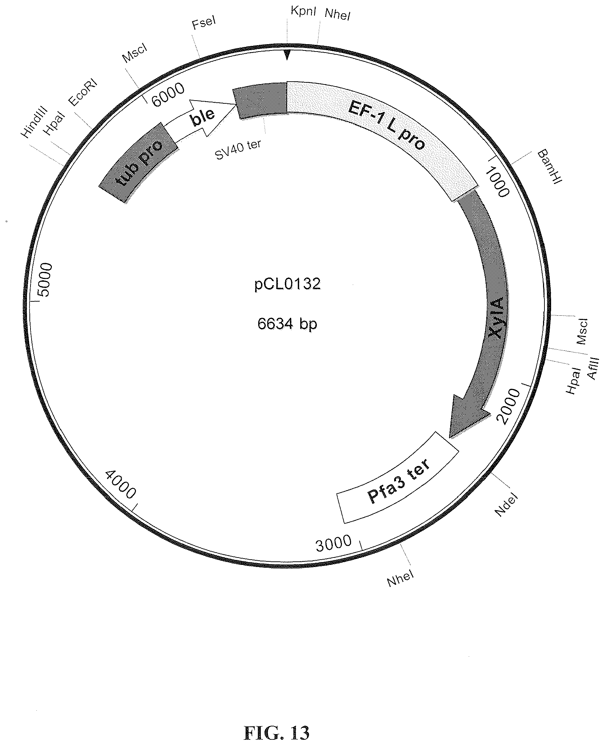

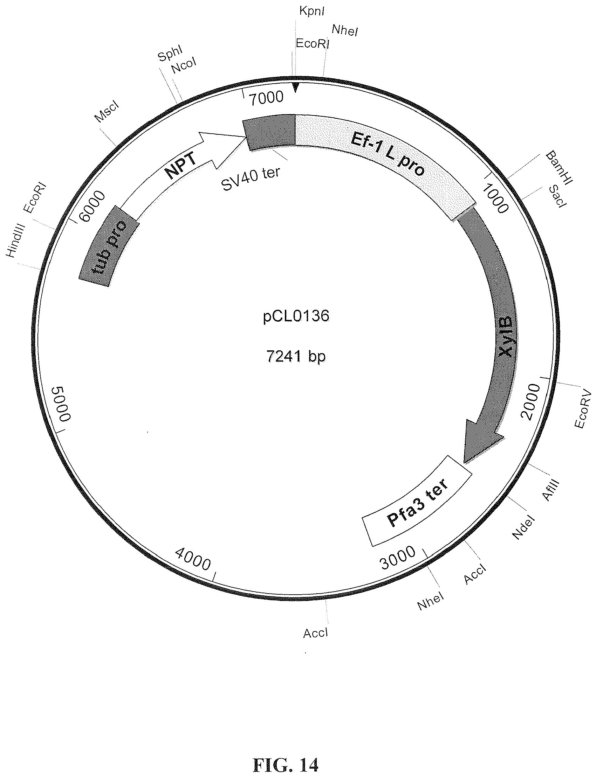

Sequences encoding the *Piromyces* sp. E2 xylose isomerase (CAB76571) and *Piromyces* sp. E2 xylulose kinase (AJ249910) were sent to Blue Heron Biotechnology (Bothell,WA) for codon optimization and synthesis as guided by the *Schizochytrium* codon usage table shown in **FIG. 2****.** "Xy1A" (SEQ ID NO:6) is the codon-optimized nucleic acid sequence of CAB76571 (**FIG. 11**), while "Xy1B" (SEQ ID NO:7) is the codon-optimized nucleic acid sequence of AJ249910 (**FIG. 12**). SEQ ID NO:6 was cloned into the vector pCL0121 resulting in the vector designated pCL0132 **(****FIG. 13** and SEQ ID NO:16). SEQ ID NO:7 was cloned into the vector pCL0122 resulting in the vector designated pCL0136 (**FIG. 14** and SEQ ID NO:20).

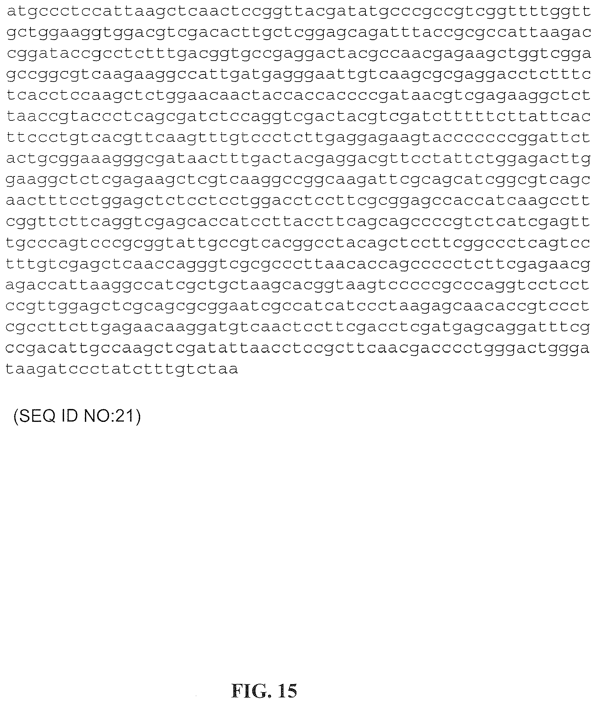

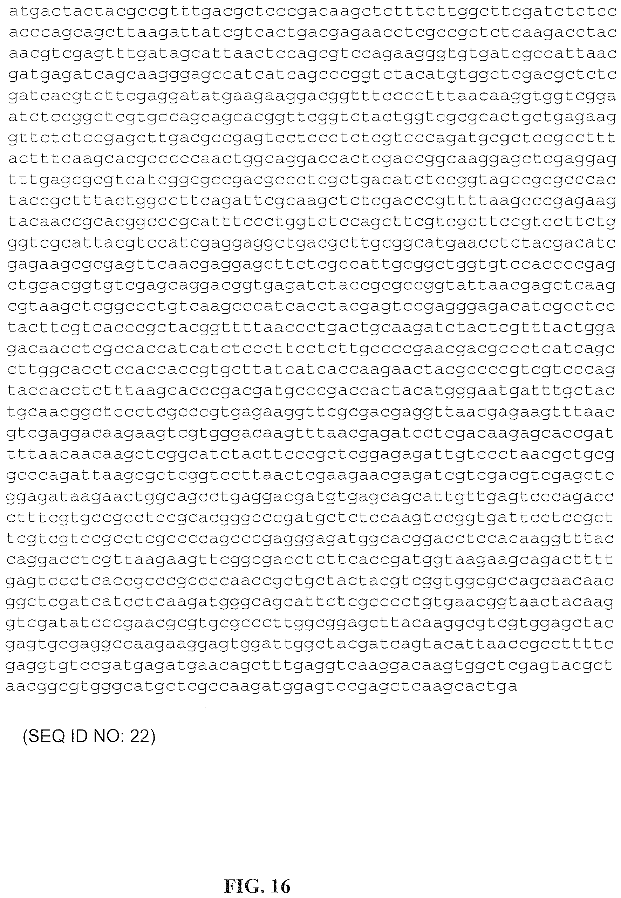

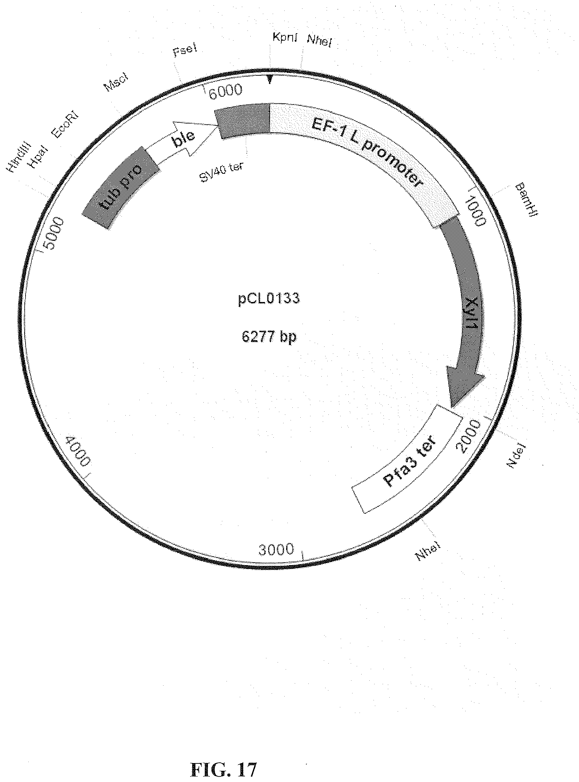

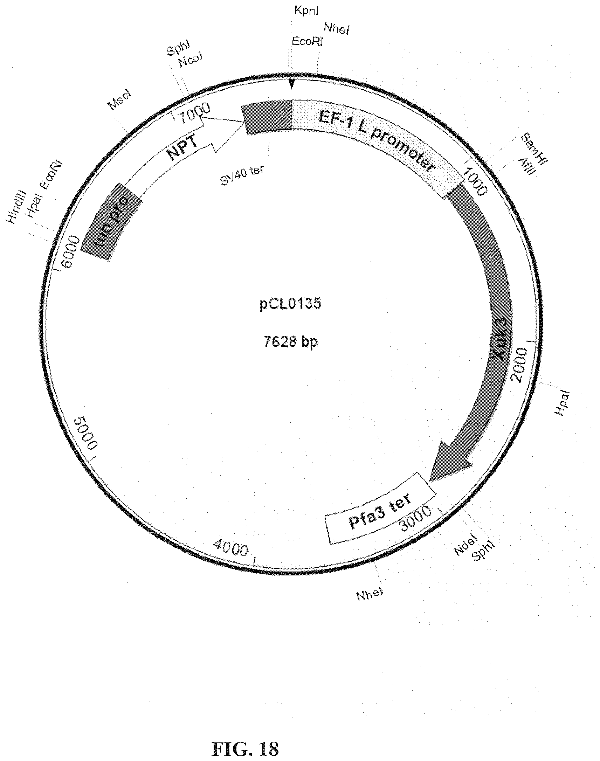

Sequences encoding the *Pichia stipitis* xylose reductase (X59465) and *Pichia stipitis* xylulose kinase (AF127802) were sent to Blue Heron Biotechnology (Bothell,WA) for codon optimization and synthesis as guided by the *Schizochytrium* codon usage table shown in **FIG. 2****.** "Xyl1" (SEQ ID NO:21) is the codon-optimized nucleic acid sequence of X59465 (**FIG. 15**), while "Xuk3" (SEQ ID NO:22) is the codon-optimized nucleic acid sequence of AF127802 (**FIG. 16**). SEQ ID NO:21 was cloned into the vector pCL0121 resulting in the vector designated pCL0133 (**FIG. 17** and SEQ ID NO: 17). SEQ ID NO:22 was cloned into the vector pCL0122, resulting in the vector designated pCL0135 (**FIG. 18** and SEQ ID NO:19).

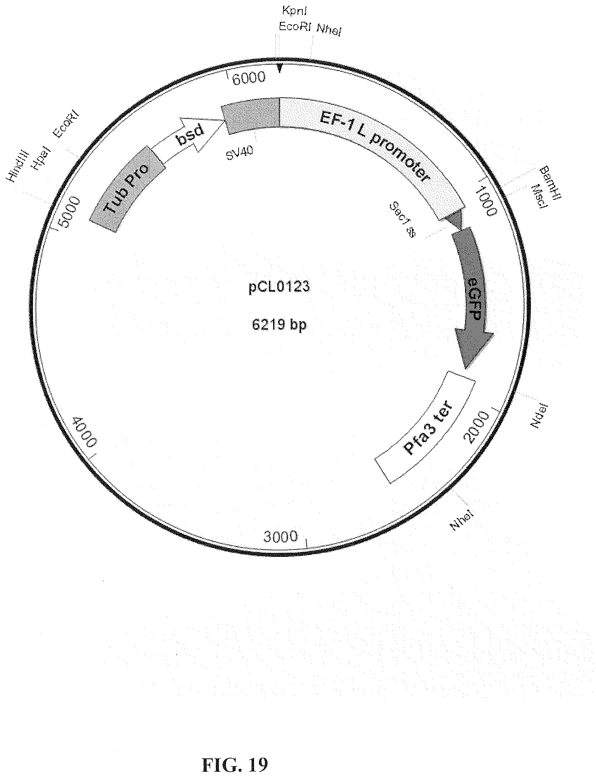

Vector pCL0123 was created by ligating a 5095 bp fragment which had been liberated from pCL0120 by digestion with HindIII and KpnI to a synthetic selectable marker cassette designed to confer resistance to ZEOCIN™. The cassette was comprised of an alpha tubulin promoter to drive expression of the sh ble gene (for ZEOCIN™). The transcript of the selectable marker gene was terminated by an SV40 terminator. A map of vector pCL0123 is shown in **FIG. 19** and SEQ ID NO: 13.

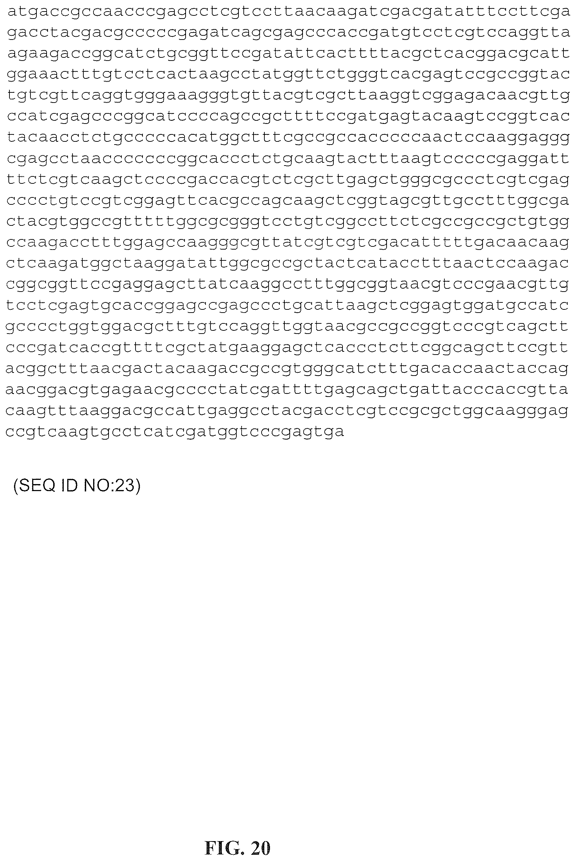

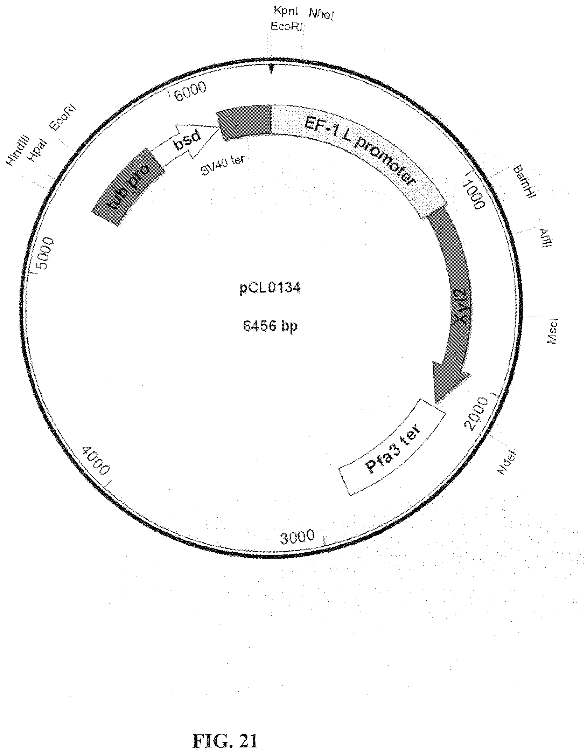

Sequences encoding the *Pichia stipitis* Xylitol dehydrogenase (X55392) was sent to Blue Heron Biotechnology (Bothell,WA) for codon optimization and synthesis as guided by the *Schizochytrium* codon usage table shown in **FIG. 2**. "Xyl2" (SEQ ID NO:23) is the codon-optimized nucleic acid sequence of X55392 (**FIG. 20**). SEQ ID NO:23 was cloned into the vector pCL0123 resulting in the vector designated pCL0134 (**FIG. 21** and SEQ ID NO: 18).

### EXAMPLE 2

### Transformation of Schizochytrium with Xylose Transporter, Xylose Isomerase, Xylose Reductase, Xylitol Dehydrogenase, or Xylulose Kinase Expression Vectors

*Schizochytrium* wild type strain ATCC 20888 was individually transformed with vectors pCL0130 (*Candida intermedia* xylose transporter), pCL0131 (*Arabidopsis thaliana* xylose transporter), pCL0132 (*Piromyces* sp. E2 xylose isomerase), pCL0133 (*Pichia stipitis* xylose reductase), pCL0134 (*Pichia stipitis* Xylitol dehydrogenase), pCL0135 (*Pichia stipitis* xylulose kinase), or pCL0136 (*Piromyces* sp. E2 xylulose kinase) using electroporation with enzyme pretreatment as described below.

*Electroporation with enzyme pretreatment -* Cells were grown in 50 mL of M50-20 media (*see* U.S. Publ. No. 2008/0022422) on a shaker at 200 rpm for 2 days at 30°C. The cells were diluted at 1:100 into M2B media (see following paragraph) and grown overnight (16-24 h), attempting to reach mid-log phase growth (OD₆₀₀ of 1.5-2.5). The cells were centrifuged in a 50 mL conical tube for 5 min at about 3000 x g. The supernatant was removed and the cells were resuspended in 1 M mannitol, pH 5.5, in a suitable volume to reach a final concentration of 2 OD₆₀₀ units. 5 mL of cells were aliquoted into a 25 mL shaker flask and amended with 10 mM CaCl₂ (1.0 M stock, filter sterilized) and 0.25 mg/mL Protease XIV (10 mg/mL stock, filter sterilized; Sigma-Aldrich, St. Louis, MO). Flasks were incubated on a shaker at 30°C and about 100 rpm for 4 h. Cells were monitored under the microscope to determine the degree of protoplasting, with single cells desired. The cells were centrifuged for 5 min at about 2500 x g in round-bottom tubes (*i.e.,* 14 mL Falcon™ tubes, BD Biosciences, San Jose, CA). The supernatant was removed and the cells were gently resuspended with 5 mL of ice cold 10% glycerol. The cells were recentrifuged for 5 min at about 2500 x g in round-bottom tubes. The supernatant was removed and the cells were gently resuspended with 500 µL of ice cold 10% glycerol, using wide-bore pipette tips. 90 µL of cells were aliquoted into a prechilled electro-cuvette (Gene Pulser® cuvette - 0.1 cm gap or 0.2 cm gap, Bio-Rad, Hercules, CA). 1 µg to 5 µg of DNA (in less than or equal to a 10 µL volume) was added to the cuvette, mixed gently with a pipette tip, and placed on ice for 5 min. Cells were electroporated at 200 ohms (resistance), 25 µF (capacitance), and either 250V (for 0.1 cm gap) or 500V (0.2 cm gap). 0.5 mL of M50-20 media was added immediately to the cuvette. The cells were then transferred to 4.5 mL of M50-20 media in a 25 mL shaker flask and incubated for 2-3 h at 30°C and about 100 rpm on a shaker. The cells were centrifuged for 5 min at about 2500 x g in round bottom tubes. The supernatant was removed and the cell pellet was resupended in 0.5 mL of M50-20 media. Cells were plated onto an appropriate number (2 to 5) of M2B plates with appropriate selection and incubated at 30°C.

M2B medium consisting of 10 g/L glucose, 0.8 g/L (NH₄)₂SO₄, 5 g/L Na₂SO₄, 2 g/L MgSO₄•7H₂O, 0.5 g/L KH₂PO₄, 0.5 g/L KCl, 0.1 g/L CaCl₂•2H₂O, 0.1 M MES (pH 6.0) 0.1% PB26 metals , and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO₄•7H₂O, 1 g/L MnCl₂•₄ H₂O, 800 mg/mL ZnSO₄•H₂O, 20 mg/mL CoCl₂•H₂O, 10 mg/mL Na₂MoO₄•2H₂O, 600 mg/mL CuSO₄•5H₂O, and 800 mg/mL NiSO₄•6H₂O. PB26 stock solutions were filter sterilized separately and added to the broth after autoclaving. Glucose, KH₂PO₄, and CaCl₂•2H₂O were each autoclaved separately from the remainder of the broth ingredients before mixing to prevent salt precipitation and carbohydrate caramelizing. All medium ingredients were purchased from Sigma Chemical (St. Louis, MO).

The transformants were selected for growth on solid media containing the appropriate antibiotic. Between 20 and 100 primary transformants of each vector were re-plated to "xylose-SSFM" solid media which is the same as SSFM (described below) except that it contains xylose instead of glucose as a sole carbon source, and no antibiotic were added. No growth was observed for any clones resulting from transformation with any individual vector under these conditions.

SSFM media: 50 g/L glucose, 13.6 g/L Na₂SO₄, 0.7 g/L K₂SO₄, 0.36 g/L KCl, 2.3 g/L MgSO₄•7H₂O, 0.1M MES (pH 6.0), 1.2 g/L (NH₄)₂SO₄, 0.13 g/L monosodium glutamate, 0.056 g/L KH₂PO₄, and 0.2 g/L CaCl₂•2H₂O. Vitamins were added at 1 mL/L from a stock consisting of 0.16 g/L vitamin B12, 9.7 g/L thiamine, and 3.3 g/L Ca-pantothenate. Trace metals were added at 2 mL/L from a stock consisting of 1 g/L citric acid, 5.2 g/L FeSO₄•7H₂O, 1.5 g/L MnCl₂•₄H₂O, 1.5 g/L ZnSO₄•7H ₂O, 0.02 g/L CoCl₂•6H₂O, 0.02 g/L Na₂MoO₄•2H₂O, 1.0 g/L CuSO₄•5H₂O, and 1.0 g/L NiSO₄•6H₂O, adjusted to pH 2.5.

gDNA from primary transformants of pCL0130 and pCL0131 was extracted and purified and used as a template for PCR to check for the presence of the transgene.

*Genomic DNA Extraction Protocol for Schizochytrium -* The *Schizochytrium* transformants were grown in 50 ml of media. 25 ml of culture was aseptically pipetted into a 50 ml conical vial and centrifuge for 4 minutes at 3000 x g to form a pellet. The supernatant was removed and the pellet stored at -80 °C until use. The pellet was resuspended in approximately 4-5 volumes of a solution consisting of 20 mM Tris pH 8, 10 mM EDTA, 50 mM NaCl, 0.5% SDS and 100 ug/ml of Proteinase K in a 50 ml conical vial. The pellet was incubated at 50 °C with gentle rocking for 1 hour. Once lysed, 100 ug/ml of RNAse A was added and the solution was rocked for 10 minutes at 37°C. Next, 2 volumes of phenol:cholorform:isoamyl alcohol was added and the solution was rocked at room temperature for 1 hour and then centrifuged at 8000 x g for 15 minutes. The supernatant was transferred into a clean tube. Again, 2 volumes of phenol:cholorform:isoamyl alcohol was added and the solution was rocked at room temperature for 1 hour and then centrifuged at 8000 x g for 15 minutes and the supernatant was transferred into a clean tube. An equal volume of chloroform was added to the resulting supernatant and the solution was rocked at room temperature for 30 minutes. The solution was centrifuged at 8000 x g for 15 minutes and the supernatant was transferred into a clean tube. An equal volume of chloroform was added to the resulting supernatant and the solution was rocked at room temperature for 30 minutes. The solution was centrifuged at 8000 x g for 15 minutes and the supernatant was transferred into a clean tube. 0.3 volumes of 3M NaOAc and 2 volumes of 100% EtOH to were added to the supernatant, which was rocked gently for a few minutes. The DNA was spooled with a sterile glass rod and dip DNA into 70% EtOH for 1-2 minutes. The DNA was transferred into a 1.7 ml microfuge tube and allow to air dry for 10 minutes. Up to 0.5 ml of prewarmed elution buffer (Tris buffer, 10 mM, pH 8.0) was added to the DNA and placed at 4 °C overnight.

Alternatively, after the RNAase incubation, the DNA was further purified using a Qiagen Genomic tip 500/G column (Qiagen, Inc USA, Valencia, CA), following the manufacturers protocol.

*PCR -* The primers used for detecting the GXS1 transgene were 5'CL0130 (CCTCGGGCGGCGTCCTCTT) (SEQ ID NO:8) and 3'CL0130 (GGCGGCCTTCTCCTGGTTGC) (SEQ ID NO:9). The primers used for detecting the At5g17010 transgene were 5'CL0131 (CTACTCCGTTGTTGCCGCCATCCT) (SEQ ID NO:10) and 3'CL0131 (CCGCCGACCATACCGAGAACGA) (SEQ ID NO:11). Of 10 clones resulting from the pCL0130 transformation, 4 were found to harbor the GSX1 transgene; and of 10 clones resulting from the pCL0131 transformation, 7 were found to harbor the At5g17010 transgene. One clone from each of these pCL0130 and pCL0131 positive transformants was selected for transformation with a second vector selected from pCL0132, pCL0133, pCL0134, pCL0135, or pCL0136 to result in the following combinations: pCL0130+pCL0132, pCL0130+pCL0133, pCL0130+pCL0134, pCL0130+pCL0135, pCL0130+pCL0136, pCL0131+pCL0132, pCL0131+pCL0133, pCL0131+pCL0134, pCL0131+pCL0135, and pCL0131+pCL0136. After transformation with the second vector, each culture was plated to SSFM with antibiotics or to xylose-SSFM directly. Of the many colonies which appeared after 4-12 days of growth on SSFM plates with antibiotics, 5 clones from each transformation were re-plated to xylose-SSFM. No growth was observed for any of the clones resulting from co-transformation of either pCL0130 or pCL0131 with any one of the other vectors on xylose-SSFM solid media plates.

### EXAMPLE 3

### Expression of Xylose Transporters in Schizochytrium

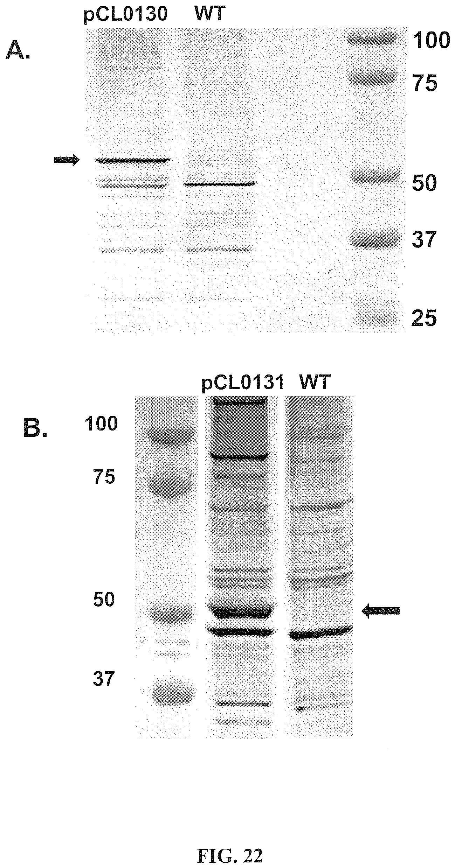

Supernatants of 50 mL shake-flask cultures grown in sPFM for 3 days were collected after cultures were centrifuged at 5000 x g. sPFM media is described in Table 1, below. Culture pellets were lysed for extractions of proteins as previously described. *See* U.S. Publ. No. 2010/0233760. SDS-PAGE of culture pellet lysates was performed and gels were either stained directly with Coomassie dye or used for electrotransfer to PVDF membranes. After transfer, PVDF membranes were used for Western blotting according to common procedures. *See,* e.g., Sambrook *et al.* Briefly, membranes were probed with a 1:500 dilution of primary antisera derived from rabbits, which had been immunized by injection (Open Biosystems Products, Huntsville, AL) with a preparation of xylose transporter peptide conjugated to keyhole limpet hemocyanin (KLH). The specific synthetic peptides conjugated to KLH were RLRKLPIDHPDSLEELRD (SEQ ID NO:24) - "130-pl" or ETKGLTLEEIEAKCL (SEQ ID NO:25) - "131-p2 (Open Biosystems Products, Huntsville, AL) for generating antisera specific to either SEQ ID NO:2 (C. *albicans* GSX1) or SEQ ID NO:3 (*A. thaliana* At5g17010), respectively. The antisera was then followed by addition of a secondary, mouse anti-rabbit IgG monoclonal antibody conjugated to alkaline phosphatase (Promega Corporation, Madison, WI). BCIP/NBT reagent was then applied to the membrane to develop the signal. The Western blot of culture pellet lysates from a clone transformed with pCL0130 and wild type *Schizochytrium* strain ATCC 20888 are shown in **FIG. 22** **A.** The Western blot of culture pellet lysates from a clone transformed with pCL0131 and wild type *Schizochytrium* strain ATCC 20888 are shown in **FIG. 22** **B**. In each example, of all the bands in either wild type or transformant lysates, only one was found to be unique to a given transformant and in each case this unique band corresponded to the predicted size of the expressed xylose transporter.

**Table 1: sPFM Media**

| **Component** | **Amount per liter** |
|---|---|
| Na₂SO₄ | 13.62 g |
| K₂SO₄ | 0.72 g |
| KCl | 0.56 g |
| MgSO₄·7H₂O | 2.27 g |
| (NH₄)2SO₄ | 3 g |
| CaCl₂·2H₂O* | 0.19 g |
| MSG | 3 g |
| MES (100 mM) pH 6 | 21.4 g |
| KH₂O₄* | 0.4 g |
| Glucose* | 50 g |
| Vitamin B12* | 0.16 mg |
| Thiamine* | 9.75 mg |
| CaPantothenate* | 3.33 mg |
| Citric Acid* | 2 mg |
| FeSO₄·7H₂O* | 10.3 mg |
| MnCl₂·4H₂O* | 3.1 mg |
| ZnSO₄·7H₂O* | 1.93 mg |
| CoCl₂·6H₂O* | 0.04 mg |
| Na₂MoO₄·2H₂O* | 0.04 mg |
| CuSO₄·5H₂O* | 2.07 mg |
| NiSO₄·6H₂O* | 2.07 mg |
| pH to 2.5 with HCl | |

| | |
|---|---|
| * Added after autoclaving. | |

### EXAMPLE 4

### Growth on Xylose of Schizochytrium Transformed with Xylose Transporter, Xylose Isomerase, and Xylulose Kinase Expression Vectors

Co-transformations of *Schizochytrium* wild type strain ATCC 20888 were performed with two different mixtures of vectors. The mixtures contained either the *Candida intermedia* xylose transporter (pCL0130) or the *Arabidopsis thaliana* xylose transporter (pCL0131) with two additional vectors: a vector containing *Piromyces* sp. E2 xylose isomerase (pCL0132) and a vector containing *Piromyces* sp. E2 xylose kinase (pCL0136). One combination of vectors, termed the 130 Piromyces Series, included pCL0130, pCL0132, and pCL0136. Another combination of vectors, termed the 131 Piromyces Series, included pCL0131, pCL0132, and pCL0136. Transformants were plated directly on solid xylose SSFM media and after 3-5 weeks colonies were picked and further propagated in liquid xylose-SSFM. Two colonies were recovered from the 130 Piromyces Series transformations and four colonies from the 131 Piromyces Series transformations. Several rounds of serial transfers in xylose-containing liquid media improved growth rates of the transformants. Transformants of the 130 Piromyces Series and the 131 Piromyces Series were also replated to solid SSFM media containing either sulfometum methyl (SMM), ZEOCIN™, or paromomycin. All transformants plated to these media were resistant to each antibiotic tested, indicating that transformants harbored all three of their respective vectors. *Schizochytrium* cells transformed with a xylose transporter, a xylose isomerase, and a xylulose kinase were able to grow in solid and liquid SSFM media containing xylose as a sole carbon source.

One clone from the Piromyces 130 Series and one clone from the Piromyces 131 Series were serially passaged in xylose-containing liquid media. Values for dry cell weight (DCW), fat as a weight percent of DCW (% Fat), and grams of fat per liter of cell culture (g/L Fat) for each culture were measured at serial passage numbers 5, 11, and 19. Results are shown in Table 2, below.

**Table 2: Dry Cell Weight, % Fat, and g/L Fat for a Piromyces 130 Series and a Piromyces 131 Series Transformant Grown in Xylose-Containing Media**

| | **Piromyces 130 series** | **Piromyces 131 series** |
|---|---|---|
| Dry Cell Weight (DCW) (mg) | | |
| Passage 5 | 0.12 | 0.13 |
| Passage 11 | 0.36 | 0.26 |
| Passage 19 | 0.33 | 0.33 |

| % Fat | | |
|---|---|---|
| Passage 5 | nd | nd |
| Passage 11 | 38.03 | 39.53 |
| Passage 19 | 43.30 | 50.50 |

| g/L Fat | | |
|---|---|---|
| Passage 5 | nd | nd |
| Passage 12 | 2.74 | 2.06 |
| Passage 19 | 2.85 | 3.38 |
| *nd* = *not determined* | | |

The same clones were further grown in liquid culture for FAME analysis. Colonies picked from solid media plates selective for growth on xylose as a carbon source were propagated in liquid media containing xylose as the primary carbon source over 19 serial transfers. The inoculum used to start the 19th serial passage was also used to begin a comparative control culture for each clone in liquid media containing glucose as the primary carbon source. Wild type (WT) *Schizochytrium* was also grown in parallel in glucose-containing liquid medium. No growth of the wild type *Schizochytrium* strain was detected in xylose- containing liquid medium and thus FAME analysis and other growth characterization was not possible.

Oils were extracted from cell cultures using standard procedures and analyzed for fatty acid composition as percent of total fatty acid methyl esters (FAMEs). *See,* e.g., U.S. Publ. No. 2010/0239533.

Dry cell weight (DCW), fat as a weight % of DCW (% Fat), grams of fat per liter of cell culture (g/L Fat), milligrams of DHA per gram of total fat, percent values of each fat detected, and summations of percent values of fat classes (saturates, monounsaturates, and fats of greater than 18 carbons in length) are listed in Table 3, below.

**Table 3: FAME Analysis from a Piromyces 130 Series and a Piromyces 131 Series Transformant**

| | **WT grown on glucose** | **130 Series grown on glucose** | **130 Series grown on xylose** | **131 Series grown on glucose** | **131 Series grown on xylose** |
|---|---|---|---|---|---|
| | | | | | |
| DCW mg | 0.82 | 0.80 | 0.33 | 0.73 | 0.33 |
| DCW g/L | 16.38 | 15.95 | 6.59 | 14.61 | 6.68 |
| % Fat | 60.58 | 65.88 | 43.34 | 60.39 | 50.53 |
| g/L fat | 9.92 | 10.51 | 2.85 | 8.82 | 3.38 |
| Fatty Acid Profile: | | | | | |
| % 12:0 | 0.24 | 0.32 | 0.08 | 0.30 | 0.00 |
| % 12:1 | 0.00 | 0.00 | 0.00 | 0.00 | 0.00 |
| % 13:0 | 0.00 | 0.00 | 0.00 | 0.00 | 0.00 |
| % 13:1 | 0.00 | 0.00 | 0.00 | 0.00 | 0.00 |
| % 14:0 | 10.65 | 12.04 | 6.82 | 12.73 | 6.90 |
| % 14:1 | 0.00 | 0.00 | 0.00 | 0.00 | 0.00 |
| % 15:1 | 0.25 | 0.29 | 0.32 | 0.35 | 0.00 |
| % 16:0 | 34.43 | 29.40 | 25.43 | 30.27 | 32.48 |
| % 16:1 | 9.24 | 13.11 | 8.32 | 11.82 | 6.57 |
| % 16:2 | 0.00 | 0.00 | 0.00 | 0.00 | 0.00 |
| % 16:3 | 0.00 | 0.00 | 0.00 | 0.00 | 0.00 |
| % 17:0 | 0.00 | 0.00 | 0.00 | 0.00 | 0.00 |
| % 18:0 | 1.22 | 1.00 | 1.06 | 0.82 | 1.31 |
| % 18:1 n-9 | 0.00 | 0.00 | 0.00 | 0.00 | 0.00 |
| % 18:1 n-7 | 5.77 | 6.24 | 7.79 | 5.21 | 6.88 |
| % 18:2 | 0.00 | 0.00 | 0.00 | 0.00 | 0.00 |
| % 18:3 n-6 | 0.00 | 0.00 | 0.00 | 0.00 | 0.00 |
| % 18:3 n-3 | 0.00 | 0.00 | 0.15 | 0.00 | 0.00 |
| % 18:4 n-3 | 0.00 | 0.00 | 0.00 | 0.00 | 0.00 |
| % 20:0 | 0.00 | 0.00 | 0.00 | 0.00 | 0.00 |
| % 20:1 n-9 | 0.00 | 0.00 | 0.00 | 0.00 | 0.00 |
| % 20:2 | 0.00 | 0.00 | 0.00 | 0.00 | 0.00 |
| % 20:3 n-9 | 0.00 | 0.00 | 0.00 | 0.00 | 0.00 |
| % 20:3 n-6 | 0.00 | 0.00 | 0.00 | 0.00 | 0.00 |
| % 20:3 n-3 | 0.00 | 0.00 | 0.00 | 0.00 | 0.00 |
| % 20:4 ARA | 0.00 | 0.00 | 0.00 | 0.00 | 0.00 |
| % 20:5 n-3 EPA | 0.00 | 0.00 | 1.58 | 0.61 | 2.09 |
| % 22:0 | 0.00 | 0.00 | 0.00 | 0.00 | 0.00 |
| % 22:1 | 0.00 | 0.00 | 0.00 | 0.00 | 0.00 |
| % 22:2 | 0.00 | 0.00 | 0.00 | 0.00 | 0.00 |
| % 22:3 | 0.00 | 0.00 | 0.00 | 0.00 | 0.00 |
| % 22:4 n-6 | 0.00 | 0.00 | 0.00 | 0.00 | 0.00 |
| % 22:5 n-6 | 10.21 | 10.12 | 14.05 | 10.56 | 12.18 |
| % 22:5 n-3 DPA | 0.00 | 0.00 | 0.30 | 0.00 | 0.00 |
| % 22:6 n-3 DHA | 27.98 | 27.48 | 33.58 | 26.87 | 31.59 |
| % 24:0 | 0.00 | 0.00 | 0.00 | 0.00 | 0.00 |
| % 24:1 | 0.00 | 0.00 | 0.00 | 0.00 | 0.00 |
| % Unknown | 0.00 | 0.00 | 0.53 | 0.47 | 0.00 |
| | | | | | |
| mg/g DHA | 167.67 | 179.05 | 144.03 | 160.50 | 157.88 |
| Saturated FAME | 45.32 | 41.76 | 32.34 | 43.30 | 39.38 |
| Monounsaturate d FAME | 15.26 | 19.63 | 16.42 | 17.37 | 13.45 |
| >C 18 FAME | 38.19 | 37.60 | 49.51 | 38.04 | 45.86 |

### EXAMPLE 5

### Growth on Xylose of Schizochytrium transformed with Xylose Transporter, Xylose Reductase, Xylitol Dehydrogenase, and Xylulose Kinase Expression Vectors

Co-transformations of *Schizochytrium* wild type strain ATCC 20888 were performed in which a vector containing the *Candida intermedia* xylose transporter (pCL0130) was co-transformed with three additional vectors: a vector containing *Pichia stipitis* xylose reductase (pCL0133), a vector containing *Pichia stipitis* Xylitol dehydrogenase (pCL0134), and a vector containing *Pichia stipitis* xylulose kinase (pCL0135). This combination of vectors was termed the 130 Pichia Series. Transformants were plated directly on solid xylose SSFM media and after 3-5 weeks colonies were picked and further propagated in liquid xylose-SSFM. Cotransformation rates for the 130 Pichia Series were similar to those for the 130 Piromyces Series and 131 Piromyces Series of Example 4. *Schizochytrium* cells transformed with a *Candida intermedia* xylose transporter, a xylose reductase, a xylitol dehydrogenase, and a xylulose kinase were able to grow in solid and liquid SSFM media containing xylose as a sole carbon source.

All of the various aspects, embodiments, and options described herein can be combined in any and all variations.

## Claims

1. A method of producing a thraustochytrid cell culture, comprising:
a. transforming a thraustochytrid cell with nucleic acid molecules comprising a polynucleotide sequence encoding a heterologous xylose transporter, a heterologous xylose isomerase, and a heterologous xylulose kinase; or with nucleic acid molecules comprising a polynucleotide sequence encoding a heterologous xylose transporter, a heterologous xylulose kinase, a heterologous xylose reductase, and a heterologous xylitol dehydrogenase; wherein the heterologous genes are codon-optimised for expression in the thraustochytrid cell, and the heterologous genes are under the control of any promoter sequence, any terminator sequence and/or any other regulatory sequence that is functional in a thraustochytrid cell ; and
b. growing the transformed thraustochytrid cell in a culture medium comprising xylose as a carbon source.

2. The transformed thraustochytrid cell defined in claim 1, wherein the polynucleotide sequence encoding the heterologous xylose transporter is at least 90% identical to a sequence selected from the polynucleotide sequence of Accession No. AJ875406, BT015128, AF127802, AJ249910, X59465, or X55392; a polynucleotide sequence encoding the amino acid sequence of Accession No. CAB76571; the polynucleotide sequence of SEQ ID NO:2, SEQ ID NO:3, SEQ ID NO:6, SEQ ID NO:7, SEQ ID NO:21, SEQ ID NO:22, or SEQ ID NO:23; and combinations thereof.

3. The transformed thraustochytrid cell defined in any of claims 1-2, wherein the thraustochytrid is a *Schizochytrium* or a *Thraustochytrium.*

4. A thraustochytrid culture comprising:
a. the thraustochytrid cell as defined in of any of claims 1-3, and
b. a cell culture medium comprising xylose as a carbon source.

5. A method of producing a thraustochytrid biomass, comprising:
a. growing the thraustochytrid cell as defined in any of claims 1-3 in a culture medium comprising xylose as a carbon source, and
b. harvesting the biomass from the culture medium.

6. A method of producing a microbial oil, comprising:
a. growing the thraustochytrid cell as defined in any of claims 1-3 in a culture medium comprising xylose as a carbon source to produce a biomass, and
b. extracting an oil from the biomass.

7. A method of producing a food product, cosmetic, industrial composition, or pharmaceutical composition for an animal or human, comprising:
a. growing the thraustochytrid cell as defined in any of claims 1-3 in a culture medium comprising xylose as a carbon source,
b. harvesting a biomass from the culture medium, and
c. preparing the food product, cosmetic, industrial composition, or pharmaceutical composition from the biomass.

8. The method of claim 7, further comprising extracting an oil from the biomass and preparing the food product, cosmetic, industrial composition, or pharmaceutical composition from the oil.

9. The method of claim 7 or claim 8, wherein the food product is selected from (a) an infant formula, (b) milk, (c) a beverage, (d) a therapeutic drink, (e) a nutritional drink, (f) an additive for animal or human food, (g) nutritional supplement, (h) animal feed, and (i) a combination of (b), (c), (d), and/or (e).

10. The method of claim 9, wherein the animal feed is selected from (a) an aquaculture feed, (b) a domestic animal feed, (c) a zoological animal feed, (d) a work animal feed, (e) a livestock feed, and (f) a combination of (b), (c), (d), and/or (e).

11. A method for producing a biofuel, comprising:
a. growing the thraustochytrid cell as defined in any of claims 1-3 in a culture medium comprising xylose as a carbon source to produce a biomass,
b. extracting an oil from the biomass, and
c. producing a biofuel by transesterifying the oil, cracking the oil, processing the oil by thermal depolymerization, adding the oil to a petroleum refining process, or a combination thereof.

## Patentansprüche

1. Verfahren zur Herstellung einer Thraustochytriden-Zellkultur, umfassend:
a. Transformieren einer Thraustochytriden-Zelle mit Nukleinsäuremolekülen, die Polynukleotidsequenz umfassen, die für einen heterologen Xylosetransporter, eine heterologe Xyloseisomerase und eine heterologe Xylulosekinase codiert; oder mit Nukleinsäuremolekülen, die eine Polynukleotidsequenz umfassen, die für einen heterologen Xylosetransporter, eine heterologe Xylulosekinase, eine heterologe Xylosereduktase und eine heterologe Xylitdehydrogenase codiert; wobei die heterologen Gene für Expression in der Thraustochytriden-Zelle codon-optimiert sind und die heterologen Gene unter der Kontrolle einer beliebigen Promotersequenz, einer beliebigen Terminatorsequenz und/oder einer beliebigen sonstigen Regulationssequenz, die in einer Thraustochytriden-Zelle funktional ist, stehen; und
b. Züchten der transformierten Thraustochytriden-Zelle in einem Kulturmedium, das Xylose als Kohlenstoffquelle umfasst.

2. Transformierte Thraustochytriden-Zelle wie in Anspruch 1 definiert, wobei die Polynukleotidsequenz, die für den heterologen Xylosetransporter codiert, zu einer Sequenz, ausgewählt aus der Polynukleotidsequenz der Zugangs-Nr. AJ875406, BT015128, AF127802, AJ249910, X59465 oder X55392; einer Polynukleotidsequenz, die für die Aminosäuresequenz der Zugangs-Nr. CAB76571 codiert; der Polynukleotidsequenz gemäß SEQ ID NO: 2, SEQ ID NO: 3, SEQ ID NO: 6, SEQ ID NO: 7, SEQ ID NO: 21, SEQ ID NO: 22 oder SEQ ID NO: 23; und Kombinationen davon zu mindestens 90% identisch ist.

3. Transformierte Thraustochytriden-Zelle wie in einem der Ansprüche 1-2 definiert, wobei es sich bei dem Thraustochytriden um ein *Schizochytrium* oder ein *Thraustochytrium* handelt.

4. Thraustochytriden-Kultur, umfassend:
a. die Thraustochytriden-Zelle wie in einem der Ansprüche 1-3 definiert, und
b. ein Zellkulturmedium, das Xylose als Kohlenstoffquelle umfasst.

5. Verfahren zur Herstellung einer Thraustochytriden-Biomasse, umfassend:
a. Züchten der Thraustochytriden-Zelle wie in einem der Ansprüche 1-3 definiert in einem Kulturmedium, das Xylose als Kohlenstoffquelle umfasst, und
b. Ernten der Biomasse aus dem Kulturmedium.

6. Verfahren zur Herstellung eines mikrobiellen Öls, umfassend:
a. Züchten der Thraustochytriden-Zelle wie in einem der Ansprüche 1-3 definiert in einem Kulturmedium, das Xylose als Kohlenstoffquelle umfasst, um eine Biomasse herzustellen, und
b. Extrahieren eines Öls aus der Biomasse.

7. Verfahren zur Herstellung eines Nahrungsmittelprodukts, eines Kosmetikums, einer industriellen Zusammensetzung oder einer pharmazeutischen Zusammensetzung für ein Tier oder einen Menschen, umfassend:
a. Züchten der Thraustochytriden-Zelle wie in einem der Ansprüche 1-3 definiert in einem Kulturmedium, das Xylose als Kohlenstoffquelle umfasst,
b. Ernten einer Biomasse aus dem Kulturmedium, und
c. Herstellen des Nahrungsmittelprodukts, des Kosmetikums, der industriellen Zusammensetzung oder der pharmazeutischen Zusammensetzung aus der Biomasse.

8. Verfahren nach Anspruch 7, weiterhin umfassend das Extrahieren eines Öls aus der Biomasse und das Herstellen des Nahrungsmittelprodukts, des Kosmetikums, der industriellen Zusammensetzung oder der pharmazeutischen Zusammensetzung aus dem Öl.

9. Verfahren nach Anspruch 7 oder Anspruch 8, wobei das Nahrungsmittelprodukt ausgewählt ist aus: (a) einer Babynahrung, (b) Milch, (c) einem Getränk, (d) einem therapeutischen Trank, (e) einem Nährtrank, (f) einem Zusatzstoff für Tierfutter oder menschliche Nahrung, (g) Nahrungsmittelzusatzstoff, (h) Tierfutter und (i) einer Kombination von (b), (c), (d) und/oder (e).

10. Verfahren nach Anspruch 9, wobei das Tierfutter ausgewählt ist aus: (a) einem Aquakulturfutter, (b) einem Haustierfutter, (c) einem Zootierfutter, (d) einem Arbeitstierfutter, (e) einem Nutztierfutter und (f) einer Kombination von (b), (c), (d) und/oder (e).

11. Verfahren zur Herstellung eines Biotreibstoffs, umfassend:
a. Züchten der Thraustochytriden-Zelle wie in einem der Ansprüche 1-3 definiert in einem Kulturmedium, das Xylose als Kohlenstoffquelle umfasst, um eine Biomasse herzustellen, und
b. Extrahieren eines Öls aus der Biomasse, und
c. Herstellen eines Biotreibstoffs durch Umesterung des Öls, Kracken des Öls, Verarbeiten des Öls mittels thermischer Depolymerisation, Zugeben des Öls zu einer Erdölraffination oder einer Kombination davon.

## Revendications

1. Méthode de production d'une culture cellulaire de thraustochytride, comprenant
a. la transformation d'une cellule de thraustochytride par des molécules d'acide nucléique comprenant une séquence polynucléotidique codant pour un transporteur de xylose hétérologue, une xylose isomérase hétérologue, et une xylulose kinase hétérologue ; ou par des molécules d'acide nucléique comprenant une séquence polynucléotidique codant pour un transporteur de xylose hétérologue, une xylulose kinase hétérologue, une xylose réductase hétérologue, et une xylitol déshydrogénase hétérologue ; où les gènes hétérologues sont à codon optimisé pour une expression dans la cellule de thraustochytride, et les gènes hétérologues sont sous le contrôle d'une séquence de promoteur quelconque, une séquence de terminateur quelconque et/ou toute autre séquence régulatrice qui est fonctionnelle dans une cellule de thraustochytride ; et
b. la culture de la cellule de thraustochytride transformée dans un milieu de culture comprenant du xylose comme source de carbone.

2. Cellule de thraustochytride transformée définie selon la revendication 1, où la séquence polynucléotidique codant pour le transporteur de xylose hétérologue est identique à au moins 90% par rapport à une séquence choisie parmi la séquence polynucléotidique de Numéro d'Accès AJ875406, BT015128, AF127802, AJ249910, X59465 ou X55392 ; une séquence polynucléotidique codant pour la séquence d'acides aminés de Numéro d'Accès CAB76571 ; la séquence polynucléotidique de SEQ ID n° 2, SEQ ID n° 3, SEQ ID n° 6, SEQ ID n° 7, SEQ ID n° 21, SEQ ID n° 22, ou SEQ ID n° 23 ; et des combinaisons de celles-ci.

3. Cellule de thraustochytride transformée définie selon l'une quelconque des revendications 1-2, où le thraustochytride est *Schizochytrium* ou *Thraustochytrium.*

4. Culture de thraustochytride comprenant :
a. la cellule de thraustochytride telle que définie selon l'une quelconque des revendications 1-3, et
b. un milieu de culture cellulaire comprenant du xylose comme source de carbone.

5. Méthode de production d'une biomasse de thraustochytride, comprenant :
a. la culture de la cellule de thraustochytride telle que définie selon l'une quelconque des revendications 1-3 dans un milieu de culture comprenant du xylose comme source de carbone, et
b. la récolte de la biomasse à partir du milieu de culture.

6. Méthode de production d'une huile microbienne, comprenant :
a. la culture de la cellule de thraustochytride telle que définie selon l'une quelconque des revendications 1-3 dans un milieu de culture comprenant du xylose comme source de carbone afin de produire une biomasse, et
b. l'extraction d'une huile à partir de la biomasse.

7. Méthode de production d'un produit alimentaire, d'un produit cosmétique, d'une composition industrielle, ou d'une composition pharmaceutique pour un animal ou un être humain, comprenant :
a. la culture de la cellule de thraustochytride telle que définie selon l'une quelconque des revendications 1-3 dans un milieu de culture comprenant du xylose comme source de carbone,
b. la récolte d'une biomasse à partir du milieu de culture, et
c. la préparation du produit alimentaire, du produit cosmétique, de la composition industrielle, ou de la composition pharmaceutique à partir de la biomasse.

8. Méthode selon la revendication 7, comprenant en outre l'extraction d'une huile à partir de la biomasse et la préparation du produit alimentaire, du produit cosmétique, de la composition industrielle, ou de la composition pharmaceutique à partir de l'huile.

9. Méthode selon la revendication 7 ou la revendication 8, où le produit alimentaire est choisi parmi (a) une préparation pour nourrisson, (b) du lait, (c) une boisson, (d) une boisson thérapeutique, (e) une boisson nutritionnelle, (f) un additif pour un aliment pour l'homme ou l'animal, (g) un complément nutritionnel, (h) un aliment pour animaux, et (i) une combinaison de (b), (c), (d) et/ou (e).

10. Méthode selon la revendication 9, où l'aliment pour animaux est choisi parmi (a) un aliment pour l'aquaculture, (b) un aliment pour animaux domestiques, (c) un aliment pour animaux de zoo, (d) un aliment pour animaux de travail, (e) un aliment pour animaux d'élevage, et (f) une combinaison de (b), (c), (d) et/ou (e).

11. Méthode de production d'un biocarburant, comprenant :
a. la culture de la cellule de thraustochytride telle que définie selon l'une quelconque des revendications 1-3 dans un milieu de culture comprenant du xylose comme source de carbone afin de produire une biomasse,
b. l'extraction d'une huile à partir de la biomasse, et
c. la production d'un biocarburant par la transestérification de l'huile, le craquage de l'huile, le traitement de l'huile par dépolymérisation thermique, l'addition de l'huile à un procédé de raffinage du pétrole, ou une combinaison de ceux-ci.


### SEQUENCE LISTING

<110> MARTEK BIOSCIENCES CORPORTATION
<120> Recombinant Thraustochytrids that Grow on Xylose, and Compositions, Methods of Making, and Uses Thereof
<130> 2715.051PC01
<140> To be assigned
   <141> Herewith
<150> US 61/290,471
   <151> 2009-12-28
<160> 25
<170> PatentIn version 3.5
<210> 1
   <211> 1614
   <212> DNA
   <213> Artificial Sequence
<220>
   <223> Secretion signal
<400> 1
   ggatccatga agttcgcgac ctcggtcgca attttgcttg tggccaacat agccaccgcc      60
   ctcgcgtcga tgaccaacga gacctcggac cgccctctcg tgcactttac ccccaacaag     120
   ggttggatga acgatcccaa cggcctctgg tacgacgaga aggatgctaa gtggcacctt     180
   tactttcagt acaaccctaa cgacaccgtc tggggcaccc cgctcttctg gggccacgcc     240
   acctccgacg acctcaccaa ctgggaggac cagcccattg ctatcgcccc caagcgcaac     300
   gactcgggag ctttttccgg ttccatggtt gtggactaca acaacacctc cggttttttt     360
   aacgacacca ttgacccccg ccagcgctgc gtcgccatct ggacctacaa cacgcccgag     420
   agcgaggagc agtacatcag ctacagcctt gatggaggct acacctttac cgagtaccag     480
   aagaaccctg tcctcgccgc caactccacc cagttccgcg accctaaggt tttttggtac     540
   gagccttccc agaagtggat tatgaccgcc gctaagtcgc aggattacaa gatcgagatc     600
   tacagcagcg acgacctcaa gtcctggaag cttgagtccg cctttgccaa cgagggtttt     660
   ctcggatacc agtacgagtg ccccggtctc atcgaggtcc ccaccgagca ggacccgtcc     720
   aagtcctact gggtcatgtt tatttccatc aaccctggcg cccctgccgg cggcagcttc     780
   aaccagtact tcgtcggctc ctttaacggc acgcattttg aggccttcga caaccagtcc     840
   cgcgtcgtcg acttcggcaa ggactactac gccctccaga ccttctttaa caccgacccc     900
   acctacggca gcgccctcgg tattgcttgg gcctccaact gggagtactc cgctttcgtc     960
   cccactaacc cctggcgcag ctcgatgtcc ctcgtccgca agttttcgct taacaccgag    1020
   taccaggcca accccgagac cgagcttatt aacctgaagg ccgagcctat tctcaacatc    1080
   tccaacgctg gcccctggtc ccgctttgct actaacacta ccctcaccaa ggccaactcc    1140
   tacaacgtcg atctctccaa ctccaccggt actcttgagt ttgagctcgt ctacgccgtc 1200
   aacaccaccc agaccatctc caagtccgtc ttcgccgacc tctccctctg gttcaagggc 1260
   cttgaggacc ccgaggagta cctgcgcatg ggttttgagg tctccgcctc ctccttcttc 1320
   ctcgatcgcg gtaactccaa ggttaagttt gtcaaggaga acccctactt tactaaccgt 1380
   atgagcgtca acaaccagcc ctttaagtcc gagaacgatc ttagctacta caaggtttac 1440
   ggcctcctcg accagaacat tctcgagctc tactttaacg acggagatgt cgtcagcacc 1500
   aacacctact ttatgaccac tggaaacgcc ctcggcagcg tgaacatgac caccggagtc 1560
   gacaacctct tttacattga caagtttcag gttcgcgagg ttaagtaaca tatg 1614
<210> 2
   <211> 1569
   <212> DNA
   <213> Candida intermedia
<220>
   <223> xylose transporter protein GXS1, codon optimized
<400> 2
   atgggcctcg aggataaccg catggttaag cgctttgtca acgtgggcga gaagaaggcc 60
   ggtagcaccg ccatggccat cattgttggc ctcttcgcgg cctcgggcgg cgtcctcttc 120
   ggctacgaca ccggcactat ctcgggcgtc atgactatgg actacgttct cgcccgctac 180
   ccctccaaca agcactcctt caccgctgac gagtcgtcgc tcatcgtttc cattctttcg 240
   gtcggcacct tcttcggcgc cctctgcgcc ccgttcctca acgataccct cggccgccgc 300
   tggtgcctca tcctcagcgc cctcattgtc tttaacatcg gcgccatcct ccaggtcatt 360
   tccaccgcca tccccctgct ctgcgcgggc cgcgttatcg ccggtttcgg tgtcggcctc 420
   atttccgcca ccatcccgct ctaccagtcc gagactgctc cgaagtggat tcgcggcgcc 480
   atcgtttcct gctaccagtg ggccatcact atcggacttt tcctcgcttc ctgcgtcaac 540
   aagggcaccg agcacatgac caactccggt tcgtaccgta ttcctctggc catccagtgc 600
   ctctggggcc tcatccttgg tattggcatg attttcctcc ctgagacccc ccgcttctgg 660
   atttcgaagg gcaaccagga gaaggccgcc gagtccctcg cccgtctccg caagctcccc 720
   atcgaccatc ctgatagcct tgaggagctt cgcgatatta ctgccgccta cgagttcgag 780
   accgtctacg gtaagtccag ctggtcccag gtcttttccc acaagaacca tcagctcaag 840
   cgcctcttta ccggcgttgc cattcaggcc tttcagcagc tcaccggagt taactttatc 900
   ttttactacg gcaccacctt ttttaagcgc gccggagtca acggattcac catcagcctt 960
   gccaccaaca tcgttaacgt cggcagcact attcccggca ttcttctcat ggaggtcctc 1020
   ggccgccgca acatgctcat gggcggtgcc accggcatgt cgctgtcgca gcttatcgtc 1080
   gccattgtcg gagttgccac gtcggagaac aacaagtcga gccagtcggt cctcgtcgct 1140
   ttctcgtgca tctttatcgc tttttttgcc gccacctggg gtccctgcgc ctgggtcgtc 1200
   gtcggcgagc tctttcccct tcgcactcgc gctaagtccg tttccctctg caccgcgtcc 1260
   aactggctct ggaactgggg cattgcttac gccaccccct acatggtcga cgaggataag 1320
   ggtaacctcg gcagcaacgt tttttttatt tggggaggct tcaacctcgc ttgcgtcttt 1380
   ttcgcgtggt acttcattta cgagaccaag ggcctttccc tcgagcaggt tgatgagctc 1440
   tacgagcatg tttcgaaggc gtggaagtcc aagggttttg tcccgtccaa gcactccttt 1500
   cgcgagcagg tcgaccagca gatggactcc aagaccgagg ccattatgag cgaggaggcg 1560
   tcggtttaa 1569
<210> 3
   <211> 1512
   <212> DNA
   <213> Arabidopsis thaliana
<220>
   <223> xylose transporter protein At5g17010, codon optimized
<400> 3
   atggccctcg accctgagca gcagcagccc atttcctccg tgtcgcgcga gtttggtaag      60
   tcgtccggtg agatctcccc cgagcgtgag cctctcatta aggagaacca cgtccccgag     120
   aactactccg ttgttgccgc catcctcccc ttcctcttcc cggccctggg tggcctcctt     180
   tacggttacg agattggcgc tacgtcgtgc gctacgattt cccttcagtc cccctccctc     240
   tccggcatct cctggtacaa cctctcctcc gtcgatgttg gcctcgtcac ttccggttcc     300
   ctctacggtg ctctgtttgg ctccattgtt gccttcacca ttgccgacgt tattggccgt     360
   cgcaaggagc ttatcctcgc tgctctcctc tacctcgtcg gtgccctcgt taccgctctc     420
   gcccctacgt actccgttct catcatcggc cgtgtcattt acggtgtttc cgtcggtctt     480
   gccatgcatg ctgcccctat gtacatcgcg gagaccgccc cgtcccccat ccgcggccag     540
   ctcgtttccc tcaaggagtt tttcatcgtt ctcggtatgg tcggcggata cggcattggt     600
   tccctcaccg tcaacgtcca ctccggttgg cgctacatgt acgctacctc cgttcccctc     660
   gctgtgatca tgggcattgg catgtggtgg cttcctgcct ccccccgttg gctcctcctc     720
   cgcgtcattc agggtaaggg taacgttgag aaccagcgcg aggctgccat taagtccctc     780
   tgctgcctcc gtggtcctgc cttcgtcgac tcggccgccg agcaggtcaa cgagattctc     840
   gccgagctta ccttcgttgg cgaggataag gaggtcacct tcggcgagct cttccaggga     900
   aagtgcctca aggccctcat tatcggcggc ggccttgttc tctttcagca gatcaccggt     960
   cagccttcgg tcctctacta cgccccctcg atcctccaga ctgcgggctt ctccgccgcc    1020
   ggcgatgcta cccgcgtttc cattcttctc ggcctcctca agctcattat gaccggtgtc    1080
   gccgtcgtcg ttatcgatcg tctcggccgt cgccctctcc tcctcggcgg agtcggtggt    1140
   atggttgttt cgctctttct ccttggctcg tactaccttt tcttcagcgc ttcccccgtc    1200
   gtcgccgttg tcgccctcct tctctacgtg ggttgctacc agctctcctt tggccccatt    1260
   ggctggctta tgatttccga gatttttccc ctcaagctcc gtggtcgcgg actctccctt    1320
   gccgtgcttg tcaactttgg tgccaacgcc ctcgtcacct ttgccttttc ccctctcaag    1380
   gagctcctcg gcgccggcat cctgttttgc ggctttggcg ttatctgcgt tctctccctt    1440
   gtttttatct tttttatcgt cccggagact aagggcctca cgctcgagga gatcgaggcg    1500
   aagtgcctct aa                                                        1512
<210> 4
   <211> 6175
   <212> DNA
   <213> Artificial Sequence
<220>
   <223> vector pCL0121
<400> 4
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg ggggggaggg agaggcagga     840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tgaagttcgc    1080
   gacctcggtc gcaattttgc ttgtggccaa catagccacc gccctcgcgc agagcgatgg    1140
   ctgcaccccc accgaccaga cgatggtgag caagggcgag gagctgttca ccggggtggt    1200
   gcccatcctg gtcgagctgg acggcgacgt aaacggccac aagttcagcg tgtccggcga    1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt 2280
   acatgagcgt tttcaagctg gagcgtgaga tcataccttt cttgatcgta atgttccaac 2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatggccaa gttgaccagt gccgttccgg tgctcaccgc 5580
   gcgcgacgtc gccggagcgg tcgagttctg gaccgaccgg ctcgggttct cccgggactt 5640
   cgtggaggac gacttcgccg gtgtggtccg ggacgacgtg accctgttca tcagcgcggt 5700
   ccaggaccag gtggtgccgg acaacaccct ggcctgggtg tgggtgcgcg gcctggacga 5760
   gctgtacgcc gagtggtcgg aggtcgtgtc cacgaacttc cgggacgcct ccgggccggc 5820
   catgaccgag atcggcgagc agccgtgggg gcgggagttc gccctgcgcg acccggccgg 5880
   caactgcgtg cacttcgtgg ccgaggagca ggactgacac gtgctacgag atttcgattc 5940
   caccgccgcc ttctatgaaa ggttgggctt cggaatcgtt ttccgggacg ccggctggat 6000
   gatcctccag cgcggggatc tcatgctgga gttcttcgcc caccccaact tgtttattgc 6060
   agcttataat ggttacaaat aaagcaatag catcacaaat ttcacaaata aagcattttt 6120
   ttcactgcat tctagttgtg gtttgtccaa actcatcaat gtatcttatc ggtac 6175
<210> 5
   <211> 6611
   <212> DNA
   <213> Artificial Sequence
<220>
   <223> vector pCL0122
<400> 5
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag      720
   accgccaacg cagagaccga gaccgaggta cgtcgcgccc gagcacgccg cgacgcgcgg      780
   cagggacgag gagcacgacg ccgcgccgcg ccgcgcgggg ggggggaggg agaggcagga      840
   cgcgggagcg agcgtgcatg tttccgcgcg agacgacgcc gcgcgcgctg gagaggagat      900
   aaggcgcttg gatcgcgaga gggccagcca ggctggaggc gaaaatgggt ggagaggata      960
   gtatcttgcg tgcttggacg aggagactga cgaggaggac ggatacgtcg atgatgatgt     1020
   gcacagagaa gaagcagttc gaaagcgact actagcaagc aagggatcca tgaagttcgc     1080
   gacctcggtc gcaattttgc ttgtggccaa catagccacc gccctcgcgc agagcgatgg     1140
   ctgcaccccc accgaccaga cgatggtgag caagggcgag gagctgttca ccggggtggt     1200
   gcccatcctg gtcgagctgg acggcgacgt aaacggccac aagttcagcg tgtccggcga     1260
   gggcgagggc gatgccacct acggcaagct gaccctgaag ttcatctgca ccaccggcaa     1320
   gctgcccgtg ccctggccca ccctcgtgac caccctgacc tacggcgtgc agtgcttcag     1380
   ccgctacccc gaccacatga agcagcacga cttcttcaag tccgccatgc ccgaaggcta     1440
   cgtccaggag cgcaccatct tcttcaagga cgacggcaac tacaagaccc gcgccgaggt     1500
   gaagttcgag ggcgacaccc tggtgaaccg catcgagctg aagggcatcg acttcaagga     1560
   ggacggcaac atcctgggac acaagctgga gtacaactac aacagccaca acgtctatat     1620
   catggccgac aagcagaaga acggcatcaa ggtgaacttc aagatccgcc acaacatcga     1680
   ggacggcagc gtgcagctcg ccgaccacta ccagcagaac acccccatcg gcgacggccc     1740
   cgtgctgctg cccgacaacc actacctgag cacccagtcc gccctgagca aagaccccaa     1800
   cgagaagcgc gatcacatgg tcctgctgga gttcgtgacc gccgccggga tcactctcgg     1860
   catggacgag ctgtacaagc accaccatca ccaccactaa catatgagtt atgagatccg     1920
   aaagtgaacc ttgtcctaac ccgacagcga atggcgggag ggggcgggct aaaagatcgt     1980
   attacatagt atttttcccc tactctttgt gtttgtcttt tttttttttt tgaacgcatt     2040
   caagccactt gtctgggttt acttgtttgt ttgcttgctt gcttgcttgc ttgcctgctt     2100
   cttggtcaga cggcccaaaa aagggaaaaa attcattcat ggcacagata agaaaaagaa     2160
   aaagtttgtc gaccaccgtc atcagaaagc aagagaagag aaacactcgc gctcacattc     2220
   tcgctcgcgt aagaatctta gccacgcata cgaagtaatt tgtccatctg gcgaatcttt     2280
   acatgagcgt tttcaagctg gagcgtgaga tcataccttt cttgatcgta atgttccaac     2340
   cttgcatagg cctcgttgcg atccgctagc aatgcgtcgt actcccgttg caactgcgcc     2400
   atcgcctcat tgtgacgtga gttcagattc ttctcgagac cttcgagcgc tgctaatttc     2460
   gcctgacgct ccttcttttg tgcttccatg acacgccgct tcaccgtgcg ttccacttct     2520
   tcctcagaca tgcccttggc tgcctcgacc tgctcggtaa aacgggcccc agcacgtgct     2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatgattga acaagatgga ttgcacgcag gttctccggc 5580
   cgcttgggtg gagaggctat tcggctatga ctgggcacaa cagacaatcg gctgctctga 5640
   tgccgccgtg ttccggctgt cagcgcaggg gcgcccggtt ctttttgtca agaccgacct 5700
   gtccggtgcc ctgaatgaac tgcaggacga ggcagcgcgg ctatcgtggc tggccacgac 5760
   gggcgttcct tgcgcagctg tgctcgacgt tgtcactgaa gcgggaaggg actggctgct 5820
   attgggcgaa gtgccggggc aggatctcct gtcatctcac cttgctcctg ccgagaaagt 5880
   atccatcatg gctgatgcaa tgcggcggct gcatacgctt gatccggcta cctgcccatt 5940
   cgaccaccaa gcgaaacatc gcatcgagcg agcacgtact cggatggaag ccggtcttgt 6000
   cgatcaggat gatctggacg aagagcatca ggggctcgcg ccagccgaac tgttcgccag 6060
   gctcaaggcg cgcatgcccg acggcgatga tctcgtcgtg acccatggcg atgcctgctt 6120
   gccgaatatc atggtggaaa atggccgctt ttctggattc atcgactgtg gccggctggg 6180
   tgtggcggac cgctatcagg acatagcgtt ggctacccgt gatattgctg aagagcttgg 6240
   cggcgaatgg gctgaccgct tcctcgtgct ttacggtatc gccgctcccg attcgcagcg 6300
   catcgccttc tatcgccttc ttgacgagtt cttctgacac gtgctacgag atttcgattc 6360
   caccgccgcc ttctatgaaa ggttgggctt cggaatcgtt ttccgggacg ccggctggat 6420
   gatcctccag cgcggggatc tcatgctgga gttcttcgcc caccccaact tgtttattgc 6480
   agcttataat ggttacaaat aaagcaatag catcacaaat ttcacaaata aagcattttt 6540
   ttcactgcat tctagttgtg gtttgtccaa actcatcaat gtatcttatc atgtctgaat 6600
   tcccggggta c 6611
<210> 6
   <211> 1314
   <212> DNA
   <213> Piromyces sp.
<220>
   <223> E2 xylose isomerase protein "Xy1A," codon optimized
<400> 6
   atggctaagg agtacttccc ccagatccag aagattaagt tcgagggtaa ggacagcaag 60
   aacccgctcg cctttcatta ctacgacgcc gagaaggagg tgatgggcaa gaagatgaag 120
   gactggcttc gctttgctat ggcttggtgg cacactctct gcgctgaggg cgcggaccag 180
   tttggcggcg gtacgaagag ctttccgtgg aacgagggca ctgacgctat tgagattgct 240
   aagcagaagg ttgacgctgg tttcgagatt atgcagaagc tcggtattcc gtactactgc 300
   tttcacgatg tcgacctcgt ttccgagggc aactcgatcg aggagtacga gtcgaacctc 360
   aaggctgtgg ttgcctacct caaggagaag cagaaggaga ccggaatcaa gctcctctgg 420
   agcaccgcca acgttttcgg ccacaagcgc tacatgaacg gcgcctccac caaccctgac 480
   ttcgatgttg ttgcccgcgc tattgtccag attaagaacg ccatcgacgc tggtatcgag 540
   ctcggagccg agaactacgt tttttggggc ggacgcgagg gttacatgtc cctcctcaac 600
   accgaccaga agcgtgagaa ggagcacatg gccactatgc ttaccatggc ccgcgactac 660
   gcccgcagca agggttttaa gggtactttt ctcattgagc cgaagcccat ggagccgacc 720
   aagcaccagt acgacgtcga caccgagacc gccattggct tccttaaggc ccacaacctt 780
   gacaaggatt ttaaggtgaa catcgaggtt aaccacgcta cgcttgccgg ccacaccttt 840
   gagcatgagc tcgcctgcgc tgttgacgcc ggaatgcttg gttccattga cgccaaccgc 900
   ggcgactacc agaacggctg ggacaccgac cagtttccga ttgaccagta cgagctcgtc 960
   caggcctgga tggagatcat ccgtggtgga ggctttgtta ccggtggtac gaacttcgac 1020
   gccaagacgc gccgtaacag cacggacctc gaggacatca tcattgctca tgtgtcgggc 1080
   atggacgcca tggctcgcgc ccttgagaac gctgctaagc tcctccagga gagcccctac 1140
   acgaagatga agaaggagcg ctacgcgtcg tttgacagcg gaatcggtaa ggacttcgag 1200
   gatggcaagc tcaccctgga gcaggtgtac gagtacggta agaagaacgg cgagccgaag 1260
   cagaccagcg gcaagcagga gctctacgag gccattgtcg ccatgtacca gtag 1314
<210> 7
   <211> 1485
   <212> DNA
   <213> Piromyces sp.
<220>
   <223> E2 xylulose kinase protein "XylB," codon optimized
<400> 7
   atgaagaccg tcgccggcat cgatcttgga acccagtcca tgaaggttgt catttacgac 60
   tacgagaaga aggagatcat cgagtccgcc tcgtgcccta tggagctcat tagcgagtcg 120
   gacggaaccc gcgagcagac gactgagtgg tttgacaagg gtctcgaggt gtgctttgga 180
   aagctctccg ctgataacaa gaagaccatt gaggcgattg gcatctccgg ccagctccac 240
   ggcttcgtcc ctctcgatgc gaacggaaag gcgctctaca acatcaagct ctggtgcgac 300
   accgccactg tggaggagtg caagatcatt actgacgccg ccggcggcga caaggctgtc 360
   atcgacgcgc tcggcaacct catgctcacc ggattcaccg ccccgaagat tctctggctc 420
   aagcgcaaca agcccgaggc ctttgctaac ctcaagtaca ttatgctgcc ccacgattac 480
   ctcaactgga agctgactgg agactacgtc atggagtacg gcgacgcctc cggcaccgcc 540
   ctttttgatt cgaagaaccg ctgctggtcg aagaagattt gcgacattat tgatcctaag 600
   ctgctcgacc ttctccctaa gctcattgag ccctcggccc ccgccggtaa ggtcaacgac 660
   gaggccgcca aggcgtacgg cattcccgcc ggaatccccg tttccgctgg cggcggtgat 720
   aacatgatgg gtgcggtcgg tactggcacc gtcgctgacg gattcctcac gatgagcatg 780
   ggcacctccg gaactcttta cggctactcg gacaagccta tttccgaccc ggctaacggc 840
   ctcagcggct tctgcagctc cacgggcggc tggcttcccc tcctttgcac catgaactgc 900
   accgtcgcca ccgagttcgt ccgcaacctt tttcagatgg atatcaagga gctgaacgtc 960
   gaggctgcta agtccccctg cggcagcgag ggcgttcttg tcattccttt cttcaacggc 1020
   gagcgcaccc cgaacctccc caacggccgc gcctcgatta ccggcctcac ctccgcgaac 1080
   acgtcccgcg ccaacatcgc tcgcgcctcc tttgagtcgg ccgtctttgc catgcgcggt 1140
   ggcctcgatg cgtttcgtaa gctcggattc cagcccaagg agattcgcct catcggcggt 1200
   ggttcgaagt ccgacctctg gcgccagatc gctgctgaca ttatgaacct tcccatccgt 1260
   gtcccccttc tcgaggaggc cgccgccctc ggcggagctg tccaggccct ttggtgcctt 1320
   aagaaccagt ccggtaagtg cgacatcgtc gagctttgca aggagcatat caagattgac 1380
   gagtccaaga acgccaaccc gattgccgag aacgtcgccg tgtacgataa ggcctacgat 1440
   gagtactgca aggtcgttaa cacgctcagc cctctgtacg cctaa 1485
<210> 8
   <211> 19
   <212> DNA
   <213> Artificial Sequence
<220>
   <223> Primer 5' CL0130
<400> 8
   cctcgggcgg cgtcctctt 19
<210> 9
   <211> 20
   <212> DNA
   <213> Artificial Sequence
<220>
   <223> Primer 3' CL0130
<400> 9
   ggcggccttc tcctggttgc 20
<210> 10
   <211> 24
   <212> DNA
   <213> Artificial Sequence
<220>
   <223> Primer 5' CL0131
<400> 10
   ctactccgtt gttgccgcca tcct 24
<210> 11
   <211> 22
   <212> DNA
   <213> Primer 3' CL0131
<400> 11
   ccgccgacca taccgagaac ga 22
<210> 12
   <211> 9291
   <212> DNA
   <213> Artificial Sequence
<220>
   <223> vector pCL0120
<400> 12
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg ggggggaggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagttcgc 1080
   gacctcggtc gcaattttgc ttgtggccaa catagccacc gccctcgcgc agagcgatgg 1140
   ctgcaccccc accgaccaga cgatggtgag caagggcgag gagctgttca ccggggtggt 1200
   gcccatcctg gtcgagctgg acggcgacgt aaacggccac aagttcagcg tgtccggcga 1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt 2280
   acatgagcgt tttcaagctg gagcgtgaga tcataccttt cttgatcgta atgttccaac 2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag ctttgcctca 5100
   acgcaactag gcccaggcct actttcactg tgtcttgtct tgcctttcac accgaccgag 5160
   tgtgcacaac cgtgttttgc acaaagcgca agatgctcac tcgactgtga agcaaaggtt 5220
   gcgcgcaagc gactgcgact gcgaggatga ggatgactgg cagcctgttc aaaaactgaa 5280
   aatccgcgat gggtcagctg ccattcgcgc atgacgcctg cgagagacaa gttaactcgt 5340
   gtcactggca tgtcctagca tctttacgcg agcaaaattc aatcgcttta ttttttcagt 5400
   ttcgtaacct tctcgcaacc gcgaatcgcc gtttcagcct gactaatctg cagctgcgtg 5460
   gcactgtcag tcagtcagtc agtcgtgcgc gctgttccag caccgaggtc gcgcgtcgcc 5520
   gcgcctggac cgctgctgct actgctagtg gcacggcagg taggagcttg ttgccggaac 5580
   accagcagcc gccagtcgac gccagccagg ggaaagtccg gcgtcgaagg gagaggaagg 5640
   cggcgtgtgc aaactaacgt tgaccactgg cgcccgccga cacgagcagg aagcaggcag 5700
   ctgcagagcg cagcgcgcaa gtgcagaatg cgcgaaagat ccacttgcgc gcggcgggcg 5760
   cgcacttgcg ggcgcggcgc ggaacagtgc ggaaaggagc ggtgcagacg gcgcgcagtg 5820
   acagtgggcg caaagccgcg cagtaagcag cggcggggaa cggtatacgc agtgccgcgg 5880
   gccgccgcac acagaagtat acgcgggccg aagtggggcg tcgcgcgcgg gaagtgcgga 5940
   atggcgggca aggaaaggag gagacggaaa gagggcggga aagagagaga gagagagtga 6000
   aaaaagaaag aaagaaagaa agaaagaaag aaagctcgga gccacgccgc ggggagagag 6060
   agaaatgaaa gcacggcacg gcaaagcaaa gcaaagcaga cccagccaga cccagccgag 6120
   ggaggagcgc gcgcaggacc cgcgcggcga gcgagcgagc acggcgcgcg agcgagcgag 6180
   cgagcgagcg cgcgagcgag caaggcttgc tgcgagcgat cgagcgagcg agcgggaagg 6240
   atgagcgcga cccgcgcggc gacgaggaca gcggcggcgc tgtcctcggc gctgacgacg 6300
   cctgtaaagc agcagcagca gcagcagctg cgcgtaggcg cggcgtcggc acggctggcg 6360
   gccgcggcgt tctcgtccgg cacgggcgga gacgcggcca agaaggcggc cgcggcgagg 6420
   gcgttctcca cgggacgcgg ccccaacgcg acacgcgaga agagctcgct ggccacggtc 6480
   caggcggcga cggacgatgc gcgcttcgtc ggcctgaccg gcgcccaaat ctttcatgag 6540
   ctcatgcgcg agcaccaggt ggacaccatc tttggctacc ctggcggcgc cattctgccc 6600
   gtttttgatg ccatttttga gagtgacgcg cttcaagttc attctcgctc gccacgagca 6660
   gggcgccggc cacatggccg agggctacgc gcgcgccacg ggcaagcccg gcgttgtcct 6720
   cgtcacctcg ggccctggag ccaccaacac catcaccccg atcatggatg cttacatgga 6780
   cggtacgccg ctgctcgtgt tcaccggcca ggtgcagacc tctgctgtcg gcacggacgc 6840
   tttccaggag tgtgacattg ttggcatcag ccgcgcgtgc accaagtgga acgtcatggt 6900
   caaggacgtg aaggagctcc cgcgccgcat caatgaggcc tttgagattg ccatgagcgg 6960
   ccgcccgggt cccgtgctcg tcgatcttcc taaggatgtg accgccgttg agctcaagga 7020
   aatgcccgac agctcccccc aggttgctgt gcgccagaag caaaaggtcg agcttttcca 7080
   caaggagcgc attggcgctc ctggcacggc cgacttcaag ctcattgccg agatgatcaa 7140
   ccgtgcggag cgacccgtca tctatgctgg ccagggtgtc atgcagagcc cgttgaatgg 7200
   cccggctgtg ctcaaggagt tcgcggagaa ggccaacatt cccgtgacca ccaccatgca 7260
   gggtctcggc ggctttgacg agcgtagtcc cctctccctc aagatgctcg gcatgcacgg 7320
   ctctgcctac gccaactact cgatgcagaa cgccgatctt atcctggcgc tcggtgcccg 7380
   ctttgatgat cgtgtgacgg gccgcgttga cgcctttgct ccggaggctc gccgtgccga 7440
   gcgcgagggc cgcggtggca tcgttcactt tgagatttcc cccaagaacc tccacaaggt 7500
   cgtccagccc accgtcgcgg tcctcggcga cgtggtcgag aacctcgcca acgtcacgcc 7560
   ccacgtgcag cgccaggagc gcgagccgtg gtttgcgcag atcgccgatt ggaaggagaa 7620
   gcaccctttt ctgctcgagt ctgttgattc ggacgacaag gttctcaagc cgcagcaggt 7680
   cctcacggag cttaacaagc agattctcga gattcaggag aaggacgccg accaggaggt 7740
   ctacatcacc acgggcgtcg gaagccacca gatgcaggca gcgcagttcc ttacctggac 7800
   caagccgcgc cagtggatct cctcgggtgg cgccggcact atgggctacg gccttccctc 7860
   ggccattggc gccaagattg ccaagcccga tgctattgtt attgacatcg atggtgatgc 7920
   ttcttattcg atgaccggta tggaattgat cacagcagcc gaattcaagg ttggcgtgaa 7980
   gattcttctt ttgcagaaca actttcaggg catggtcaag aacgttcagg atctctttta 8040
   cgacaagcgc tactcgggcc accgccatgt tcaacccgcg cttcgacaag gtcgccgatg 8100
   cgatgcgtgc caagggtctc tactgcgcga aacagtcgga gctcaaggac aagatcaagg 8160
   agtttctcga gtacgatgag ggtcccgtcc tcctcgaggt tttcgtggac aaggacacgc 8220
   tcgtcttgcc catggtcccc gctggctttc cgctccacga gatggtcctc gagcctccta 8280
   agcccaagga cgcctaagtt cttttttcca tggcgggcga gcgagcgagc gcgcgagcgc 8340
   gcaagtgcgc aagcgccttg ccttgctttg cttcgcttcg ctttgctttg cttcacacaa 8400
   cctaagtatg aattcaagtt ttcttgcttg tcggcgatgc ctgcctgcca accagccagc 8460
   catccggccg gccgtccttg acgccttcgc ttccggcgcg gccatcgatt caattcaccc 8520
   atccgatacg ttccgccccc tcacgtccgt ctgcgcacga cccctgcacg accacgccaa 8580
   ggccaacgcg ccgctcagct cagcttgtcg acgagtcgca cgtcacatat ctcagatgca 8640
   tttggactgt gagtgttatt atgccactag cacgcaacga tcttcggggt cctcgctcat 8700
   tgcatccgtt cgggccctgc aggcgtggac gcgagtcgcc gccgagacgc tgcagcaggc 8760
   cgctccgacg cgagggctcg agctcgccgc gcccgcgcga tgtctgcctg gcgccgactg 8820
   atctctggag cgcaaggaag acacggcgac gcgaggagga ccgaagagag acgctggggt 8880
   atgcaggata tacccggggc gggacattcg ttccgcatac actcccccat tcgagcttgc 8940
   tcgtccttgg cagagccgag cgcgaacggt tccgaacgcg gcaaggattt tggctctggt 9000
   gggtggactc cgatcgaggc gcaggttctc cgcaggttct cgcaggccgg cagtggtcgt 9060
   tagaaatagg gagtgccgga gtcttgacgc gccttagctc actctccgcc cacgcgcgca 9120
   tcgccgccat gccgccgtcc cgtctgtcgc tgcgctggcc gcgaccggct gcgccagagt 9180
   acgacagtgg gacagagctc gaggcgacgc gaatcgctcg ggttgtaagg gtttcaaggg 9240
   tcgggcgtcg tcgcgtgcca aagtgaaaat agtagggggg ggggggggta c 9291
<210> 13
   <211> 6219
   <212> DNA
   <213> Artificial Sequence
<220>
   <223> vector pCL0123
<400> 13
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg ggggggaggg agaggcagga      840
   cgcgggagcg agcgtgcatg tttccgcgcg agacgacgcc gcgcgcgctg gagaggagat      900
   aaggcgcttg gatcgcgaga gggccagcca ggctggaggc gaaaatgggt ggagaggata      960
   gtatcttgcg tgcttggacg aggagactga cgaggaggac ggatacgtcg atgatgatgt     1020
   gcacagagaa gaagcagttc gaaagcgact actagcaagc aagggatcca tgaagttcgc     1080
   gacctcggtc gcaattttgc ttgtggccaa catagccacc gccctcgcgc agagcgatgg     1140
   ctgcaccccc accgaccaga cgatggtgag caagggcgag gagctgttca ccggggtggt     1200
   gcccatcctg gtcgagctgg acggcgacgt aaacggccac aagttcagcg tgtccggcga     1260
   gggcgagggc gatgccacct acggcaagct gaccctgaag ttcatctgca ccaccggcaa     1320
   gctgcccgtg ccctggccca ccctcgtgac caccctgacc tacggcgtgc agtgcttcag     1380
   ccgctacccc gaccacatga agcagcacga cttcttcaag tccgccatgc ccgaaggcta     1440
   cgtccaggag cgcaccatct tcttcaagga cgacggcaac tacaagaccc gcgccgaggt     1500
   gaagttcgag ggcgacaccc tggtgaaccg catcgagctg aagggcatcg acttcaagga     1560
   ggacggcaac atcctgggac acaagctgga gtacaactac aacagccaca acgtctatat     1620
   catggccgac aagcagaaga acggcatcaa ggtgaacttc aagatccgcc acaacatcga     1680
   ggacggcagc gtgcagctcg ccgaccacta ccagcagaac acccccatcg gcgacggccc     1740
   cgtgctgctg cccgacaacc actacctgag cacccagtcc gccctgagca aagaccccaa     1800
   cgagaagcgc gatcacatgg tcctgctgga gttcgtgacc gccgccggga tcactctcgg     1860
   catggacgag ctgtacaagc accaccatca ccaccactaa catatgagtt atgagatccg     1920
   aaagtgaacc ttgtcctaac ccgacagcga atggcgggag ggggcgggct aaaagatcgt     1980
   attacatagt atttttcccc tactctttgt gtttgtcttt tttttttttt tgaacgcatt     2040
   caagccactt gtctgggttt acttgtttgt ttgcttgctt gcttgcttgc ttgcctgctt     2100
   cttggtcaga cggcccaaaa aagggaaaaa attcattcat ggcacagata agaaaaagaa     2160
   aaagtttgtc gaccaccgtc atcagaaagc aagagaagag aaacactcgc gctcacattc     2220
   tcgctcgcgt aagaatctta gccacgcata cgaagtaatt tgtccatctg gcgaatcttt     2280
   acatgagcgt tttcaagctg gagcgtgaga tcataccttt cttgatcgta atgttccaac     2340
   cttgcatagg cctcgttgcg atccgctagc aatgcgtcgt actcccgttg caactgcgcc     2400
   atcgcctcat tgtgacgtga gttcagattc ttctcgagac cttcgagcgc tgctaatttc     2460
   gcctgacgct ccttcttttg tgcttccatg acacgccgct tcaccgtgcg ttccacttct     2520
   tcctcagaca tgcccttggc tgcctcgacc tgctcggtaa aacgggcccc agcacgtgct     2580
   acgagatttc gattccaccg ccgccttcta tgaaaggttg ggcttcggaa tcgttttccg     2640
   ggacgccggc tggatgatcc tccagcgcgg ggatctcatg ctggagttct tcgcccaccc     2700
   caacttgttt attgcagctt ataatggtta caaataaagc aatagcatca caaatttcac     2760
   aaataaagca tttttttcac tgcattctag ttgtggtttg tccaaactca tcaatgtatc     2820
   ttatcataca tggtcgacct gcaggaacct gcattaatga atcggccaac gcgcggggag     2880
   aggcggtttg cgtattgggc gctcttccgc ttcctcgctc actgactcgc tgcgctcggt     2940
   cgttcggctg cggcgagcgg tatcagctca ctcaaaggcg gtaatacggt tatccacaga     3000
   atcaggggat aacgcaggaa agaacatgtg agcaaaaggc cagcaaaagg ccaggaaccg     3060
   taaaaaggcc gcgttgctgg cgtttttcca taggctccgc ccccctgacg agcatcacaa     3120
   aaatcgacgc tcaagtcaga ggtggcgaaa cccgacagga ctataaagat accaggcgtt     3180
   tccccctgga agctccctcg tgcgctctcc tgttccgacc ctgccgctta ccggatacct     3240
   gtccgccttt ctcccttcgg gaagcgtggc gctttctcat agctcacgct gtaggtatct     3300
   cagttcggtg taggtcgttc gctccaagct gggctgtgtg cacgaacccc ccgttcagcc     3360
   cgaccgctgc gccttatccg gtaactatcg tcttgagtcc aacccggtaa gacacgactt     3420
   atcgccactg gcagcagcca ctggtaacag gattagcaga gcgaggtatg taggcggtgc     3480
   tacagagttc ttgaagtggt ggcctaacta cggctacact agaagaacag tatttggtat     3540
   ctgcgctctg ctgaagccag ttaccttcgg aaaaagagtt ggtagctctt gatccggcaa     3600
   acaaaccacc gctggtagcg gtggtttttt tgtttgcaag cagcagatta cgcgcagaaa     3660
   aaaaggatct caagaagatc ctttgatctt ttctacgggg tctgacgctc agtggaacga     3720
   aaactcacgt taagggattt tggtcatgag attatcaaaa aggatcttca cctagatcct     3780
   tttaaattaa aaatgaagtt ttaaatcaat ctaaagtata tatgagtaaa cttggtctga     3840
   cagttaccaa tgcttaatca gtgaggcacc tatctcagcg atctgtctat ttcgttcatc     3900
   catagttgcc tgactccccg tcgtgtagat aactacgata cgggagggct taccatctgg     3960
   ccccagtgct gcaatgatac cgcgagaccc acgctcaccg gctccagatt tatcagcaat     4020
   aaaccagcca gccggaaggg ccgagcgcag aagtggtcct gcaactttat ccgcctccat     4080
   ccagtctatt aattgttgcc gggaagctag agtaagtagt tcgccagtta atagtttgcg     4140
   caacgttgtt gccattgcta caggcatcgt ggtgtcacgc tcgtcgtttg gtatggcttc     4200
   attcagctcc ggttcccaac gatcaaggcg agttacatga tcccccatgt tgtgcaaaaa     4260
   agcggttagc tccttcggtc ctccgatcgt tgtcagaagt aagttggccg cagtgttatc     4320
   actcatggtt atggcagcac tgcataattc tcttactgtc atgccatccg taagatgctt     4380
   ttctgtgact ggtgagtact caaccaagtc attctgagaa tagtgtatgc ggcgaccgag     4440
   ttgctcttgc ccggcgtcaa tacgggataa taccgcgcca catagcagaa ctttaaaagt     4500
   gctcatcatt ggaaaacgtt cttcggggcg aaaactctca aggatcttac cgctgttgag     4560
   atccagttcg atgtaaccca ctcgtgcacc caactgatct tcagcatctt ttactttcac     4620
   cagcgtttct gggtgagcaa aaacaggaag gcaaaatgcc gcaaaaaagg gaataagggc     4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatgatgcc tttgtctcaa gaagaatcca ccctcattga 5580
   aagagcaacg gctacaatca acagcatccc catctctgaa gactacagcg tcgccagcgc 5640
   agctctctct agcgacggcc gcatcttcac tggtgtcaat gtatatcatt ttactggggg 5700
   accttgtgca gaactcgtgg tgctgggcac tgctgctgct gcggcagctg gcaacctgac 5760
   ttgtatcgtc gcgatcggaa atgagaacag gggcatcttg agcccctgtg gacggtgccg 5820
   acaggtgctt ctcgatctgc atcctgggat caaagccata gtgaaggaca gtgatggaca 5880
   gccgacggca gttgggattc gtgaattgct gccctctggt tatgtgtggg agggctaaca 5940
   cgtgctccgt gctacgagat ttcgattcca ccgccgcctt ctatgaaagg ttgggcttcg 6000
   gaatcgtttt ccgggacgcc ggctggatga tcctccagcg cggggatctc atgctggagt 6060
   tcttcgccca ccccaacttg tttattgcag cttataatgg ttacaaataa agcaatagca 6120
   tcacaaattt cacaaataaa gcattttttt cactgcattc tagttgtggt ttgtccaaac 6180
   tcatcaatgt atcttatcat gtctgaattc ccggggtac 6219
<210> 14
   <211> 10029
   <212> DNA
   <213> Artificial Sequence
<220>
   <223> vector pCL0130
<400> 14
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc    120
   gctagctcgc tcgcgccgtg ctgcagccag cagggcagca ccgcacggca ggcaggtccc    180
   ggcgcggatc gatcgatcca tcgatccatc gatccatcga tcgtgcggtc aaaaagaaag    240
   gaagaagaaa ggaaaaagaa aggcgtgcgc acccgagtgc gcgctgagcg cccgctcgcg    300
   gtcccgcgga gcctccgcgt tagtccccgc cccgcgccgc gcagtccccc gggaggcatc    360
   gcgcacctct cgccgccccc tcgcgcctcg ccgattcccc gcctcccctt ttccgcttct    420
   tcgccgcctc cgctcgcggc cgcgtcgccc gcgccccgct ccctatctgc tccccagggg    480
   ggcactccgc accttttgcg cccgctgccg ccgccgcggc cgccccgccg ccctggtttc    540
   ccccgcgagc gcggccgcgt cgccgcgcaa agactcgccg cgtgccgccc cgagcaacgg    600
   gtggcggcgg cgcggcggcg ggcggggcgc ggcggcgcgt aggcggggct aggcgccggc    660
   taggcgaaac gccgcccccg ggcgccgccg ccgcccgctc cagagcagtc gccgcgccag    720
   accgccaacg cagagaccga gaccgaggta cgtcgcgccc gagcacgccg cgacgcgcgg    780
   cagggacgag gagcacgacg ccgcgccgcg ccgcgcgggg gggggagggg agaggcagga     840
   cgcgggagcg agcgtgcatg tttccgcgcg agacgacgcc gcgcgcgctg gagaggagat    900
   aaggcgcttg gatcgcgaga gggccagcca ggctggaggc gaaaatgggt ggagaggata    960
   gtatcttgcg tgcttggacg aggagactga cgaggaggac ggatacgtcg atgatgatgt   1020
   gcacagagaa gaagcagttc gaaagcgact actagcaagc aagggatcca tgggcctcga   1080
   ggataaccgc atggttaagc gctttgtcaa cgtgggcgag aagaaggccg gtagcaccgc   1140
   catggccatc attgttggcc tcttcgcggc ctcgggcggc gtcctcttcg gctacgacac   1200
   cggcactatc tcgggcgtca tgactatgga ctacgttctc gcccgctacc cctccaacaa   1260
   gcactccttc accgctgacg agtcgtcgct catcgtttcc attctttcgg tcggcacctt   1320
   cttcggcgcc ctctgcgccc cgttcctcaa cgataccctc ggccgccgct ggtgcctcat   1380
   cctcagcgcc ctcattgtct ttaacatcgg cgccatcctc caggtcattt ccaccgccat   1440
   ccccctgctc tgcgcgggcc gcgttatcgc cggtttcggt gtcggcctca tttccgccac   1500
   catcccgctc taccagtccg agactgctcc gaagtggatt cgcggcgcca tcgtttcctg   1560
   ctaccagtgg gccatcacta tcggactttt cctcgcttcc tgcgtcaaca agggcaccga   1620
   gcacatgacc aactccggtt cgtaccgtat tcctctggcc atccagtgcc tctggggcct   1680
   catccttggt attggcatga ttttcctccc tgagaccccc cgcttctgga tttcgaaggg   1740
   caaccaggag aaggccgccg agtccctcgc ccgtctccgc aagctcccca tcgaccatcc   1800
   tgatagcctt gaggagcttc gcgatattac tgccgcctac gagttcgaga ccgtctacgg   1860
   taagtccagc tggtcccagg tcttttccca caagaaccat cagctcaagc gcctctttac   1920
   cggcgttgcc attcaggcct ttcagcagct caccggagtt aactttatct tttactacgg   1980
   caccaccttt tttaagcgcg ccggagtcaa cggattcacc atcagccttg ccaccaacat   2040
   cgttaacgtc ggcagcacta ttcccggcat tcttctcatg gaggtcctcg gccgccgcaa 2100
   catgctcatg ggcggtgcca ccggcatgtc gctgtcgcag cttatcgtcg ccattgtcgg 2160
   agttgccacg tcggagaaca acaagtcgag ccagtcggtc ctcgtcgctt tctcgtgcat 2220
   ctttatcgct ttttttgccg ccacctgggg tccctgcgcc tgggtcgtcg tcggcgagct 2280
   ctttcccctt cgcactcgcg ctaagtccgt ttccctctgc accgcgtcca actggctctg 2340
   gaactggggc attgcttacg ccacccccta catggtcgac gaggataagg gtaacctcgg 2400
   cagcaacgtt ttttttattt ggggaggctt caacctcgct tgcgtctttt tcgcgtggta 2460
   cttcatttac gagaccaagg gcctttccct cgagcaggtt gatgagctct acgagcatgt 2520
   ttcgaaggcg tggaagtcca agggttttgt cccgtccaag cactcctttc gcgagcaggt 2580
   cgaccagcag atggactcca agaccgaggc cattatgagc gaggaggcgt cggtttaaca 2640
   tatgagttat gagatccgaa agtgaacctt gtcctaaccc gacagcgaat ggcgggaggg 2700
   ggcgggctaa aagatcgtat tacatagtat tttcccccta ctctttgtgt ttgtcttttt 2760
   tttttttttg aacgcattca agccacttgt ctgggtttac ttgtttgttt gcttgcttgc 2820
   ttgcttgctt gcctgcttct tggtcagacg gcccaaaaaa gggaaaaaat tcattcatgg 2880
   cacagataag aaaaagaaaa agtttgtcga ccaccgtcat cagaaagcaa gagaagagaa 2940
   acactcgcgc tcacattctc gctcgcgtaa gaatcttagc cacgcatacg aagtaatttg 3000
   tccatctggc gaatctttac atgagcgttt tcaagctgga gcgtgagatc atacctttct 3060
   tgatcgtaat gttccaacct tgcataggcc tcgttgcgat ccgctagcaa tgcgtcgtac 3120
   tcccgttgca actgcgccat cgcctcattg tgacgtgagt tcagattctt ctcgagacct 3180
   tcgagcgctg ctaatttcgc ctgacgctcc ttcttttgtg cttccatgac acgccgcttc 3240
   accgtgcgtt ccacttcttc ctcagacatg cccttggctg cctcgacctg ctcggtaaaa 3300
   cgggccccag cacgtgctac gagatttcga ttccaccgcc gccttctatg aaaggttggg 3360
   cttcggaatc gttttccggg acgccggctg gatgatcctc cagcgcgggg atctcatgct 3420
   ggagttcttc gcccacccca acttgtttat tgcagcttat aatggttaca aataaagcaa 3480
   tagcatcaca aatttcacaa ataaagcatt tttttcactg cattctagtt gtggtttgtc 3540
   caaactcatc aatgtatctt atcatacatg gtcgacctgc aggaacctgc attaatgaat 3600
   cggccaacgc gcggggagag gcggtttgcg tattgggcgc tcttccgctt cctcgctcac 3660
   tgactcgctg cgctcggtcg ttcggctgcg gcgagcggta tcagctcact caaaggcggt 3720
   aatacggtta tccacagaat caggggataa cgcaggaaag aacatgtgag caaaaggcca 3780
   gcaaaaggcc aggaaccgta aaaaggccgc gttgctggcg tttttccata ggctccgccc 3840
   ccctgacgag catcacaaaa atcgacgctc aagtcagagg tggcgaaacc cgacaggact 3900
   ataaagatac caggcgtttc cccctggaag ctccctcgtg cgctctcctg ttccgaccct 3960
   gccgcttacc ggatacctgt ccgcctttct cccttcggga agcgtggcgc tttctcatag 4020
   ctcacgctgt aggtatctca gttcggtgta ggtcgttcgc tccaagctgg gctgtgtgca 4080
   cgaacccccc gttcagcccg accgctgcgc cttatccggt aactatcgtc ttgagtccaa 4140
   cccggtaaga cacgacttat cgccactggc agcagccact ggtaacagga ttagcagagc 4200
   gaggtatgta ggcggtgcta cagagttctt gaagtggtgg cctaactacg gctacactag 4260
   aagaacagta tttggtatct gcgctctgct gaagccagtt accttcggaa aaagagttgg 4320
   tagctcttga tccggcaaac aaaccaccgc tggtagcggt ggtttttttg tttgcaagca 4380
   gcagattacg cgcagaaaaa aaggatctca agaagatcct ttgatctttt ctacggggtc 4440
   tgacgctcag tggaacgaaa actcacgtta agggattttg gtcatgagat tatcaaaaag 4500
   gatcttcacc tagatccttt taaattaaaa atgaagtttt aaatcaatct aaagtatata 4560
   tgagtaaact tggtctgaca gttaccaatg cttaatcagt gaggcaccta tctcagcgat 4620
   ctgtctattt cgttcatcca tagttgcctg actccccgtc gtgtagataa ctacgatacg 4680
   ggagggctta ccatctggcc ccagtgctgc aatgataccg cgagacccac gctcaccggc 4740
   tccagattta tcagcaataa accagccagc cggaagggcc gagcgcagaa gtggtcctgc 4800
   aactttatcc gcctccatcc agtctattaa ttgttgccgg gaagctagag taagtagttc 4860
   gccagttaat agtttgcgca acgttgttgc cattgctaca ggcatcgtgg tgtcacgctc 4920
   gtcgtttggt atggcttcat tcagctccgg ttcccaacga tcaaggcgag ttacatgatc 4980
   ccccatgttg tgcaaaaaag cggttagctc cttcggtcct ccgatcgttg tcagaagtaa 5040
   gttggccgca gtgttatcac tcatggttat ggcagcactg cataattctc ttactgtcat 5100
   gccatccgta agatgctttt ctgtgactgg tgagtactca accaagtcat tctgagaata 5160
   gtgtatgcgg cgaccgagtt gctcttgccc ggcgtcaata cgggataata ccgcgccaca 5220
   tagcagaact ttaaaagtgc tcatcattgg aaaacgttct tcggggcgaa aactctcaag 5280
   gatcttaccg ctgttgagat ccagttcgat gtaacccact cgtgcaccca actgatcttc 5340
   agcatctttt actttcacca gcgtttctgg gtgagcaaaa acaggaaggc aaaatgccgc 5400
   aaaaaaggga ataagggcga cacggaaatg ttgaatactc atactcttcc tttttcaata 5460
   ttattgaagc atttatcagg gttattgtct catgagcgga tacatatttg aatgtattta 5520
   gaaaaataaa caaatagggg ttccgcgcac atttccccga aaagtgccac ctgacgtcta 5580
   agaaaccatt attatcatga cattaaccta taaaaatagg cgtatcacga ggccctttcg 5640
   tctcgcgcgt ttcggtgatg acggtgaaaa cctctgacac atgcagctcc cggagacggt 5700
   cacagcttgt ctgtaagcgg atgccgggag cagacaagcc cgtcagggcg cgtcagcggg 5760
   tgttggcggg tgtcggggct ggcttaacta tgcggcatca gagcagattg tactgagagt 5820
   gcaccaagct ttgcctcaac gcaactaggc ccaggcctac tttcactgtg tcttgtcttg 5880
   cctttcacac cgaccgagtg tgcacaaccg tgttttgcac aaagcgcaag atgctcactc 5940
   gactgtgaag caaaggttgc gcgcaagcga ctgcgactgc gaggatgagg atgactggca 6000
   gcctgttcaa aaactgaaaa tccgcgatgg gtcagctgcc attcgcgcat gacgcctgcg 6060
   agagacaagt taactcgtgt cactggcatg tcctagcatc tttacgcgag caaaattcaa 6120
   tcgctttatt ttttcagttt cgtaaccttc tcgcaaccgc gaatcgccgt ttcagcctga 6180
   ctaatctgca gctgcgtggc actgtcagtc agtcagtcag tcgtgcgcgc tgttccagca 6240
   ccgaggtcgc gcgtcgccgc gcctggaccg ctgctgctac tgctagtggc acggcaggta 6300
   ggagcttgtt gccggaacac cagcagccgc cagtcgacgc cagccagggg aaagtccggc 6360
   gtcgaaggga gaggaaggcg gcgtgtgcaa actaacgttg accactggcg cccgccgaca 6420
   cgagcaggaa gcaggcagct gcagagcgca gcgcgcaagt gcagaatgcg cgaaagatcc 6480
   acttgcgcgc ggcgggcgcg cacttgcggg cgcggcgcgg aacagtgcgg aaaggagcgg 6540
   tgcagacggc gcgcagtgac agtgggcgca aagccgcgca gtaagcagcg gcggggaacg 6600
   gtatacgcag tgccgcgggc cgccgcacac agaagtatac gcgggccgaa gtggggcgtc 6660
   gcgcgcggga agtgcggaat ggcgggcaag gaaaggagga gacggaaaga gggcgggaaa 6720
   gagagagaga gagagtgaaa aaagaaagaa agaaagaaag aaagaaagaa agctcggagc 6780
   cacgccgcgg ggagagagag aaatgaaagc acggcacggc aaagcaaagc aaagcagacc 6840
   cagccagacc cagccgaggg aggagcgcgc gcaggacccg cgcggcgagc gagcgagcac 6900
   ggcgcgcgag cgagcgagcg agcgagcgcg cgagcgagca aggcttgctg cgagcgatcg 6960
   agcgagcgag cgggaaggat gagcgcgacc cgcgcggcga cgaggacagc ggcggcgctg 7020
   tcctcggcgc tgacgacgcc tgtaaagcag cagcagcagc agcagctgcg cgtaggcgcg 7080
   gcgtcggcac ggctggcggc cgcggcgttc tcgtccggca cgggcggaga cgcggccaag 7140
   aaggcggccg cggcgagggc gttctccacg ggacgcggcc ccaacgcgac acgcgagaag 7200
   agctcgctgg ccacggtcca ggcggcgacg gacgatgcgc gcttcgtcgg cctgaccggc 7260
   gcccaaatct ttcatgagct catgcgcgag caccaggtgg acaccatctt tggctaccct 7320
   ggcggcgcca ttctgcccgt ttttgatgcc atttttgaga gtgacgcgct tcaagttcat 7380
   tctcgctcgc cacgagcagg gcgccggcca catggccgag ggctacgcgc gcgccacggg 7440
   caagcccggc gttgtcctcg tcacctcggg ccctggagcc accaacacca tcaccccgat 7500
   catggatgct tacatggacg gtacgccgct gctcgtgttc accggccagg tgcagacctc 7560
   tgctgtcggc acggacgctt tccaggagtg tgacattgtt ggcatcagcc gcgcgtgcac 7620
   caagtggaac gtcatggtca aggacgtgaa ggagctcccg cgccgcatca atgaggcctt 7680
   tgagattgcc atgagcggcc gcccgggtcc cgtgctcgtc gatcttccta aggatgtgac 7740
   cgccgttgag ctcaaggaaa tgcccgacag ctccccccag gttgctgtgc gccagaagca 7800
   aaaggtcgag cttttccaca aggagcgcat tggcgctcct ggcacggccg acttcaagct 7860
   cattgccgag atgatcaacc gtgcggagcg acccgtcatc tatgctggcc agggtgtcat 7920
   gcagagcccg ttgaatggcc cggctgtgct caaggagttc gcggagaagg ccaacattcc 7980
   cgtgaccacc accatgcagg gtctcggcgg ctttgacgag cgtagtcccc tctccctcaa 8040
   gatgctcggc atgcacggct ctgcctacgc caactactcg atgcagaacg ccgatcttat 8100
   cctggcgctc ggtgcccgct ttgatgatcg tgtgacgggc cgcgttgacg cctttgctcc 8160
   ggaggctcgc cgtgccgagc gcgagggccg cggtggcatc gttcactttg agatttcccc 8220
   caagaacctc cacaaggtcg tccagcccac cgtcgcggtc ctcggcgacg tggtcgagaa 8280
   cctcgccaac gtcacgcccc acgtgcagcg ccaggagcgc gagccgtggt ttgcgcagat 8340
   cgccgattgg aaggagaagc acccttttct gctcgagtct gttgattcgg acgacaaggt 8400
   tctcaagccg cagcaggtcc tcacggagct taacaagcag attctcgaga ttcaggagaa 8460
   ggacgccgac caggaggtct acatcaccac gggcgtcgga agccaccaga tgcaggcagc 8520
   gcagttcctt acctggacca agccgcgcca gtggatctcc tcgggtggcg ccggcactat 8580
   gggctacggc cttccctcgg ccattggcgc caagattgcc aagcccgatg ctattgttat 8640
   tgacatcgat ggtgatgctt cttattcgat gaccggtatg gaattgatca cagcagccga 8700
   attcaaggtt ggcgtgaaga ttcttctttt gcagaacaac tttcagggca tggtcaagaa 8760
   cgttcaggat ctcttttacg acaagcgcta ctcgggccac cgccatgttc aacccgcgct 8820
   tcgacaaggt cgccgatgcg atgcgtgcca agggtctcta ctgcgcgaaa cagtcggagc 8880
   tcaaggacaa gatcaaggag tttctcgagt acgatgaggg tcccgtcctc ctcgaggttt 8940
   tcgtggacaa ggacacgctc gtcttgccca tggtccccgc tggctttccg ctccacgaga 9000
   tggtcctcga gcctcctaag cccaaggacg cctaagttct tttttccatg gcgggcgagc 9060
   gagcgagcgc gcgagcgcgc aagtgcgcaa gcgccttgcc ttgctttgct tcgcttcgct 9120
   ttgctttgct tcacacaacc taagtatgaa ttcaagtttt cttgcttgtc ggcgatgcct 9180
   gcctgccaac cagccagcca tccggccggc cgtccttgac gccttcgctt ccggcgcggc 9240
   catcgattca attcacccat ccgatacgtt ccgccccctc acgtccgtct gcgcacgacc 9300
   cctgcacgac cacgccaagg ccaacgcgcc gctcagctca gcttgtcgac gagtcgcacg 9360
   tcacatatct cagatgcatt tggactgtga gtgttattat gccactagca cgcaacgatc 9420
   ttcggggtcc tcgctcattg catccgttcg ggccctgcag gcgtggacgc gagtcgccgc 9480
   cgagacgctg cagcaggccg ctccgacgcg agggctcgag ctcgccgcgc ccgcgcgatg 9540
   tctgcctggc gccgactgat ctctggagcg caaggaagac acggcgacgc gaggaggacc 9600
   gaagagagac gctggggtat gcaggatata cccggggcgg gacattcgtt ccgcatacac 9660
   tcccccattc gagcttgctc gtccttggca gagccgagcg cgaacggttc cgaacgcggc 9720
   aaggattttg gctctggtgg gtggactccg atcgaggcgc aggttctccg caggttctcg 9780
   caggccggca gtggtcgtta gaaataggga gtgccggagt cttgacgcgc cttagctcac 9840
   tctccgccca cgcgcgcatc gccgccatgc cgccgtcccg tctgtcgctg cgctggccgc 9900
   gaccggctgc gccagagtac gacagtggga cagagctcga ggcgacgcga atcgctcggg 9960
   ttgtaagggt ttcaagggtc gggcgtcgtc gcgtgccaaa gtgaaaatag tagggggggg 10020
   ggggggtac 10029
<210> 15
   <211> 9972
   <212> DNA
   <213> Artificial Sequence
<220>
   <223> vector pCL0131
<400> 15
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg gggggagggg agaggcagga     840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tggccctcga    1080
   ccctgagcag cagcagccca tttcctccgt gtcgcgcgag tttggtaagt cgtccggtga    1140
   gatctccccc gagcgtgagc ctctcattaa ggagaaccac gtccccgaga actactccgt    1200
   tgttgccgcc atcctcccct tcctcttccc ggccctgggt ggcctccttt acggttacga    1260
   gattggcgct acgtcgtgcg ctacgatttc ccttcagtcc ccctccctct ccggcatctc    1320
   ctggtacaac ctctcctccg tcgatgttgg cctcgtcact tccggttccc tctacggtgc    1380
   tctgtttggc tccattgttg ccttcaccat tgccgacgtt attggccgtc gcaaggagct 1440
   tatcctcgct gctctcctct acctcgtcgg tgccctcgtt accgctctcg cccctacgta 1500
   ctccgttctc atcatcggcc gtgtcattta cggtgtttcc gtcggtcttg ccatgcatgc 1560
   tgcccctatg tacatcgcgg agaccgcccc gtcccccatc cgcggccagc tcgtttccct 1620
   caaggagttt ttcatcgttc tcggtatggt cggcggatac ggcattggtt ccctcaccgt 1680
   caacgtccac tccggttggc gctacatgta cgctacctcc gttcccctcg ctgtgatcat 1740
   gggcattggc atgtggtggc ttcctgcctc cccccgttgg ctcctcctcc gcgtcattca 1800
   gggtaagggt aacgttgaga accagcgcga ggctgccatt aagtccctct gctgcctccg 1860
   tggtcctgcc ttcgtcgact cggccgccga gcaggtcaac gagattctcg ccgagcttac 1920
   cttcgttggc gaggataagg aggtcacctt cggcgagctc ttccagggaa agtgcctcaa 1980
   ggccctcatt atcggcggcg gccttgttct ctttcagcag atcaccggtc agccttcggt 2040
   cctctactac gccccctcga tcctccagac tgcgggcttc tccgccgccg gcgatgctac 2100
   ccgcgtttcc attcttctcg gcctcctcaa gctcattatg accggtgtcg ccgtcgtcgt 2160
   tatcgatcgt ctcggccgtc gccctctcct cctcggcgga gtcggtggta tggttgtttc 2220
   gctctttctc cttggctcgt actacctttt cttcagcgct tcccccgtcg tcgccgttgt 2280
   cgccctcctt ctctacgtgg gttgctacca gctctccttt ggccccattg gctggcttat 2340
   gatttccgag atttttcccc tcaagctccg tggtcgcgga ctctcccttg ccgtgcttgt 2400
   caactttggt gccaacgccc tcgtcacctt tgccttttcc cctctcaagg agctcctcgg 2460
   cgccggcatc ctgttttgcg gctttggcgt tatctgcgtt ctctcccttg tttttatctt 2520
   ttttatcgtc ccggagacta agggcctcac gctcgaggag atcgaggcga agtgcctcta 2580
   acatatgagt tatgagatcc gaaagtgaac cttgtcctaa cccgacagcg aatggcggga 2640
   gggggcgggc taaaagatcg tattacatag tatttttccc ctactctttg tgtttgtctt 2700
   tttttttttt ttgaacgcat tcaagccact tgtctgggtt tacttgtttg tttgcttgct 2760
   tgcttgcttg cttgcctgct tcttggtcag acggcccaaa aaagggaaaa aattcattca 2820
   tggcacagat aagaaaaaga aaaagtttgt cgaccaccgt catcagaaag caagagaaga 2880
   gaaacactcg cgctcacatt ctcgctcgcg taagaatctt agccacgcat acgaagtaat 2940
   ttgtccatct ggcgaatctt tacatgagcg ttttcaagct ggagcgtgag atcatacctt 3000
   tcttgatcgt aatgttccaa ccttgcatag gcctcgttgc gatccgctag caatgcgtcg 3060
   tactcccgtt gcaactgcgc catcgcctca ttgtgacgtg agttcagatt cttctcgaga 3120
   ccttcgagcg ctgctaattt cgcctgacgc tccttctttt gtgcttccat gacacgccgc 3180
   ttcaccgtgc gttccacttc ttcctcagac atgcccttgg ctgcctcgac ctgctcggta 3240
   aaacgggccc cagcacgtgc tacgagattt cgattccacc gccgccttct atgaaaggtt 3300
   gggcttcgga atcgttttcc gggacgccgg ctggatgatc ctccagcgcg gggatctcat 3360
   gctggagttc ttcgcccacc ccaacttgtt tattgcagct tataatggtt acaaataaag 3420
   caatagcatc acaaatttca caaataaagc atttttttca ctgcattcta gttgtggttt 3480
   gtccaaactc atcaatgtat cttatcatac atggtcgacc tgcaggaacc tgcattaatg 3540
   aatcggccaa cgcgcgggga gaggcggttt gcgtattggg cgctcttccg cttcctcgct 3600
   cactgactcg ctgcgctcgg tcgttcggct gcggcgagcg gtatcagctc actcaaaggc 3660
   ggtaatacgg ttatccacag aatcagggga taacgcagga aagaacatgt gagcaaaagg 3720
   ccagcaaaag gccaggaacc gtaaaaaggc cgcgttgctg gcgtttttcc ataggctccg 3780
   cccccctgac gagcatcaca aaaatcgacg ctcaagtcag aggtggcgaa acccgacagg 3840
   actataaaga taccaggcgt ttccccctgg aagctccctc gtgcgctctc ctgttccgac 3900
   cctgccgctt accggatacc tgtccgcctt tctcccttcg ggaagcgtgg cgctttctca 3960
   tagctcacgc tgtaggtatc tcagttcggt gtaggtcgtt cgctccaagc tgggctgtgt 4020
   gcacgaaccc cccgttcagc ccgaccgctg cgccttatcc ggtaactatc gtcttgagtc 4080
   caacccggta agacacgact tatcgccact ggcagcagcc actggtaaca ggattagcag 4140
   agcgaggtat gtaggcggtg ctacagagtt cttgaagtgg tggcctaact acggctacac 4200
   tagaagaaca gtatttggta tctgcgctct gctgaagcca gttaccttcg gaaaaagagt 4260
   tggtagctct tgatccggca aacaaaccac cgctggtagc ggtggttttt ttgtttgcaa 4320
   gcagcagatt acgcgcagaa aaaaaggatc tcaagaagat cctttgatct tttctacggg 4380
   gtctgacgct cagtggaacg aaaactcacg ttaagggatt ttggtcatga gattatcaaa 4440
   aaggatcttc acctagatcc ttttaaatta aaaatgaagt tttaaatcaa tctaaagtat 4500
   atatgagtaa acttggtctg acagttacca atgcttaatc agtgaggcac ctatctcagc 4560
   gatctgtcta tttcgttcat ccatagttgc ctgactcccc gtcgtgtaga taactacgat 4620
   acgggagggc ttaccatctg gccccagtgc tgcaatgata ccgcgagacc cacgctcacc 4680
   ggctccagat ttatcagcaa taaaccagcc agccggaagg gccgagcgca gaagtggtcc 4740
   tgcaacttta tccgcctcca tccagtctat taattgttgc cgggaagcta gagtaagtag 4800
   ttcgccagtt aatagtttgc gcaacgttgt tgccattgct acaggcatcg tggtgtcacg 4860
   ctcgtcgttt ggtatggctt cattcagctc cggttcccaa cgatcaaggc gagttacatg 4920
   atcccccatg ttgtgcaaaa aagcggttag ctccttcggt cctccgatcg ttgtcagaag 4980
   taagttggcc gcagtgttat cactcatggt tatggcagca ctgcataatt ctcttactgt 5040
   catgccatcc gtaagatgct tttctgtgac tggtgagtac tcaaccaagt cattctgaga 5100
   atagtgtatg cggcgaccga gttgctcttg cccggcgtca atacgggata ataccgcgcc 5160
   acatagcaga actttaaaag tgctcatcat tggaaaacgt tcttcggggc gaaaactctc 5220
   aaggatctta ccgctgttga gatccagttc gatgtaaccc actcgtgcac ccaactgatc 5280
   ttcagcatct tttactttca ccagcgtttc tgggtgagca aaaacaggaa ggcaaaatgc 5340
   cgcaaaaaag ggaataaggg cgacacggaa atgttgaata ctcatactct tcctttttca 5400
   atattattga agcatttatc agggttattg tctcatgagc ggatacatat ttgaatgtat 5460
   ttagaaaaat aaacaaatag gggttccgcg cacatttccc cgaaaagtgc cacctgacgt 5520
   ctaagaaacc attattatca tgacattaac ctataaaaat aggcgtatca cgaggccctt 5580
   tcgtctcgcg cgtttcggtg atgacggtga aaacctctga cacatgcagc tcccggagac 5640
   ggtcacagct tgtctgtaag cggatgccgg gagcagacaa gcccgtcagg gcgcgtcagc 5700
   gggtgttggc gggtgtcggg gctggcttaa ctatgcggca tcagagcaga ttgtactgag 5760
   agtgcaccaa gctttgcctc aacgcaacta ggcccaggcc tactttcact gtgtcttgtc 5820
   ttgcctttca caccgaccga gtgtgcacaa ccgtgttttg cacaaagcgc aagatgctca 5880
   ctcgactgtg aagcaaaggt tgcgcgcaag cgactgcgac tgcgaggatg aggatgactg 5940
   gcagcctgtt caaaaactga aaatccgcga tgggtcagct gccattcgcg catgacgcct 6000
   gcgagagaca agttaactcg tgtcactggc atgtcctagc atctttacgc gagcaaaatt 6060
   caatcgcttt attttttcag tttcgtaacc ttctcgcaac cgcgaatcgc cgtttcagcc 6120
   tgactaatct gcagctgcgt ggcactgtca gtcagtcagt cagtcgtgcg cgctgttcca 6180
   gcaccgaggt cgcgcgtcgc cgcgcctgga ccgctgctgc tactgctagt ggcacggcag 6240
   gtaggagctt gttgccggaa caccagcagc cgccagtcga cgccagccag gggaaagtcc 6300
   ggcgtcgaag ggagaggaag gcggcgtgtg caaactaacg ttgaccactg gcgcccgccg 6360
   acacgagcag gaagcaggca gctgcagagc gcagcgcgca agtgcagaat gcgcgaaaga 6420
   tccacttgcg cgcggcgggc gcgcacttgc gggcgcggcg cggaacagtg cggaaaggag 6480
   cggtgcagac ggcgcgcagt gacagtgggc gcaaagccgc gcagtaagca gcggcgggga 6540
   acggtatacg cagtgccgcg ggccgccgca cacagaagta tacgcgggcc gaagtggggc 6600
   gtcgcgcgcg ggaagtgcgg aatggcgggc aaggaaagga ggagacggaa agagggcggg 6660
   aaagagagag agagagagtg aaaaaagaaa gaaagaaaga aagaaagaaa gaaagctcgg 6720
   agccacgccg cggggagaga gagaaatgaa agcacggcac ggcaaagcaa agcaaagcag 6780
   acccagccag acccagccga gggaggagcg cgcgcaggac ccgcgcggcg agcgagcgag 6840
   cacggcgcgc gagcgagcga gcgagcgagc gcgcgagcga gcaaggcttg ctgcgagcga 6900
   tcgagcgagc gagcgggaag gatgagcgcg acccgcgcgg cgacgaggac agcggcggcg 6960
   ctgtcctcgg cgctgacgac gcctgtaaag cagcagcagc agcagcagct gcgcgtaggc 7020
   gcggcgtcgg cacggctggc ggccgcggcg ttctcgtccg gcacgggcgg agacgcggcc 7080
   aagaaggcgg ccgcggcgag ggcgttctcc acgggacgcg gccccaacgc gacacgcgag 7140
   aagagctcgc tggccacggt ccaggcggcg acggacgatg cgcgcttcgt cggcctgacc 7200
   ggcgcccaaa tctttcatga gctcatgcgc gagcaccagg tggacaccat ctttggctac 7260
   cctggcggcg ccattctgcc cgtttttgat gccatttttg agagtgacgc gcttcaagtt 7320
   cattctcgct cgccacgagc agggcgccgg ccacatggcc gagggctacg cgcgcgccac 7380
   gggcaagccc ggcgttgtcc tcgtcacctc gggccctgga gccaccaaca ccatcacccc 7440
   gatcatggat gcttacatgg acggtacgcc gctgctcgtg ttcaccggcc aggtgcagac 7500
   ctctgctgtc ggcacggacg ctttccagga gtgtgacatt gttggcatca gccgcgcgtg 7560
   caccaagtgg aacgtcatgg tcaaggacgt gaaggagctc ccgcgccgca tcaatgaggc 7620
   ctttgagatt gccatgagcg gccgcccggg tcccgtgctc gtcgatcttc ctaaggatgt 7680
   gaccgccgtt gagctcaagg aaatgcccga cagctccccc caggttgctg tgcgccagaa 7740
   gcaaaaggtc gagcttttcc acaaggagcg cattggcgct cctggcacgg ccgacttcaa 7800
   gctcattgcc gagatgatca accgtgcgga gcgacccgtc atctatgctg gccagggtgt 7860
   catgcagagc ccgttgaatg gcccggctgt gctcaaggag ttcgcggaga aggccaacat 7920
   tcccgtgacc accaccatgc agggtctcgg cggctttgac gagcgtagtc ccctctccct 7980
   caagatgctc ggcatgcacg gctctgccta cgccaactac tcgatgcaga acgccgatct 8040
   tatcctggcg ctcggtgccc gctttgatga tcgtgtgacg ggccgcgttg acgcctttgc 8100
   tccggaggct cgccgtgccg agcgcgaggg ccgcggtggc atcgttcact ttgagatttc 8160
   ccccaagaac ctccacaagg tcgtccagcc caccgtcgcg gtcctcggcg acgtggtcga 8220
   gaacctcgcc aacgtcacgc cccacgtgca gcgccaggag cgcgagccgt ggtttgcgca 8280
   gatcgccgat tggaaggaga agcacccttt tctgctcgag tctgttgatt cggacgacaa 8340
   ggttctcaag ccgcagcagg tcctcacgga gcttaacaag cagattctcg agattcagga 8400
   gaaggacgcc gaccaggagg tctacatcac cacgggcgtc ggaagccacc agatgcaggc 8460
   agcgcagttc cttacctgga ccaagccgcg ccagtggatc tcctcgggtg gcgccggcac 8520
   tatgggctac ggccttccct cggccattgg cgccaagatt gccaagcccg atgctattgt 8580
   tattgacatc gatggtgatg cttcttattc gatgaccggt atggaattga tcacagcagc 8640
   cgaattcaag gttggcgtga agattcttct tttgcagaac aactttcagg gcatggtcaa 8700
   gaacgttcag gatctctttt acgacaagcg ctactcgggc caccgccatg ttcaacccgc 8760
   gcttcgacaa ggtcgccgat gcgatgcgtg ccaagggtct ctactgcgcg aaacagtcgg 8820
   agctcaagga caagatcaag gagtttctcg agtacgatga gggtcccgtc ctcctcgagg 8880
   ttttcgtgga caaggacacg ctcgtcttgc ccatggtccc cgctggcttt ccgctccacg 8940
   agatggtcct cgagcctcct aagcccaagg acgcctaagt tcttttttcc atggcgggcg 9000
   agcgagcgag cgcgcgagcg cgcaagtgcg caagcgcctt gccttgcttt gcttcgcttc 9060
   gctttgcttt gcttcacaca acctaagtat gaattcaagt tttcttgctt gtcggcgatg 9120
   cctgcctgcc aaccagccag ccatccggcc ggccgtcctt gacgccttcg cttccggcgc 9180
   ggccatcgat tcaattcacc catccgatac gttccgcccc ctcacgtccg tctgcgcacg 9240
   acccctgcac gaccacgcca aggccaacgc gccgctcagc tcagcttgtc gacgagtcgc 9300
   acgtcacata tctcagatgc atttggactg tgagtgttat tatgccacta gcacgcaacg 9360
   atcttcgggg tcctcgctca ttgcatccgt tcgggccctg caggcgtgga cgcgagtcgc 9420
   cgccgagacg ctgcagcagg ccgctccgac gcgagggctc gagctcgccg cgcccgcgcg 9480
   atgtctgcct ggcgccgact gatctctgga gcgcaaggaa gacacggcga cgcgaggagg 9540
   accgaagaga gacgctgggg tatgcaggat atacccgggg cgggacattc gttccgcata 9600
   cactccccca ttcgagcttg ctcgtccttg gcagagccga gcgcgaacgg ttccgaacgc 9660
   ggcaaggatt ttggctctgg tgggtggact ccgatcgagg cgcaggttct ccgcaggttc 9720
   tcgcaggccg gcagtggtcg ttagaaatag ggagtgccgg agtcttgacg cgccttagct 9780
   cactctccgc ccacgcgcgc atcgccgcca tgccgccgtc ccgtctgtcg ctgcgctggc 9840
   cgcgaccggc tgcgccagag tacgacagtg ggacagagct cgaggcgacg cgaatcgctc 9900
   gggttgtaag ggtttcaagg gtcgggcgtc gtcgcgtgcc aaagtgaaaa tagtaggggg 9960
   gggggggggt ac 9972
<210> 16
   <211> 6634
   <212> DNA
   <213> Artificial Sequence
<220>
   <223> vector pCL0132
<400> 16
   ctcttatctg cctcgcgccg ttgaccgccg cttgactctt ggcgcttgcc gctcgcatcc        60
   tgcctcgctc gcgcaggcgg gcgggcgagt gggtgggtcc gcagccttcc gcgctcgccc       120
   gctagctcgc tcgcgccgtg ctgcagccag cagggcagca ccgcacggca ggcaggtccc       180
   ggcgcggatc gatcgatcca tcgatccatc gatccatcga tcgtgcggtc aaaaagaaag       240
   gaagaagaaa ggaaaaagaa aggcgtgcgc acccgagtgc gcgctgagcg cccgctcgcg       300
   gtcccgcgga gcctccgcgt tagtccccgc cccgcgccgc gcagtccccc gggaggcatc       360
   gcgcacctct cgccgccccc tcgcgcctcg ccgattcccc gcctcccctt ttccgcttct       420
   tcgccgcctc cgctcgcggc cgcgtcgccc gcgccccgct ccctatctgc tccccagggg       480
   ggcactccgc accttttgcg cccgctgccg ccgccgcggc cgccccgccg ccctggtttc       540
   ccccgcgagc gcggccgcgt cgccgcgcaa agactcgccg cgtgccgccc cgagcaacgg       600
   gtggcggcgg cgcggcggcg ggcggggcgc ggcggcgcgt aggcggggct aggcgccggc       660
   taggcgaaac gccgcccccg ggcgccgccg ccgcccgctc cagagcagtc gccgcgccag       720
   accgccaacg cagagaccga gaccgaggta cgtcgcgccc gagcacgccg cgacgcgcgg       780
   cagggacgag gagcacgacg ccgcgccgcg ccgcgcgggg ggggggaggg agaggcagga     840
   cgcgggagcg agcgtgcatg tttccgcgcg agacgacgcc gcgcgcgctg gagaggagat    900
   aaggcgcttg gatcgcgaga gggccagcca ggctggaggc gaaaatgggt ggagaggata    960
   gtatcttgcg tgcttggacg aggagactga cgaggaggac ggatacgtcg atgatgatgt   1020
   gcacagagaa gaagcagttc gaaagcgact actagcaagc aagggatcca tggctaagga   1080
   gtacttcccc cagatccaga agattaagtt cgagggtaag gacagcaaga acccgctcgc   1140
   ctttcattac tacgacgccg agaaggaggt gatgggcaag aagatgaagg actggcttcg   1200
   ctttgctatg gcttggtggc acactctctg cgctgagggc gcggaccagt ttggcggcgg   1260
   tacgaagagc tttccgtgga acgagggcac tgacgctatt gagattgcta agcagaaggt   1320
   tgacgctggt ttcgagatta tgcagaagct cggtattccg tactactgct ttcacgatgt   1380
   cgacctcgtt tccgagggca actcgatcga ggagtacgag tcgaacctca aggctgtggt   1440
   tgcctacctc aaggagaagc agaaggagac cggaatcaag ctcctctgga gcaccgccaa   1500
   cgttttcggc cacaagcgct acatgaacgg cgcctccacc aaccctgact tcgatgttgt   1560
   tgcccgcgct attgtccaga ttaagaacgc catcgacgct ggtatcgagc tcggagccga   1620
   gaactacgtt ttttggggcg gacgcgaggg ttacatgtcc ctcctcaaca ccgaccagaa   1680
   gcgtgagaag gagcacatgg ccactatgct taccatggcc cgcgactacg cccgcagcaa   1740
   gggttttaag ggtacttttc tcattgagcc gaagcccatg gagccgacca agcaccagta   1800
   cgacgtcgac accgagaccg ccattggctt ccttaaggcc cacaaccttg acaaggattt   1860
   taaggtgaac atcgaggtta accacgctac gcttgccggc cacacctttg agcatgagct   1920
   cgcctgcgct gttgacgccg gaatgcttgg ttccattgac gccaaccgcg gcgactacca   1980
   gaacggctgg gacaccgacc agtttccgat tgaccagtac gagctcgtcc aggcctggat   2040
   ggagatcatc cgtggtggag gctttgttac cggtggtacg aacttcgacg ccaagacgcg   2100
   ccgtaacagc acggacctcg aggacatcat cattgctcat gtgtcgggca tggacgccat   2160
   ggctcgcgcc cttgagaacg ctgctaagct cctccaggag agcccctaca cgaagatgaa   2220
   gaaggagcgc tacgcgtcgt ttgacagcgg aatcggtaag gacttcgagg atggcaagct   2280
   caccctggag caggtgtacg agtacggtaa gaagaacggc gagccgaagc agaccagcgg   2340
   caagcaggag ctctacgagg ccattgtcgc catgtaccag tagcatatga gttatgagat   2400
   ccgaaagtga accttgtcct aacccgacag cgaatggcgg gagggggcgg gctaaaagat   2460
   cgtattacat agtatttttc ccctactctt tgtgtttgtc tttttttttt ttttgaacgc   2520
   attcaagcca cttgtctggg tttacttgtt tgtttgcttg cttgcttgct tgcttgcctg   2580
   cttcttggtc agacggccca aaaaagggaa aaaattcatt catggcacag ataagaaaaa   2640
   gaaaaagttt gtcgaccacc gtcatcagaa agcaagagaa gagaaacact cgcgctcaca   2700
   ttctcgctcg cgtaagaatc ttagccacgc atacgaagta atttgtccat ctggcgaatc   2760
   tttacatgag cgttttcaag ctggagcgtg agatcatacc tttcttgatc gtaatgttcc 2820
   aaccttgcat aggcctcgtt gcgatccgct agcaatgcgt cgtactcccg ttgcaactgc 2880
   gccatcgcct cattgtgacg tgagttcaga ttcttctcga gaccttcgag cgctgctaat 2940
   ttcgcctgac gctccttctt ttgtgcttcc atgacacgcc gcttcaccgt gcgttccact 3000
   tcttcctcag acatgccctt ggctgcctcg acctgctcgg taaaacgggc cccagcacgt 3060
   gctacgagat ttcgattcca ccgccgcctt ctatgaaagg ttgggcttcg gaatcgtttt 3120
   ccgggacgcc ggctggatga tcctccagcg cggggatctc atgctggagt tcttcgccca 3180
   ccccaacttg tttattgcag cttataatgg ttacaaataa agcaatagca tcacaaattt 3240
   cacaaataaa gcattttttt cactgcattc tagttgtggt ttgtccaaac tcatcaatgt 3300
   atcttatcat acatggtcga cctgcaggaa cctgcattaa tgaatcggcc aacgcgcggg 3360
   gagaggcggt ttgcgtattg ggcgctcttc cgcttcctcg ctcactgact cgctgcgctc 3420
   ggtcgttcgg ctgcggcgag cggtatcagc tcactcaaag gcggtaatac ggttatccac 3480
   agaatcaggg gataacgcag gaaagaacat gtgagcaaaa ggccagcaaa aggccaggaa 3540
   ccgtaaaaag gccgcgttgc tggcgttttt ccataggctc cgcccccctg acgagcatca 3600
   caaaaatcga cgctcaagtc agaggtggcg aaacccgaca ggactataaa gataccaggc 3660
   gtttccccct ggaagctccc tcgtgcgctc tcctgttccg accctgccgc ttaccggata 3720
   cctgtccgcc tttctccctt cgggaagcgt ggcgctttct catagctcac gctgtaggta 3780
   tctcagttcg gtgtaggtcg ttcgctccaa gctgggctgt gtgcacgaac cccccgttca 3840
   gcccgaccgc tgcgccttat ccggtaacta tcgtcttgag tccaacccgg taagacacga 3900
   cttatcgcca ctggcagcag ccactggtaa caggattagc agagcgaggt atgtaggcgg 3960
   tgctacagag ttcttgaagt ggtggcctaa ctacggctac actagaagaa cagtatttgg 4020
   tatctgcgct ctgctgaagc cagttacctt cggaaaaaga gttggtagct cttgatccgg 4080
   caaacaaacc accgctggta gcggtggttt ttttgtttgc aagcagcaga ttacgcgcag 4140
   aaaaaaagga tctcaagaag atcctttgat cttttctacg gggtctgacg ctcagtggaa 4200
   cgaaaactca cgttaaggga ttttggtcat gagattatca aaaaggatct tcacctagat 4260
   ccttttaaat taaaaatgaa gttttaaatc aatctaaagt atatatgagt aaacttggtc 4320
   tgacagttac caatgcttaa tcagtgaggc acctatctca gcgatctgtc tatttcgttc 4380
   atccatagtt gcctgactcc ccgtcgtgta gataactacg atacgggagg gcttaccatc 4440
   tggccccagt gctgcaatga taccgcgaga cccacgctca ccggctccag atttatcagc 4500
   aataaaccag ccagccggaa gggccgagcg cagaagtggt cctgcaactt tatccgcctc 4560
   catccagtct attaattgtt gccgggaagc tagagtaagt agttcgccag ttaatagttt 4620
   gcgcaacgtt gttgccattg ctacaggcat cgtggtgtca cgctcgtcgt ttggtatggc 4680
   ttcattcagc tccggttccc aacgatcaag gcgagttaca tgatccccca tgttgtgcaa 4740
   aaaagcggtt agctccttcg gtcctccgat cgttgtcaga agtaagttgg ccgcagtgtt 4800
   atcactcatg gttatggcag cactgcataa ttctcttact gtcatgccat ccgtaagatg 4860
   cttttctgtg actggtgagt actcaaccaa gtcattctga gaatagtgta tgcggcgacc 4920
   gagttgctct tgcccggcgt caatacggga taataccgcg ccacatagca gaactttaaa 4980
   agtgctcatc attggaaaac gttcttcggg gcgaaaactc tcaaggatct taccgctgtt 5040
   gagatccagt tcgatgtaac ccactcgtgc acccaactga tcttcagcat cttttacttt 5100
   caccagcgtt tctgggtgag caaaaacagg aaggcaaaat gccgcaaaaa agggaataag 5160
   ggcgacacgg aaatgttgaa tactcatact cttccttttt caatattatt gaagcattta 5220
   tcagggttat tgtctcatga gcggatacat atttgaatgt atttagaaaa ataaacaaat 5280
   aggggttccg cgcacatttc cccgaaaagt gccacctgac gtctaagaaa ccattattat 5340
   catgacatta acctataaaa ataggcgtat cacgaggccc tttcgtctcg cgcgtttcgg 5400
   tgatgacggt gaaaacctct gacacatgca gctcccggag acggtcacag cttgtctgta 5460
   agcggatgcc gggagcagac aagcccgtca gggcgcgtca gcgggtgttg gcgggtgtcg 5520
   gggctggctt aactatgcgg catcagagca gattgtactg agagtgcacc aagcttgagg 5580
   tctgtcgata atccactttt ccattgattt tccaggtttc gttaactcat gccactgagc 5640
   aaaacttcgg tctttcctaa caaaagctct cctcacaaag catggcgcgg caacggacgt 5700
   gtcctcatac tccactgcca cacaaggtcg ataaactaag ctcctcacaa atagaggaga 5760
   attccactga caactgaaaa caatgtatga gagacgatca ccactggagc ggcgcggcgg 5820
   ttgggcgcgg aggtcggcag caaaaacaag cgactcgccg agcaaacccg aatcagcctt 5880
   cagacggtcg tgcctaacaa cacgccgttc taccccgcct tcttcgcgcc ccttcgcgtc 5940
   caagcatcct tcaagtttat ctctctagtt caacttcaag aagaacaaca ccaccaacac 6000
   catggccaag ttgaccagtg ccgttccggt gctcaccgcg cgcgacgtcg ccggagcggt 6060
   cgagttctgg accgaccggc tcgggttctc ccgggacttc gtggaggacg acttcgccgg 6120
   tgtggtccgg gacgacgtga ccctgttcat cagcgcggtc caggaccagg tggtgccgga 6180
   caacaccctg gcctgggtgt gggtgcgcgg cctggacgag ctgtacgccg agtggtcgga 6240
   ggtcgtgtcc acgaacttcc gggacgcctc cgggccggcc atgaccgaga tcggcgagca 6300
   gccgtggggg cgggagttcg ccctgcgcga cccggccggc aactgcgtgc acttcgtggc 6360
   cgaggagcag gactgacacg tgctacgaga tttcgattcc accgccgcct tctatgaaag 6420
   gttgggcttc ggaatcgttt tccgggacgc cggctggatg atcctccagc gcggggatct 6480
   catgctggag ttcttcgccc accccaactt gtttattgca gcttataatg gttacaaata 6540
   aagcaatagc atcacaaatt tcacaaataa agcatttttt tcactgcatt ctagttgtgg 6600
   tttgtccaaa ctcatcaatg tatcttatcg gtac 6634
<210> 17
   <211> 6277
   <212> DNA
   <213> Artificial Sequence
<220>
   <223> vector pCL0133
<400> 17
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg ggggggaggg agaggcagga      840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tgccctccat    1080
   taagctcaac tccggttacg atatgcccgc cgtcggtttt ggttgctgga aggtggacgt    1140
   cgacacttgc tcggagcaga tttaccgcgc cattaagacc ggataccgcc tctttgacgg    1200
   tgccgaggac tacgccaacg agaagctggt cggagccggc gtcaagaagg ccattgatga    1260
   gggaattgtc aagcgcgagg acctctttct cacctccaag ctctggaaca actaccacca    1320
   ccccgataac gtcgagaagg ctcttaaccg taccctcagc gatctccagg tcgactacgt    1380
   cgatcttttt cttattcact tccctgtcac gttcaagttt gtccctcttg aggagaagta    1440
   cccccccgga ttctactgcg gaaagggcga taactttgac tacgaggacg ttcctattct    1500
   ggagacttgg aaggctctcg agaagctcgt caaggccggc aagattcgca gcatcggcgt    1560
   cagcaacttt cctggagctc tcctcctgga cctccttcgc ggagccacca tcaagccttc    1620
   ggttcttcag gtcgagcacc atccttacct tcagcagccc cgtctcatcg agtttgccca    1680
   gtcccgcggt attgccgtca cggcctacag ctccttcggc cctcagtcct ttgtcgagct 1740
   caaccagggt cgcgccctta acaccagccc cctcttcgag aacgagacca ttaaggccat 1800
   cgctgctaag cacggtaagt cccccgccca ggtcctcctc cgttggagct cgcagcgcgg 1860
   aatcgccatc atccctaaga gcaacaccgt ccctcgcctt cttgagaaca aggatgtcaa 1920
   ctccttcgac ctcgatgagc aggatttcgc cgacattgcc aagctcgata ttaacctccg 1980
   cttcaacgac ccctgggact gggataagat ccctatcttt gtctaacata tgagttatga 2040
   gatccgaaag tgaaccttgt cctaacccga cagcgaatgg cgggaggggg cgggctaaaa 2100
   gatcgtatta catagtattt ttcccctact ctttgtgttt gtcttttttt tttttttgaa 2160
   cgcattcaag ccacttgtct gggtttactt gtttgtttgc ttgcttgctt gcttgcttgc 2220
   ctgcttcttg gtcagacggc ccaaaaaagg gaaaaaattc attcatggca cagataagaa 2280
   aaagaaaaag tttgtcgacc accgtcatca gaaagcaaga gaagagaaac actcgcgctc 2340
   acattctcgc tcgcgtaaga atcttagcca cgcatacgaa gtaatttgtc catctggcga 2400
   atctttacat gagcgttttc aagctggagc gtgagatcat acctttcttg atcgtaatgt 2460
   tccaaccttg cataggcctc gttgcgatcc gctagcaatg cgtcgtactc ccgttgcaac 2520
   tgcgccatcg cctcattgtg acgtgagttc agattcttct cgagaccttc gagcgctgct 2580
   aatttcgcct gacgctcctt cttttgtgct tccatgacac gccgcttcac cgtgcgttcc 2640
   acttcttcct cagacatgcc cttggctgcc tcgacctgct cggtaaaacg ggccccagca 2700
   cgtgctacga gatttcgatt ccaccgccgc cttctatgaa aggttgggct tcggaatcgt 2760
   tttccgggac gccggctgga tgatcctcca gcgcggggat ctcatgctgg agttcttcgc 2820
   ccaccccaac ttgtttattg cagcttataa tggttacaaa taaagcaata gcatcacaaa 2880
   tttcacaaat aaagcatttt tttcactgca ttctagttgt ggtttgtcca aactcatcaa 2940
   tgtatcttat catacatggt cgacctgcag gaacctgcat taatgaatcg gccaacgcgc 3000
   ggggagaggc ggtttgcgta ttgggcgctc ttccgcttcc tcgctcactg actcgctgcg 3060
   ctcggtcgtt cggctgcggc gagcggtatc agctcactca aaggcggtaa tacggttatc 3120
   cacagaatca ggggataacg caggaaagaa catgtgagca aaaggccagc aaaaggccag 3180
   gaaccgtaaa aaggccgcgt tgctggcgtt tttccatagg ctccgccccc ctgacgagca 3240
   tcacaaaaat cgacgctcaa gtcagaggtg gcgaaacccg acaggactat aaagatacca 3300
   ggcgtttccc cctggaagct ccctcgtgcg ctctcctgtt ccgaccctgc cgcttaccgg 3360
   atacctgtcc gcctttctcc cttcgggaag cgtggcgctt tctcatagct cacgctgtag 3420
   gtatctcagt tcggtgtagg tcgttcgctc caagctgggc tgtgtgcacg aaccccccgt 3480
   tcagcccgac cgctgcgcct tatccggtaa ctatcgtctt gagtccaacc cggtaagaca 3540
   cgacttatcg ccactggcag cagccactgg taacaggatt agcagagcga ggtatgtagg 3600
   cggtgctaca gagttcttga agtggtggcc taactacggc tacactagaa gaacagtatt 3660
   tggtatctgc gctctgctga agccagttac cttcggaaaa agagttggta gctcttgatc 3720
   cggcaaacaa accaccgctg gtagcggtgg tttttttgtt tgcaagcagc agattacgcg 3780
   cagaaaaaaa ggatctcaag aagatccttt gatcttttct acggggtctg acgctcagtg 3840
   gaacgaaaac tcacgttaag ggattttggt catgagatta tcaaaaagga tcttcaccta 3900
   gatcctttta aattaaaaat gaagttttaa atcaatctaa agtatatatg agtaaacttg 3960
   gtctgacagt taccaatgct taatcagtga ggcacctatc tcagcgatct gtctatttcg 4020
   ttcatccata gttgcctgac tccccgtcgt gtagataact acgatacggg agggcttacc 4080
   atctggcccc agtgctgcaa tgataccgcg agacccacgc tcaccggctc cagatttatc 4140
   agcaataaac cagccagccg gaagggccga gcgcagaagt ggtcctgcaa ctttatccgc 4200
   ctccatccag tctattaatt gttgccggga agctagagta agtagttcgc cagttaatag 4260
   tttgcgcaac gttgttgcca ttgctacagg catcgtggtg tcacgctcgt cgtttggtat 4320
   ggcttcattc agctccggtt cccaacgatc aaggcgagtt acatgatccc ccatgttgtg 4380
   caaaaaagcg gttagctcct tcggtcctcc gatcgttgtc agaagtaagt tggccgcagt 4440
   gttatcactc atggttatgg cagcactgca taattctctt actgtcatgc catccgtaag 4500
   atgcttttct gtgactggtg agtactcaac caagtcattc tgagaatagt gtatgcggcg 4560
   accgagttgc tcttgcccgg cgtcaatacg ggataatacc gcgccacata gcagaacttt 4620
   aaaagtgctc atcattggaa aacgttcttc ggggcgaaaa ctctcaagga tcttaccgct 4680
   gttgagatcc agttcgatgt aacccactcg tgcacccaac tgatcttcag catcttttac 4740
   tttcaccagc gtttctgggt gagcaaaaac aggaaggcaa aatgccgcaa aaaagggaat 4800
   aagggcgaca cggaaatgtt gaatactcat actcttcctt tttcaatatt attgaagcat 4860
   ttatcagggt tattgtctca tgagcggata catatttgaa tgtatttaga aaaataaaca 4920
   aataggggtt ccgcgcacat ttccccgaaa agtgccacct gacgtctaag aaaccattat 4980
   tatcatgaca ttaacctata aaaataggcg tatcacgagg ccctttcgtc tcgcgcgttt 5040
   cggtgatgac ggtgaaaacc tctgacacat gcagctcccg gagacggtca cagcttgtct 5100
   gtaagcggat gccgggagca gacaagcccg tcagggcgcg tcagcgggtg ttggcgggtg 5160
   tcggggctgg cttaactatg cggcatcaga gcagattgta ctgagagtgc accaagcttg 5220
   aggtctgtcg ataatccact tttccattga ttttccaggt ttcgttaact catgccactg 5280
   agcaaaactt cggtctttcc taacaaaagc tctcctcaca aagcatggcg cggcaacgga 5340
   cgtgtcctca tactccactg ccacacaagg tcgataaact aagctcctca caaatagagg 5400
   agaattccac tgacaactga aaacaatgta tgagagacga tcaccactgg agcggcgcgg 5460
   cggttgggcg cggaggtcgg cagcaaaaac aagcgactcg ccgagcaaac ccgaatcagc 5520
   cttcagacgg tcgtgcctaa caacacgccg ttctaccccg ccttcttcgc gccccttcgc 5580
   gtccaagcat ccttcaagtt tatctctcta gttcaacttc aagaagaaca acaccaccaa 5640
   caccatggcc aagttgacca gtgccgttcc ggtgctcacc gcgcgcgacg tcgccggagc 5700
   ggtcgagttc tggaccgacc ggctcgggtt ctcccgggac ttcgtggagg acgacttcgc 5760
   cggtgtggtc cgggacgacg tgaccctgtt catcagcgcg gtccaggacc aggtggtgcc 5820
   ggacaacacc ctggcctggg tgtgggtgcg cggcctggac gagctgtacg ccgagtggtc 5880
   ggaggtcgtg tccacgaact tccgggacgc ctccgggccg gccatgaccg agatcggcga 5940
   gcagccgtgg gggcgggagt tcgccctgcg cgacccggcc ggcaactgcg tgcacttcgt 6000
   ggccgaggag caggactgac acgtgctacg agatttcgat tccaccgccg ccttctatga 6060
   aaggttgggc ttcggaatcg ttttccggga cgccggctgg atgatcctcc agcgcgggga 6120
   tctcatgctg gagttcttcg cccaccccaa cttgtttatt gcagcttata atggttacaa 6180
   ataaagcaat agcatcacaa atttcacaaa taaagcattt ttttcactgc attctagttg 6240
   tggtttgtcc aaactcatca atgtatctta tcggtac 6277
<210> 18
   <211> 6456
   <212> DNA
   <213> Artificial Sequence
<220>
   <223> vector pCL0134
<400> 18
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg ggggggaggg agaggcagga    840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaccgccaa 1080
   cccgagcctc gtccttaaca agatcgacga tatttccttc gagacctacg acgcccccga 1140
   gatcagcgag cccaccgatg tcctcgtcca ggttaagaag accggcatct gcggttccga 1200
   tattcacttt tacgctcacg gacgcattgg aaactttgtc ctcactaagc ctatggttct 1260
   gggtcacgag tccgccggta ctgtcgttca ggtgggaaag ggtgttacgt cgcttaaggt 1320
   cggagacaac gttgccatcg agcccggcat ccccagccgc ttttccgatg agtacaagtc 1380
   cggtcactac aacctctgcc cccacatggc tttcgccgcc acccccaact ccaaggaggg 1440
   cgagcctaac ccccccggca ccctctgcaa gtactttaag tcccccgagg attttctcgt 1500
   caagctcccc gaccacgtct cgcttgagct gggcgccctc gtcgagcccc tgtccgtcgg 1560
   agttcacgcc agcaagctcg gtagcgttgc ctttggcgac tacgtggccg tttttggcgc 1620
   gggtcctgtc ggccttctcg ccgccgctgt ggccaagacc tttggagcca agggcgttat 1680
   cgtcgtcgac atttttgaca acaagctcaa gatggctaag gatattggcg ccgctactca 1740
   tacctttaac tccaagaccg gcggttccga ggagcttatc aaggcctttg gcggtaacgt 1800
   cccgaacgtt gtcctcgagt gcaccggagc cgagccctgc attaagctcg gagtggatgc 1860
   catcgcccct ggtggacgct ttgtccaggt tggtaacgcc gccggtcccg tcagcttccc 1920
   gatcaccgtt ttcgctatga aggagctcac cctcttcggc agcttccgtt acggctttaa 1980
   cgactacaag accgccgtgg gcatctttga caccaactac cagaacggac gtgagaacgc 2040
   ccctatcgat tttgagcagc tgattaccca ccgttacaag tttaaggacg ccattgaggc 2100
   ctacgacctc gtccgcgctg gcaagggagc cgtcaagtgc ctcatcgatg gtcccgagtg 2160
   acatatgagt tatgagatcc gaaagtgaac cttgtcctaa cccgacagcg aatggcggga 2220
   gggggcgggc taaaagatcg tattacatag tatttttccc ctactctttg tgtttgtctt 2280
   tttttttttt ttgaacgcat tcaagccact tgtctgggtt tacttgtttg tttgcttgct 2340
   tgcttgcttg cttgcctgct tcttggtcag acggcccaaa aaagggaaaa aattcattca 2400
   tggcacagat aagaaaaaga aaaagtttgt cgaccaccgt catcagaaag caagagaaga 2460
   gaaacactcg cgctcacatt ctcgctcgcg taagaatctt agccacgcat acgaagtaat 2520
   ttgtccatct ggcgaatctt tacatgagcg ttttcaagct ggagcgtgag atcatacctt 2580
   tcttgatcgt aatgttccaa ccttgcatag gcctcgttgc gatccactag caatgcgtcg 2640
   tactcccgtt gcaactgcgc catcgcctca ttgtgacgtg agttcagatt cttctcgaga 2700
   ccttcgagcg ctgctaattt cgcctgacgc tccttctttt gtgcttccat gacacgccgc 2760
   ttcaccgtgc gttccacttc ttcctcagac atgcccttgg ctgcctcgac ctgctcggta 2820
   aaacgggccc cagcacgtgc tacgagattt cgattccacc gccgccttct atgaaaggtt 2880
   gggcttcgga atcgttttcc gggacgccgg ctggatgatc ctccagcgcg gggatctcat 2940
   gctggagttc ttcgcccacc ccaacttgtt tattgcagct tataatggtt acaaataaag 3000
   caatagcatc acaaatttca caaataaagc atttttttca ctgcattcta gttgtggttt 3060
   gtccaaactc atcaatgtat cttatcatac atggtcgacc tgcaggaacc tgcattaatg 3120
   aatcggccaa cgcgcgggga gaggcggttt gcgtattggg cgctcttccg cttcctcgct 3180
   cactgactcg ctgcgctcgg tcgttcggct gcggcgagcg gtatcagctc actcaaaggc 3240
   ggtaatacgg ttatccacag aatcagggga taacgcagga aagaacatgt gagcaaaagg 3300
   ccagcaaaag gccaggaacc gtaaaaaggc cgcgttgctg gcgtttttcc ataggctccg 3360
   cccccctgac gagcatcaca aaaatcgacg ctcaagtcag aggtggcgaa acccgacagg 3420
   actataaaga taccaggcgt ttccccctgg aagctccctc gtgcgctctc ctgttccgac 3480
   cctgccgctt accggatacc tgtccgcctt tctcccttcg ggaagcgtgg cgctttctca 3540
   tagctcacgc tgtaggtatc tcagttcggt gtaggtcgtt cgctccaagc tgggctgtgt 3600
   gcacgaaccc cccgttcagc ccgaccgctg cgccttatcc ggtaactatc gtcttgagtc 3660
   caacccggta agacacgact tatcgccact ggcagcagcc actggtaaca ggattagcag 3720
   agcgaggtat gtaggcggtg ctacagagtt cttgaagtgg tggcctaact acggctacac 3780
   tagaagaaca gtatttggta tctgcgctct gctgaagcca gttaccttcg gaaaaagagt 3840
   tggtagctct tgatccggca aacaaaccac cgctggtagc ggtggttttt ttgtttgcaa 3900
   gcagcagatt acgcgcagaa aaaaaggatc tcaagaagat cctttgatct tttctacggg 3960
   gtctgacgct cagtggaacg aaaactcacg ttaagggatt ttggtcatga gattatcaaa 4020
   aaggatcttc acctagatcc ttttaaatta aaaatgaagt tttaaatcaa tctaaagtat 4080
   atatgagtaa acttggtctg acagttacca atgcttaatc agtgaggcac ctatctcagc 4140
   gatctgtcta tttcgttcat ccatagttgc ctgactcccc gtcgtgtaga taactacgat 4200
   acgggagggc ttaccatctg gccccagtgc tgcaatgata ccgcgagacc cacgctcacc 4260
   ggctccagat ttatcagcaa taaaccagcc agccggaagg gccgagcgca gaagtggtcc 4320
   tgcaacttta tccgcctcca tccagtctat taattgttgc cgggaagcta gagtaagtag 4380
   ttcgccagtt aatagtttgc gcaacgttgt tgccattgct acaggcatcg tggtgtcacg 4440
   ctcgtcgttt ggtatggctt cattcagctc cggttcccaa cgatcaaggc gagttacatg 4500
   atcccccatg ttgtgcaaaa aagcggttag ctccttcggt cctccgatcg ttgtcagaag 4560
   taagttggcc gcagtgttat cactcatggt tatggcagca ctgcataatt ctcttactgt 4620
   catgccatcc gtaagatgct tttctgtgac tggtgagtac tcaaccaagt cattctgaga 4680
   atagtgtatg cggcgaccga gttgctcttg cccggcgtca atacgggata ataccgcgcc 4740
   acatagcaga actttaaaag tgctcatcat tggaaaacgt tcttcggggc gaaaactctc 4800
   aaggatctta ccgctgttga gatccagttc gatgtaaccc actcgtgcac ccaactgatc 4860
   ttcagcatct tttactttca ccagcgtttc tgggtgagca aaaacaggaa ggcaaaatgc 4920
   cgcaaaaaag ggaataaggg cgacacggaa atgttgaata ctcatactct tcctttttca 4980
   atattattga agcatttatc agggttattg tctcatgagc ggatacatat ttgaatgtat 5040
   ttagaaaaat aaacaaatag gggttccgcg cacatttccc cgaaaagtgc cacctgacgt 5100
   ctaagaaacc attattatca tgacattaac ctataaaaat aggcgtatca cgaggccctt 5160
   tcgtctcgcg cgtttcggtg atgacggtga aaacctctga cacatgcagc tcccggagac 5220
   ggtcacagct tgtctgtaag cggatgccgg gagcagacaa gcccgtcagg gcgcgtcagc 5280
   gggtgttggc gggtgtcggg gctggcttaa ctatgcggca tcagagcaga ttgtactgag 5340
   agtgcaccaa gcttgaggtc tgtcgataat ccacttttcc attgattttc caggtttcgt 5400
   taactcatgc cactgagcaa aacttcggtc tttcctaaca aaagctctcc tcacaaagca 5460
   tggcgcggca acggacgtgt cctcatactc cactgccaca caaggtcgat aaactaagct 5520
   cctcacaaat agaggagaat tccactgaca actgaaaaca atgtatgaga gacgatcacc 5580
   actggagcgg cgcggcggtt gggcgcggag gtcggcagca aaaacaagcg actcgccgag 5640
   caaacccgaa tcagccttca gacggtcgtg cctaacaaca cgccgttcta ccccgccttc 5700
   ttcgcgcccc ttcgcgtcca agcatccttc aagtttatct ctctagttca acttcaagaa 5760
   gaacaacacc accaacacca tgatgccttt gtctcaagaa gaatccaccc tcattgaaag 5820
   agcaacggct acaatcaaca gcatccccat ctctgaagac tacagcgtcg ccagcgcagc 5880
   tctctctagc gacggccgca tcttcactgg tgtcaatgta tatcatttta ctgggggacc 5940
   ttgtgcagaa ctcgtggtgc tgggcactgc tgctgctgcg gcagctggca acctgacttg 6000
   tatcgtcgcg atcggaaatg agaacagggg catcttgagc ccctgtggac ggtgccgaca 6060
   ggtgcttctc gatctgcatc ctgggatcaa agccatagtg aaggacagtg atggacagcc 6120
   gacggcagtt gggattcgtg aattgctgcc ctctggttat gtgtgggagg gctaacacgt 6180
   gctccgtgct acgagatttc gattccaccg ccgccttcta tgaaaggttg ggcttcggaa 6240
   tcgttttccg ggacgccggc tggatgatcc tccagcgcgg ggatctcatg ctggagttct 6300
   tcgcccaccc caacttgttt attgcagctt ataatggtta caaataaagc aatagcatca 6360
   caaatttcac aaataaagca tttttttcac tgcattctag ttgtggtttg tccaaactca 6420
   tcaatgtatc ttatcatgtc tgaattcccg gggtac 6456
<210> 19
   <211> 7628
   <212> DNA
   <213> Artificial Sequence
<220>
   <223> vector pCL0135
<400> 19
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg gggggagggg agaggcagga     840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tgactactac    1080
   gccgtttgac gctcccgaca agctctttct tggcttcgat ctctccaccc agcagcttaa    1140
   gattatcgtc actgacgaga acctcgccgc tctcaagacc tacaacgtcg agtttgatag    1200
   cattaactcc agcgtccaga agggtgtgat cgccattaac gatgagatca gcaagggagc    1260
   catcatcagc ccggtctaca tgtggctcga cgctctcgat cacgtcttcg aggatatgaa    1320
   gaaggacggt ttccccttta acaaggtggt cggaatctcc ggctcgtgcc agcagcacgg    1380
   ttcggtctac tggtcgcgca ctgctgagaa ggttctctcc gagcttgacg ccgagtcctc    1440
   cctctcgtcc cagatgcgct ccgcctttac tttcaagcac gcccccaact ggcaggacca    1500
   ctcgaccggc aaggagctcg aggagtttga gcgcgtcatc ggcgccgacg ccctcgctga    1560
   catctccggt agccgcgccc actaccgctt tactggcctt cagattcgca agctctcgac    1620
   ccgttttaag cccgagaagt acaaccgcac ggcccgcatt tccctggtct ccagcttcgt    1680
   cgcttccgtc cttctgggtc gcattacgtc catcgaggag gctgacgctt gcggcatgaa    1740
   cctctacgac atcgagaagc gcgagttcaa cgaggagctt ctcgccattg cggctggtgt    1800
   ccaccccgag ctggacggtg tcgagcagga cggtgagatc taccgcgccg gtattaacga    1860
   gctcaagcgt aagctcggcc ctgtcaagcc catcacctac gagtccgagg gagacatcgc    1920
   ctcctacttc gtcacccgct acggttttaa ccctgactgc aagatctact cgtttactgg 1980
   agacaacctc gccaccatca tctcccttcc tcttgccccg aacgacgccc tcatcagcct 2040
   tggcacctcc accaccgtgc ttatcatcac caagaactac gccccgtcgt cccagtacca 2100
   cctctttaag cacccgacga tgcccgacca ctacatggga atgatttgct actgcaacgg 2160
   ctccctcgcc cgtgagaagg ttcgcgacga ggttaacgag aagtttaacg tcgaggacaa 2220
   gaagtcgtgg gacaagttta acgagatcct cgacaagagc accgatttta acaacaagct 2280
   cggcatctac ttcccgctcg gagagattgt ccctaacgct gcggcccaga ttaagcgctc 2340
   ggtccttaac tcgaagaacg agatcgtcga cgtcgagctc ggagataaga actggcagcc 2400
   tgaggacgat gtgagcagca ttgttgagtc ccagaccctt tcgtgccgcc tccgcacggg 2460
   cccgatgctc tccaagtccg gtgattcctc cgcttcgtcg tccgcctcgc cccagcccga 2520
   gggagatggc acggacctcc acaaggttta ccaggacctc gttaagaagt tcggcgacct 2580
   cttcaccgat ggtaagaagc agacttttga gtccctcacc gcccgcccca accgctgcta 2640
   ctacgtcggt ggcgccagca acaacggctc gatcatcctc aagatgggca gcattctcgc 2700
   ccctgtgaac ggtaactaca aggtcgatat cccgaacgcg tgcgcccttg gcggagctta 2760
   caaggcgtcg tggagctacg agtgcgaggc caagaaggag tggattggct acgatcagta 2820
   cattaaccgc cttttcgagg tgtccgatga gatgaacagc tttgaggtca aggacaagtg 2880
   gctcgagtac gctaacggcg tgggcatgct cgccaagatg gagtccgagc tcaagcactg 2940
   acatatgagt tatgagatcc gaaagtgaac cttgtcctaa cccgacagcg aatggcggga 3000
   gggggcgggc taaaagatcg tattacatag tatttttccc ctactctttg tgtttgtctt 3060
   tttttttttt ttgaacgcat tcaagccact tgtctgggtt tacttgtttg tttgcttgct 3120
   tgcttgcttg cttgcctgct tcttggtcag acggcccaaa aaagggaaaa aattcattca 3180
   tggcacagat aagaaaaaga aaaagtttgt cgaccaccgt catcagaaag caagagaaga 3240
   gaaacactcg cgctcacatt ctcgctcgcg taagaatctt agccacgcat acgaagtaat 3300
   ttgtccatct ggcgaatctt tacatgagcg ttttcaagct ggagcgtgag atcatacctt 3360
   tcttgatcgt aatgttccaa ccttgcatag gcctcgttgc gatccgctag caatgcgtcg 3420
   tactcccgtt gcaactgcgc catcgcctca ttgtgacgtg agttcagatt cttctcgaga 3480
   ccttcgagcg ctgctaattt cgcctgacgc tccttctttt gtgcttccat gacacgccgc 3540
   ttcaccgtgc gttccacttc ttcctcagac atgcccttgg ctgcctcgac ctgctcggta 3600
   aaacgggccc cagcacgtgc tacgagattt cgattccacc gccgccttct atgaaaggtt 3660
   gggcttcgga atcgttttcc gggacgccgg ctggatgatc ctccagcgcg gggatctcat 3720
   gctggagttc ttcgcccacc ccaacttgtt tattgcagct tataatggtt acaaataaag 3780
   caatagcatc acaaatttca caaataaagc atttttttca ctgcattcta gttgtggttt 3840
   gtccaaactc atcaatgtat cttatcatac atggtcgacc tgcaggaacc tgcattaatg 3900
   aatcggccaa cgcgcgggga gaggcggttt gcgtattggg cgctcttccg cttcctcgct 3960
   cactgactcg ctgcgctcgg tcgttcggct gcggcgagcg gtatcagctc actcaaaggc 4020
   ggtaatacgg ttatccacag aatcagggga taacgcagga aagaacatgt gagcaaaagg 4080
   ccagcaaaag gccaggaacc gtaaaaaggc cgcgttgctg gcgtttttcc ataggctccg 4140
   cccccctgac gagcatcaca aaaatcgacg ctcaagtcag aggtggcgaa acccgacagg 4200
   actataaaga taccaggcgt ttccccctgg aagctccctc gtgcgctctc ctgttccgac 4260
   cctgccgctt accggatacc tgtccgcctt tctcccttcg ggaagcgtgg cgctttctca 4320
   tagctcacgc tgtaggtatc tcagttcggt gtaggtcgtt cgctccaagc tgggctgtgt 4380
   gcacgaaccc cccgttcagc ccgaccgctg cgccttatcc ggtaactatc gtcttgagtc 4440
   caacccggta agacacgact tatcgccact ggcagcagcc actggtaaca ggattagcag 4500
   agcgaggtat gtaggcggtg ctacagagtt cttgaagtgg tggcctaact acggctacac 4560
   tagaagaaca gtatttggta tctgcgctct gctgaagcca gttaccttcg gaaaaagagt 4620
   tggtagctct tgatccggca aacaaaccac cgctggtagc ggtggttttt ttgtttgcaa 4680
   gcagcagatt acgcgcagaa aaaaaggatc tcaagaagat cctttgatct tttctacggg 4740
   gtctgacgct cagtggaacg aaaactcacg ttaagggatt ttggtcatga gattatcaaa 4800
   aaggatcttc acctagatcc ttttaaatta aaaatgaagt tttaaatcaa tctaaagtat 4860
   atatgagtaa acttggtctg acagttacca atgcttaatc agtgaggcac ctatctcagc 4920
   gatctgtcta tttcgttcat ccatagttgc ctgactcccc gtcgtgtaga taactacgat 4980
   acgggagggc ttaccatctg gccccagtgc tgcaatgata ccgcgagacc cacgctcacc 5040
   ggctccagat ttatcagcaa taaaccagcc agccggaagg gccgagcgca gaagtggtcc 5100
   tgcaacttta tccgcctcca tccagtctat taattgttgc cgggaagcta gagtaagtag 5160
   ttcgccagtt aatagtttgc gcaacgttgt tgccattgct acaggcatcg tggtgtcacg 5220
   ctcgtcgttt ggtatggctt cattcagctc cggttcccaa cgatcaaggc gagttacatg 5280
   atcccccatg ttgtgcaaaa aagcggttag ctccttcggt cctccgatcg ttgtcagaag 5340
   taagttggcc gcagtgttat cactcatggt tatggcagca ctgcataatt ctcttactgt 5400
   catgccatcc gtaagatgct tttctgtgac tggtgagtac tcaaccaagt cattctgaga 5460
   atagtgtatg cggcgaccga gttgctcttg cccggcgtca atacgggata ataccgcgcc 5520
   acatagcaga actttaaaag tgctcatcat tggaaaacgt tcttcggggc gaaaactctc 5580
   aaggatctta ccgctgttga gatccagttc gatgtaaccc actcgtgcac ccaactgatc 5640
   ttcagcatct tttactttca ccagcgtttc tgggtgagca aaaacaggaa ggcaaaatgc 5700
   cgcaaaaaag ggaataaggg cgacacggaa atgttgaata ctcatactct tcctttttca 5760
   atattattga agcatttatc agggttattg tctcatgagc ggatacatat ttgaatgtat 5820
   ttagaaaaat aaacaaatag gggttccgcg cacatttccc cgaaaagtgc cacctgacgt 5880
   ctaagaaacc attattatca tgacattaac ctataaaaat aggcgtatca cgaggccctt 5940
   tcgtctcgcg cgtttcggtg atgacggtga aaacctctga cacatgcagc tcccggagac 6000
   ggtcacagct tgtctgtaag cggatgccgg gagcagacaa gcccgtcagg gcgcgtcagc 6060
   gggtgttggc gggtgtcggg gctggcttaa ctatgcggca tcagagcaga ttgtactgag 6120
   agtgcaccaa gcttgaggtc tgtcgataat ccacttttcc attgattttc caggtttcgt 6180
   taactcatgc cactgagcaa aacttcggtc tttcctaaca aaagctctcc tcacaaagca 6240
   tggcgcggca acggacgtgt cctcatactc cactgccaca caaggtcgat aaactaagct 6300
   cctcacaaat agaggagaat tccactgaca actgaaaaca atgtatgaga gacgatcacc 6360
   actggagcgg cgcggcggtt gggcgcggag gtcggcagca aaaacaagcg actcgccgag 6420
   caaacccgaa tcagccttca gacggtcgtg cctaacaaca cgccgttcta ccccgccttc 6480
   ttcgcgcccc ttcgcgtcca agcatccttc aagtttatct ctctagttca acttcaagaa 6540
   gaacaacacc accaacacca tgattgaaca agatggattg cacgcaggtt ctccggccgc 6600
   ttgggtggag aggctattcg gctatgactg ggcacaacag acaatcggct gctctgatgc 6660
   cgccgtgttc cggctgtcag cgcaggggcg cccggttctt tttgtcaaga ccgacctgtc 6720
   cggtgccctg aatgaactgc aggacgaggc agcgcggcta tcgtggctgg ccacgacggg 6780
   cgttccttgc gcagctgtgc tcgacgttgt cactgaagcg ggaagggact ggctgctatt 6840
   gggcgaagtg ccggggcagg atctcctgtc atctcacctt gctcctgccg agaaagtatc 6900
   catcatggct gatgcaatgc ggcggctgca tacgcttgat ccggctacct gcccattcga 6960
   ccaccaagcg aaacatcgca tcgagcgagc acgtactcgg atggaagccg gtcttgtcga 7020
   tcaggatgat ctggacgaag agcatcaggg gctcgcgcca gccgaactgt tcgccaggct 7080
   caaggcgcgc atgcccgacg gcgatgatct cgtcgtgacc catggcgatg cctgcttgcc 7140
   gaatatcatg gtggaaaatg gccgcttttc tggattcatc gactgtggcc ggctgggtgt 7200
   ggcggaccgc tatcaggaca tagcgttggc tacccgtgat attgctgaag agcttggcgg 7260
   cgaatgggct gaccgcttcc tcgtgcttta cggtatcgcc gctcccgatt cgcagcgcat 7320
   cgccttctat cgccttcttg acgagttctt ctgacacgtg ctacgagatt tcgattccac 7380
   cgccgccttc tatgaaaggt tgggcttcgg aatcgttttc cgggacgccg gctggatgat 7440
   cctccagcgc ggggatctca tgctggagtt cttcgcccac cccaacttgt ttattgcagc 7500
   ttataatggt tacaaataaa gcaatagcat cacaaatttc acaaataaag catttttttc 7560
   actgcattct agttgtggtt tgtccaaact catcaatgta tcttatcatg tctgaattcc 7620
   cggggtac 7628
<210> 20
   <211> 7241
   <212> DNA
   <213> Artificial Sequence
<220>
   <223> vector pCL0136
<400> 20
   ctcttatctg cctcgcgccg ttgaccgccg cttgactctt ggcgcttgcc gctcgcatcc        60
   tgcctcgctc gcgcaggcgg gcgggcgagt gggtgggtcc gcagccttcc gcgctcgccc       120
   gctagctcgc tcgcgccgtg ctgcagccag cagggcagca ccgcacggca ggcaggtccc       180
   ggcgcggatc gatcgatcca tcgatccatc gatccatcga tcgtgcggtc aaaaagaaag       240
   gaagaagaaa ggaaaaagaa aggcgtgcgc acccgagtgc gcgctgagcg cccgctcgcg       300
   gtcccgcgga gcctccgcgt tagtccccgc cccgcgccgc gcagtccccc gggaggcatc       360
   gcgcacctct cgccgccccc tcgcgcctcg ccgattcccc gcctcccctt ttccgcttct       420
   tcgccgcctc cgctcgcggc cgcgtcgccc gcgccccgct ccctatctgc tccccagggg       480
   ggcactccgc accttttgcg cccgctgccg ccgccgcggc cgccccgccg ccctggtttc       540
   ccccgcgagc gcggccgcgt cgccgcgcaa agactcgccg cgtgccgccc cgagcaacgg       600
   gtggcggcgg cgcggcggcg ggcggggcgc ggcggcgcgt aggcggggct aggcgccggc       660
   taggcgaaac gccgcccccg ggcgccgccg ccgcccgctc cagagcagtc gccgcgccag       720
   accgccaacg cagagaccga gaccgaggta cgtcgcgccc gagcacgccg cgacgcgcgg       780
   cagggacgag gagcacgacg ccgcgccgcg ccgcgcgggg gggggggagg agaggcagga     840
   cgcgggagcg agcgtgcatg tttccgcgcg agacgacgcc gcgcgcgctg gagaggagat       900
   aaggcgcttg gatcgcgaga gggccagcca ggctggaggc gaaaatgggt ggagaggata       960
   gtatcttgcg tgcttggacg aggagactga cgaggaggac ggatacgtcg atgatgatgt      1020
   gcacagagaa gaagcagttc gaaagcgact actagcaagc aagggatcca tgaagaccgt      1080
   cgccggcatc gatcttggaa cccagtccat gaaggttgtc atttacgact acgagaagaa      1140
   ggagatcatc gagtccgcct cgtgccctat ggagctcatt agcgagtcgg acggaacccg      1200
   cgagcagacg actgagtggt ttgacaaggg tctcgaggtg tgctttggaa agctctccgc      1260
   tgataacaag aagaccattg aggcgattgg catctccggc cagctccacg gcttcgtccc      1320
   tctcgatgcg aacggaaagg cgctctacaa catcaagctc tggtgcgaca ccgccactgt      1380
   ggaggagtgc aagatcatta ctgacgccgc cggcggcgac aaggctgtca tcgacgcgct      1440
   cggcaacctc atgctcaccg gattcaccgc cccgaagatt ctctggctca agcgcaacaa      1500
   gcccgaggcc tttgctaacc tcaagtacat tatgctgccc cacgattacc tcaactggaa      1560
   gctgactgga gactacgtca tggagtacgg cgacgcctcc ggcaccgccc tttttgattc      1620
   gaagaaccgc tgctggtcga agaagatttg cgacattatt gatcctaagc tgctcgacct      1680
   tctccctaag ctcattgagc cctcggcccc cgccggtaag gtcaacgacg aggccgccaa      1740
   ggcgtacggc attcccgccg gaatccccgt ttccgctggc ggcggtgata acatgatggg 1800
   tgcggtcggt actggcaccg tcgctgacgg attcctcacg atgagcatgg gcacctccgg 1860
   aactctttac ggctactcgg acaagcctat ttccgacccg gctaacggcc tcagcggctt 1920
   ctgcagctcc acgggcggct ggcttcccct cctttgcacc atgaactgca ccgtcgccac 1980
   cgagttcgtc cgcaaccttt ttcagatgga tatcaaggag ctgaacgtcg aggctgctaa 2040
   gtccccctgc ggcagcgagg gcgttcttgt cattcctttc ttcaacggcg agcgcacccc 2100
   gaacctcccc aacggccgcg cctcgattac cggcctcacc tccgcgaaca cgtcccgcgc 2160
   caacatcgct cgcgcctcct ttgagtcggc cgtctttgcc atgcgcggtg gcctcgatgc 2220
   gtttcgtaag ctcggattcc agcccaagga gattcgcctc atcggcggtg gttcgaagtc 2280
   cgacctctgg cgccagatcg ctgctgacat tatgaacctt cccatccgtg tcccccttct 2340
   cgaggaggcc gccgccctcg gcggagctgt ccaggccctt tggtgcctta agaaccagtc 2400
   cggtaagtgc gacatcgtcg agctttgcaa ggagcatatc aagattgacg agtccaagaa 2460
   cgccaacccg attgccgaga acgtcgccgt gtacgataag gcctacgatg agtactgcaa 2520
   ggtcgttaac acgctcagcc ctctgtacgc ctaacatatg agttatgaga tccgaaagtg 2580
   aaccttgtcc taacccgaca gcgaatggcg ggagggggcg ggctaaaaga tcgtattaca 2640
   tagtattttt cccctactct ttgtgtttgt cttttttttt tttttgaacg cattcaagcc 2700
   acttgtctgg gtttacttgt ttgtttgctt gcttgcttgc ttgcttgcct gcttcttggt 2760
   cagacggccc aaaaaaggga aaaaattcat tcatggcaca gataagaaaa agaaaaagtt 2820
   tgtcgaccac cgtcatcaga aagcaagaga agagaaacac tcgcgctcac attctcgctc 2880
   gcgtaagaat cttagccacg catacgaagt aatttgtcca tctggcgaat ctttacatga 2940
   gcgttttcaa gctggagcgt gagatcatac ctttcttgat cgtaatgttc caaccttgca 3000
   taggcctcgt tgcgatccgc tagcaatgcg tcgtactccc gttgcaactg cgccatcgcc 3060
   tcattgtgac gtgagttcag attcttctcg agaccttcga gcgctgctaa tttcgcctga 3120
   cgctccttct tttgtgcttc catgacacgc cgcttcaccg tgcgttccac ttcttcctca 3180
   gacatgccct tggctgcctc gacctgctcg gtaaaacggg ccccagcacg tgctacgaga 3240
   tttcgattcc accgccgcct tctatgaaag gttgggcttc ggaatcgttt tccgggacgc 3300
   cggctggatg atcctccagc gcggggatct catgctggag ttcttcgccc accccaactt 3360
   gtttattgca gcttataatg gttacaaata aagcaatagc atcacaaatt tcacaaataa 3420
   agcatttttt tcactgcatt ctagttgtgg tttgtccaaa ctcatcaatg tatcttatca 3480
   tacatggtcg acctgcagga acctgcatta atgaatcggc caacgcgcgg ggagaggcgg 3540
   tttgcgtatt gggcgctctt ccgcttcctc gctcactgac tcgctgcgct cggtcgttcg 3600
   gctgcggcga gcggtatcag ctcactcaaa ggcggtaata cggttatcca cagaatcagg 3660
   ggataacgca ggaaagaaca tgtgagcaaa aggccagcaa aaggccagga accgtaaaaa 3720
   ggccgcgttg ctggcgtttt tccataggct ccgcccccct gacgagcatc acaaaaatcg 3780
   acgctcaagt cagaggtggc gaaacccgac aggactataa agataccagg cgtttccccc 3840
   tggaagctcc ctcgtgcgct ctcctgttcc gaccctgccg cttaccggat acctgtccgc 3900
   ctttctccct tcgggaagcg tggcgctttc tcatagctca cgctgtaggt atctcagttc 3960
   ggtgtaggtc gttcgctcca agctgggctg tgtgcacgaa ccccccgttc agcccgaccg 4020
   ctgcgcctta tccggtaact atcgtcttga gtccaacccg gtaagacacg acttatcgcc 4080
   actggcagca gccactggta acaggattag cagagcgagg tatgtaggcg gtgctacaga 4140
   gttcttgaag tggtggccta actacggcta cactagaaga acagtatttg gtatctgcgc 4200
   tctgctgaag ccagttacct tcggaaaaag agttggtagc tcttgatccg gcaaacaaac 4260
   caccgctggt agcggtggtt tttttgtttg caagcagcag attacgcgca gaaaaaaagg 4320
   atctcaagaa gatcctttga tcttttctac ggggtctgac gctcagtgga acgaaaactc 4380
   acgttaaggg attttggtca tgagattatc aaaaaggatc ttcacctaga tccttttaaa 4440
   ttaaaaatga agttttaaat caatctaaag tatatatgag taaacttggt ctgacagtta 4500
   ccaatgctta atcagtgagg cacctatctc agcgatctgt ctatttcgtt catccatagt 4560
   tgcctgactc cccgtcgtgt agataactac gatacgggag ggcttaccat ctggccccag 4620
   tgctgcaatg ataccgcgag acccacgctc accggctcca gatttatcag caataaacca 4680
   gccagccgga agggccgagc gcagaagtgg tcctgcaact ttatccgcct ccatccagtc 4740
   tattaattgt tgccgggaag ctagagtaag tagttcgcca gttaatagtt tgcgcaacgt 4800
   tgttgccatt gctacaggca tcgtggtgtc acgctcgtcg tttggtatgg cttcattcag 4860
   ctccggttcc caacgatcaa ggcgagttac atgatccccc atgttgtgca aaaaagcggt 4920
   tagctccttc ggtcctccga tcgttgtcag aagtaagttg gccgcagtgt tatcactcat 4980
   ggttatggca gcactgcata attctcttac tgtcatgcca tccgtaagat gcttttctgt 5040
   gactggtgag tactcaacca agtcattctg agaatagtgt atgcggcgac cgagttgctc 5100
   ttgcccggcg tcaatacggg ataataccgc gccacatagc agaactttaa aagtgctcat 5160
   cattggaaaa cgttcttcgg ggcgaaaact ctcaaggatc ttaccgctgt tgagatccag 5220
   ttcgatgtaa cccactcgtg cacccaactg atcttcagca tcttttactt tcaccagcgt 5280
   ttctgggtga gcaaaaacag gaaggcaaaa tgccgcaaaa aagggaataa gggcgacacg 5340
   gaaatgttga atactcatac tcttcctttt tcaatattat tgaagcattt atcagggtta 5400
   ttgtctcatg agcggataca tatttgaatg tatttagaaa aataaacaaa taggggttcc 5460
   gcgcacattt ccccgaaaag tgccacctga cgtctaagaa accattatta tcatgacatt 5520
   aacctataaa aataggcgta tcacgaggcc ctttcgtctc gcgcgtttcg gtgatgacgg 5580
   tgaaaacctc tgacacatgc agctcccgga gacggtcaca gcttgtctgt aagcggatgc 5640
   cgggagcaga caagcccgtc agggcgcgtc agcgggtgtt ggcgggtgtc ggggctggct 5700
   taactatgcg gcatcagagc agattgtact gagagtgcac caagcttgag gtctgtcgat 5760
   aatccacttt tccattgatt ttccaggttt cgttaactca tgccactgag caaaacttcg 5820
   gtctttccta acaaaagctc tcctcacaaa gcatggcgcg gcaacggacg tgtcctcata 5880
   ctccactgcc acacaaggtc gataaactaa gctcctcaca aatagaggag aattccactg 5940
   acaactgaaa acaatgtatg agagacgatc accactggag cggcgcggcg gttgggcgcg 6000
   gaggtcggca gcaaaaacaa gcgactcgcc gagcaaaccc gaatcagcct tcagacggtc 6060
   gtgcctaaca acacgccgtt ctaccccgcc ttcttcgcgc cccttcgcgt ccaagcatcc 6120
   ttcaagttta tctctctagt tcaacttcaa gaagaacaac accaccaaca ccatgattga 6180
   acaagatgga ttgcacgcag gttctccggc cgcttgggtg gagaggctat tcggctatga 6240
   ctgggcacaa cagacaatcg gctgctctga tgccgccgtg ttccggctgt cagcgcaggg 6300
   gcgcccggtt ctttttgtca agaccgacct gtccggtgcc ctgaatgaac tgcaggacga 6360
   ggcagcgcgg ctatcgtggc tggccacgac gggcgttcct tgcgcagctg tgctcgacgt 6420
   tgtcactgaa gcgggaaggg actggctgct attgggcgaa gtgccggggc aggatctcct 6480
   gtcatctcac cttgctcctg ccgagaaagt atccatcatg gctgatgcaa tgcggcggct 6540
   gcatacgctt gatccggcta cctgcccatt cgaccaccaa gcgaaacatc gcatcgagcg 6600
   agcacgtact cggatggaag ccggtcttgt cgatcaggat gatctggacg aagagcatca 6660
   ggggctcgcg ccagccgaac tgttcgccag gctcaaggcg cgcatgcccg acggcgatga 6720
   tctcgtcgtg acccatggcg atgcctgctt gccgaatatc atggtggaaa atggccgctt 6780
   ttctggattc atcgactgtg gccggctggg tgtggcggac cgctatcagg acatagcgtt 6840
   ggctacccgt gatattgctg aagagcttgg cggcgaatgg gctgaccgct tcctcgtgct 6900
   ttacggtatc gccgctcccg attcgcagcg catcgccttc tatcgccttc ttgacgagtt 6960
   cttctgacac gtgctacgag atttcgattc caccgccgcc ttctatgaaa ggttgggctt 7020
   cggaatcgtt ttccgggacg ccggctggat gatcctccag cgcggggatc tcatgctgga 7080
   gttcttcgcc caccccaact tgtttattgc agcttataat ggttacaaat aaagcaatag 7140
   catcacaaat ttcacaaata aagcattttt ttcactgcat tctagttgtg gtttgtccaa 7200
   actcatcaat gtatcttatc atgtctgaat tcccggggta c 7241
<210> 21
   <211> 957
   <212> DNA
   <213> Pichia stipitis
<220>
   <223> xylose reductase (X59465), codon optimized
<400> 21
   atgccctcca ttaagctcaa ctccggttac gatatgcccg ccgtcggttt tggttgctgg        60
   aaggtggacg tcgacacttg ctcggagcag atttaccgcg ccattaagac cggataccgc       120
   ctctttgacg gtgccgagga ctacgccaac gagaagctgg tcggagccgg cgtcaagaag       180
   gccattgatg agggaattgt caagcgcgag gacctctttc tcacctccaa gctctggaac       240
   aactaccacc accccgataa cgtcgagaag gctcttaacc gtaccctcag cgatctccag       300
   gtcgactacg tcgatctttt tcttattcac ttccctgtca cgttcaagtt tgtccctctt       360
   gaggagaagt acccccccgg attctactgc ggaaagggcg ataactttga ctacgaggac       420
   gttcctattc tggagacttg gaaggctctc gagaagctcg tcaaggccgg caagattcgc       480
   agcatcggcg tcagcaactt tcctggagct ctcctcctgg acctccttcg cggagccacc       540
   atcaagcctt cggttcttca ggtcgagcac catccttacc ttcagcagcc ccgtctcatc       600
   gagtttgccc agtcccgcgg tattgccgtc acggcctaca gctccttcgg ccctcagtcc       660
   tttgtcgagc tcaaccaggg tcgcgccctt aacaccagcc ccctcttcga gaacgagacc       720
   attaaggcca tcgctgctaa gcacggtaag tcccccgccc aggtcctcct ccgttggagc       780
   tcgcagcgcg gaatcgccat catccctaag agcaacaccg tccctcgcct tcttgagaac       840
   aaggatgtca actccttcga cctcgatgag caggatttcg ccgacattgc caagctcgat       900
   attaacctcc gcttcaacga cccctgggac tgggataaga tccctatctt tgtctaa          957
<210> 22
   <211> 1872
   <212> DNA
   <213> Pichia stipitis
<220>
   <223> xylulose kinase (AF127802), codon optimized
<400> 22
   atgactacta cgccgtttga cgctcccgac aagctctttc ttggcttcga tctctccacc      60
   cagcagctta agattatcgt cactgacgag aacctcgccg ctctcaagac ctacaacgtc     120
   gagtttgata gcattaactc cagcgtccag aagggtgtga tcgccattaa cgatgagatc     180
   agcaagggag ccatcatcag cccggtctac atgtggctcg acgctctcga tcacgtcttc     240
   gaggatatga agaaggacgg tttccccttt aacaaggtgg tcggaatctc cggctcgtgc     300
   cagcagcacg gttcggtcta ctggtcgcgc actgctgaga aggttctctc cgagcttgac     360
   gccgagtcct ccctctcgtc ccagatgcgc tccgccttta ctttcaagca cgcccccaac     420
   tggcaggacc actcgaccgg caaggagctc gaggagtttg agcgcgtcat cggcgccgac     480
   gccctcgctg acatctccgg tagccgcgcc cactaccgct ttactggcct tcagattcgc     540
   aagctctcga cccgttttaa gcccgagaag tacaaccgca cggcccgcat ttccctggtc     600
   tccagcttcg tcgcttccgt ccttctgggt cgcattacgt ccatcgagga ggctgacgct     660
   tgcggcatga acctctacga catcgagaag cgcgagttca acgaggagct tctcgccatt     720
   gcggctggtg tccaccccga gctggacggt gtcgagcagg acggtgagat ctaccgcgcc 780
   ggtattaacg agctcaagcg taagctcggc cctgtcaagc ccatcaccta cgagtccgag 840
   ggagacatcg cctcctactt cgtcacccgc tacggtttta accctgactg caagatctac 900
   tcgtttactg gagacaacct cgccaccatc atctcccttc ctcttgcccc gaacgacgcc 960
   ctcatcagcc ttggcacctc caccaccgtg cttatcatca ccaagaacta cgccccgtcg 1020
   tcccagtacc acctctttaa gcacccgacg atgcccgacc actacatggg aatgatttgc 1080
   tactgcaacg gctccctcgc ccgtgagaag gttcgcgacg aggttaacga gaagtttaac 1140
   gtcgaggaca agaagtcgtg ggacaagttt aacgagatcc tcgacaagag caccgatttt 1200
   aacaacaagc tcggcatcta cttcccgctc ggagagattg tccctaacgc tgcggcccag 1260
   attaagcgct cggtccttaa ctcgaagaac gagatcgtcg acgtcgagct cggagataag 1320
   aactggcagc ctgaggacga tgtgagcagc attgttgagt cccagaccct ttcgtgccgc 1380
   ctccgcacgg gcccgatgct ctccaagtcc ggtgattcct ccgcttcgtc gtccgcctcg 1440
   ccccagcccg agggagatgg cacggacctc cacaaggttt accaggacct cgttaagaag 1500
   ttcggcgacc tcttcaccga tggtaagaag cagacttttg agtccctcac cgcccgcccc 1560
   aaccgctgct actacgtcgg tggcgccagc aacaacggct cgatcatcct caagatgggc 1620
   agcattctcg cccctgtgaa cggtaactac aaggtcgata tcccgaacgc gtgcgccctt 1680
   ggcggagctt acaaggcgtc gtggagctac gagtgcgagg ccaagaagga gtggattggc 1740
   tacgatcagt acattaaccg ccttttcgag gtgtccgatg agatgaacag ctttgaggtc 1800
   aaggacaagt ggctcgagta cgctaacggc gtgggcatgc tcgccaagat ggagtccgag 1860
   ctcaagcact ga 1872
<210> 23
   <211> 1092
   <212> DNA
   <213> Pichia stipitis
<220>
   <223> Xylitol dehydrogenase (X55392), codon optimized
<400> 23
   atgaccgcca acccgagcct cgtccttaac aagatcgacg atatttcctt cgagacctac 60
   gacgcccccg agatcagcga gcccaccgat gtcctcgtcc aggttaagaa gaccggcatc 120
   tgcggttccg atattcactt ttacgctcac ggacgcattg gaaactttgt cctcactaag 180
   cctatggttc tgggtcacga gtccgccggt actgtcgttc aggtgggaaa gggtgttacg 240
   tcgcttaagg tcggagacaa cgttgccatc gagcccggca tccccagccg cttttccgat 300
   gagtacaagt ccggtcacta caacctctgc ccccacatgg ctttcgccgc cacccccaac 360
   tccaaggagg gcgagcctaa cccccccggc accctctgca agtactttaa gtcccccgag 420
   gattttctcg tcaagctccc cgaccacgtc tcgcttgagc tgggcgccct cgtcgagccc 480
   ctgtccgtcg gagttcacgc cagcaagctc ggtagcgttg cctttggcga ctacgtggcc 540
   gtttttggcg cgggtcctgt cggccttctc gccgccgctg tggccaagac ctttggagcc 600
   aagggcgtta tcgtcgtcga catttttgac aacaagctca agatggctaa ggatattggc 660
   gccgctactc atacctttaa ctccaagacc ggcggttccg aggagcttat caaggccttt 720
   ggcggtaacg tcccgaacgt tgtcctcgag tgcaccggag ccgagccctg cattaagctc 780
   ggagtggatg ccatcgcccc tggtggacgc tttgtccagg ttggtaacgc cgccggtccc 840
   gtcagcttcc cgatcaccgt tttcgctatg aaggagctca ccctcttcgg cagcttccgt 900
   tacggcttta acgactacaa gaccgccgtg ggcatctttg acaccaacta ccagaacgga 960
   cgtgagaacg cccctatcga ttttgagcag ctgattaccc accgttacaa gtttaaggac 1020
   gccattgagg cctacgacct cgtccgcgct ggcaagggag ccgtcaagtg cctcatcgat 1080
   ggtcccgagt ga 1092
<210> 24
   <211> 18
   <212> PRT
   <213> Artificial Sequence
<220>
   <223> synthetic peptide
<400> 24
   Arg Leu Arg Lys Leu Pro Ile Asp His Pro Asp Ser Leu Glu Glu Leu
   1 5 10 15
   Arg Asp
<210> 25
   <211> 15
   <212> PRT
   <213> Artificial Sequence
<220>
   <223> synthetic peptide
<400> 25
   Glu Thr Lys Gly Leu Thr Leu Glu Glu Ile Glu Ala Lys Cys Leu
   1               5                   10                  15